# EUROPEAN PATENT SPECIFICATION

(11) **EP 3 054 969 B1**
(45) Date of publication and mention of the grant of the patent: **10.03.2021**
(21) Application number: 14851594.3
(22) Date of filing: 10.10.2014
(51) Int. Cl.: A61K 38/10, A61K 38/00, A61K 38/12, A61P 1/10

(54) **AGONISTS OF GUANYLATE CYCLASE USEFUL FOR THE TREATMENT OF OPIOID INDUCED DYSFUNCTIONS**
GUNAYLATCYCLASE-CYCLASE-AGONISTEN ZUR BEHANDLUNG VON OPIOIDINDUZIERTEN STÖRUNGEN
AGONISTES DE GUANYLATE CYCLASE UTILES POUR LE TRAITEMENT DE TROUBLES INDUITS PAR LES OPIOÏDES

(30) Priority: 10.10.2013 US 201361889308 P
(43) Date of publication of application: 17.08.2016
(73) Proprietor: Bausch Health Ireland Limited, Dublin 24 (IE)
(72) Inventor: SHAILUBHAI, Kunwar, Audubon, Pennsylvania 19403 (US); PALEJWALA, Vaseem, Scotch Plains, New Jersey 07076 (US)
(74) Representative: Bassil, Nicholas Charles
(86) International application number: PCT/US2014/060157
(87) International publication number: WO 2015/054649

(56) References cited:
- WO-A2-2012/037380
- US-A1- 2010 120 694
- US-A1- 2010 152 118
- US-A1- 2012 220 526
- US-A1- 2012 237 593
- Anonymous: "Plecanatide Eases Chronic Constipation", , 24 May 2013 (2013-05-24), XP055341895, Retrieved from the Internet: URL:http://www.medscape.com/viewarticle/80 4738 [retrieved on 2017-02-03]
- ARSHAD ET AL.: 'The multiple and enigmatic roles of guanylyl cyclase C in intestinal homeostasis.' FEBS LETT. vol. 586, no. 18, August 2012, pages 2835 - 40, XP055216235

## Description

### FIELD OF THE INVENTION

The present disclosure relates to the therapeutic use of guanylate cyclase C (GC-C) agonists in the treatment of opioid induced dysfunctions, such as, opioid induced bowel dysfunction opioid induced esophageal dysfunction, and opioid induced respiratory depression. The agonists may be used either alone or either concurrently or sequentially with additional active agents to prevent, treat or alleviate one ore more opioid induced dysfunctions.

### BACKGROUND OF THE INVENTION

Opioids are effective and widely used medicines for pain management in cancer and non-cancer patients. However, use of opioids often has debilitating adverse effects such as opioid-induced dysfunction, including respiratory depression, esophageal dysfunction, endocrine disruption, and opioid-induced bowel dysfunction (OIBD), which comprises opioid-induced constipation, dry mouth, nausea, vomiting, gastric stasis, bloating, and abdominal pain.

A need exists for compositions and methods to treat and prevent the side of effects of opioids.

### SUMMARY OF THE INVENTION

The invention is defined in the claims. In a first aspect, the invention provides a composition comprising a guanylate cyclase receptor agonist (GCRA) peptide consisting of the amino acid sequence of any one of SEQ ID NOs: 1-54 and 56-249 for use in preventing, treating or alleviating symptoms of an opioid-induced bowel dysfunction, wherein the composition is for administration to a subject in need thereof in a therapeutically effective amount, wherein the subject is undergoing acute opioid use or undergoing chronic opioid use.

Disclosed herein is a method for preventing, treating a condition or alleviating a symptom of an opioid induced dysfunction by administering to a subject in need thereof a therapeutically effective amount of a composition that includes a guanylate cyclase receptor agonist (GCRA) peptide. The composition may further comprise an opioid and/or a cGMP-dependent phosphodiesterase inhibitor. In one embodiment, the composition comprises a guanylate cyclase receptor agonist (GCRA) peptide and an opioid. In a further embodiment, the guanylate cyclase receptor agonist (GCRA) peptide and opioid are formulated in the same capsule or tablet. In a further embodiment, the opioid is selected from the group consisting of morphine, codeine, oxycodone, hydrocodone, dihydrocodeine, propoxyphene, fentanyl, tramadol, loperamide, butorphanol, or combinations thereof.

The compositions of the present invention may further comprise a pharmaceutical carrier, excipient, or diluent. In one embodiment, the composition comprises one or more targeting materials selected from the group consisting of a pH-dependent polymer, a swellable polymer, and a degradable composition. In a further embodiment, the composition formulation is optimized for delivery to the duodenum, jejunum, ileum, terminal ileum, or ascending colon. In another further embodiment, the composition formulation is optimized for release throughout the colon. In another embodiment, the composition comprises one or more pH dependent polymers which degrade in a pH range of 4.5 to 5.5, in a pH range of 5.5 to 6.5, or in a pH range of 6.5 to 7.5. In yet a further embodiment, the composition is formulated to release in a time dependent manner.

The method described herein may further include the step of administering to the subject an effective dose of a cGMP-dependent phosphodiesterase inhibitor. For example, the cGMP-dependent phosphodiesterase inhibitor is sulindac sulfone, zaprinast, motapizone, vardenafil or sildenafil. The cGMP-dependent phosphodiesterase inhibitor can be administered either concurrently or sequentially with the GCRA peptide or pharmaceutical composition thereof.

The present disclosure also provides a composition that includes an opioid and a GCRA peptide. The composition may further include a pharmaceutical carrier, excipient or diluent.

The GCRA peptide that can be used in any method or any composition of the disclosure has the amino acid sequence of any one of Tables 2-8. For example, the GCRA peptide is SEQ ID NO: 1 (SP-304), SEQ ID NO.:9 (SP-333), SP373 (SEQ ID NO.: 104), SP364 (SEQ ID NO.:100), SP366 (SEQ ID NO.:102) or SED ID NO: 250.

### BRIEF DESCRIPTION OF DRAWINGS

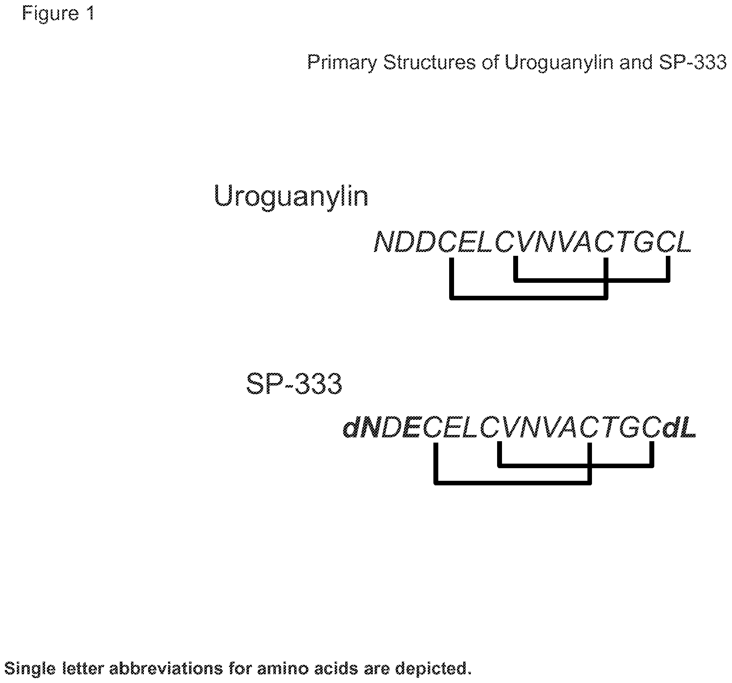

**Figure 1** is a schematic showing the primary structure of uroguanylin and SP-333. Single letter abbreviations for amino acids are depicted. SP-333 is similar to UG except for the substitution of aspartic acid (D) at position 3 from the N-terminus of UG with glutamic acid (E). Additionally, the amino acids at both termini are replaced with their respective D-stereoisomers. Uroguanylin as well as SP-333 have four cysteine (C) residues enabling the formation of two intramolecular disulfide bonds. Substituted amino acids in SP-333 are shown in bold type.

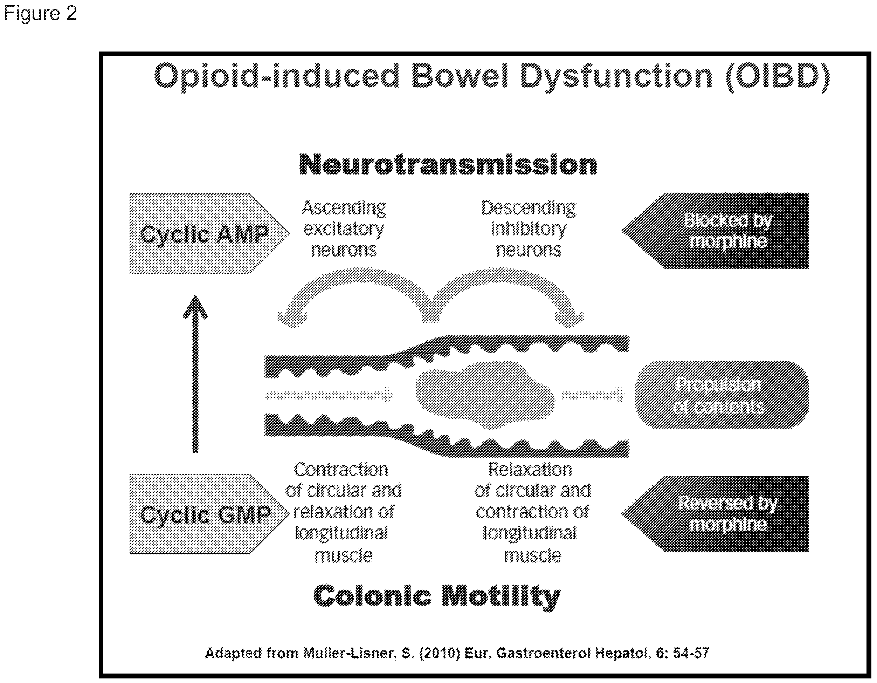

**Figure 2** is a scheme showing mechanism of action of cyclic CMP and cyclic AMP relief opioid-induced bowel dysfunction (OBD).

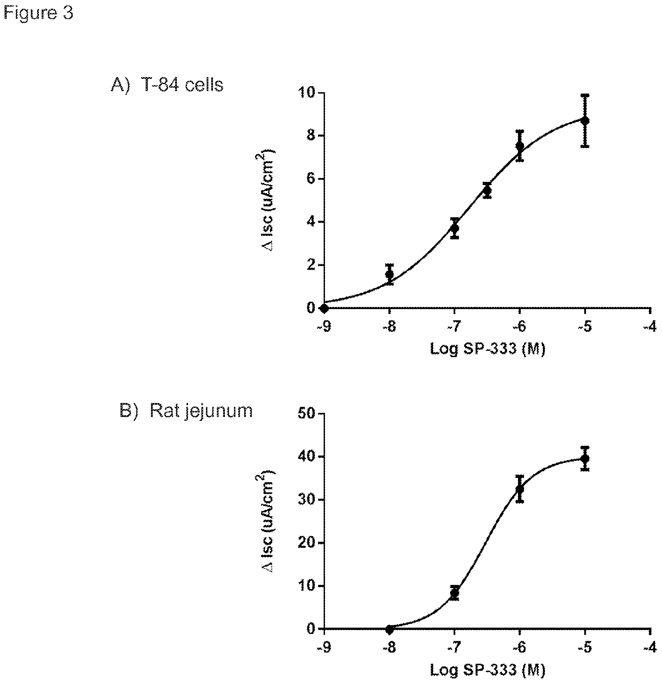

**Figure 3A-B** is a series of graphs showing that apical treatment with SP-333 stimulated *Isc* across T-84 cells monolayer and rat jejunum. A concentration dependent increase in chloride currents (measured as increase in *Isc*) detected after treating T84 cells (A) and rat jejunum tissue (B) with SP-333. The concentrations of SP-333 that induced half-maximal increase (EC50) in CI- currents are comparable between cells and tissues; 1.69 x 10⁻⁷ M and 2.99 x 10⁻⁷ M, respectively. Values are expressed as means ± S.E.M., n=4. T84 cells and jejunum from naive rats mounted on Ussing Chamber were subjected to varying concentrations of SP-333 on the apical chamber and maximal *Isc* stimulation was recorded and plotted.

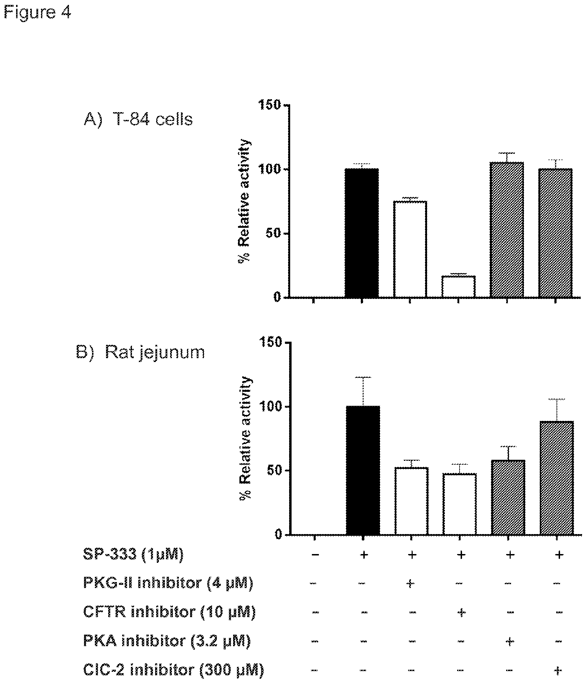

**Figure 4A-B** is a series of graphs showing that apical treatment with SP-333 stimulates *Isc* through activation of PKG-II and CFTR. Modulation of SP-333 (1 µM) stimulated apical *Isc* in T84 cells (A) rat jejunum (B) in response to treatment with the indicated inhibitors. T84 cells and rat jejunum were mounted on Ussing chamber bathed on apical and basolateral side with respective buffers as described under in Example 1. To evaluate the role played by PKA and PKG-II and to identify relative contribution of CFTR and CIC-2 towards agonists mediated release of CI- ions, experiments were performed in the presence and absence of kinase and CI⁻ channel inhibitors. Inhibitors employed were CdCl2 (CIC-2 inhibitor; Sigma-Aldrich; St Louis, MO), CFTRᵢₙₕ172 (CFTR inhibitor; Santa Cruz Biotech; Dallas, Texas), KT5823 (PKG inhibitor; R&D Systems, Minneapolis, MN), mPKI (PKA inhibitor; EMD Millipore; Billerica, MA). Apical surface of T84 cells (n=4 transwells/condition) and rat jejunum tissue (n=4-6 preparations/condition) were pretreated for 5 min with each inhibitor at the indicated concentration prior to addition of 1 µM SP-333. Peak values *of Isc* attained after addition of SP-333 was recorded and calculated in terms of percent relative activity ± SEM.

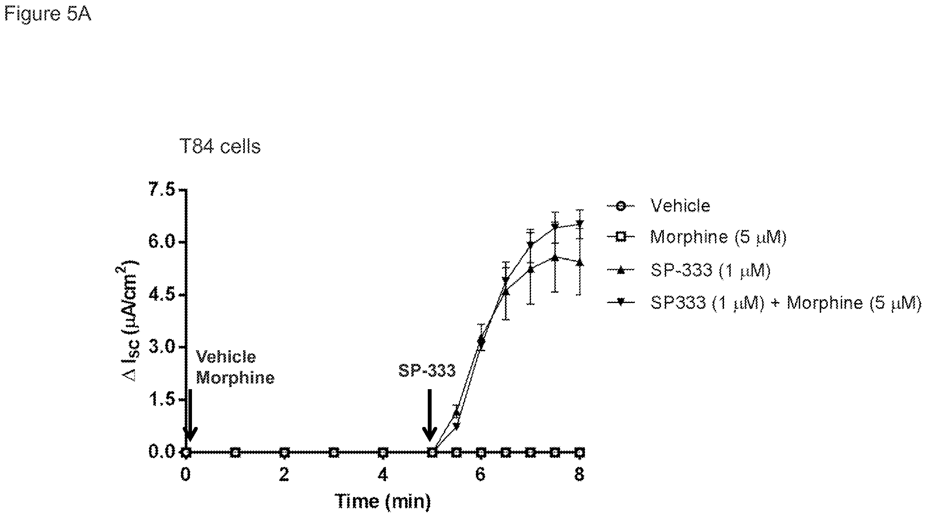

**Figure 5A-B** is a series of graphs showing that SP-333 stimulated *Isc* was not adversely affected by morphine. T84 cells (A) and rat jejunum (B) were pretreated for 5 min with 5 µM of morphine on the apical side. Subsequently, 1 µM SP-333 was added to the apical chamber and stimulation of Isc was recorded. Current recorded in the absence of opioids was considered as 100%. Relative *Isc* plotted. Values expressed as means ± S.E.M., n = 3 for T84 cells and n = 5-13 for rat jejunum.

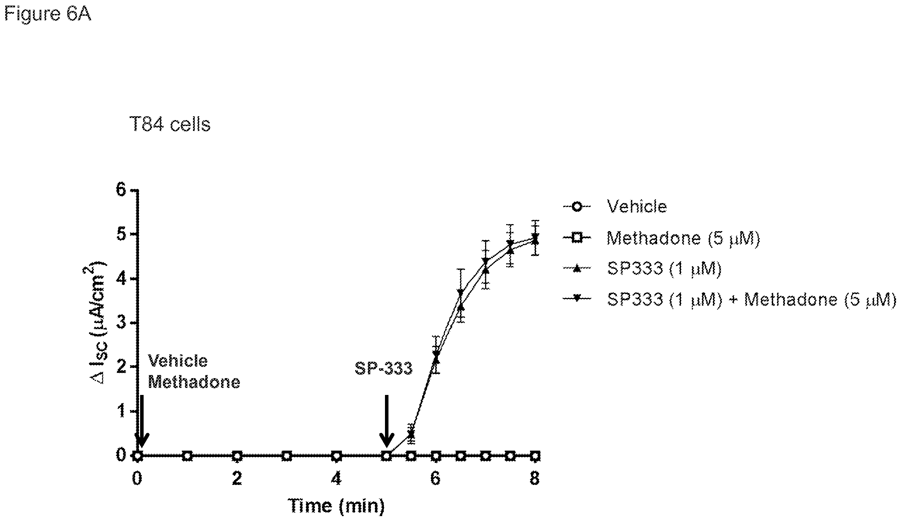

**Figure 6A-B** is a series of graphs showing that SP-333 stimulated *Isc* was not adversely affected by methadone. T84 cells (A) and rat jejunum (B) were pretreated for 5 min with 5µM of methadone on the apical side. Subsequently, 1 µM SP-333 was added to the apical chamber and stimulation of Isc was recorded. Current recorded in the absence of opioids was considered as 100%. Relative *Isc* plotted. Values expressed as means ± S.E.M., n = 3 for T84 cells and n = 5-13 for rat jejunum for each opioid.

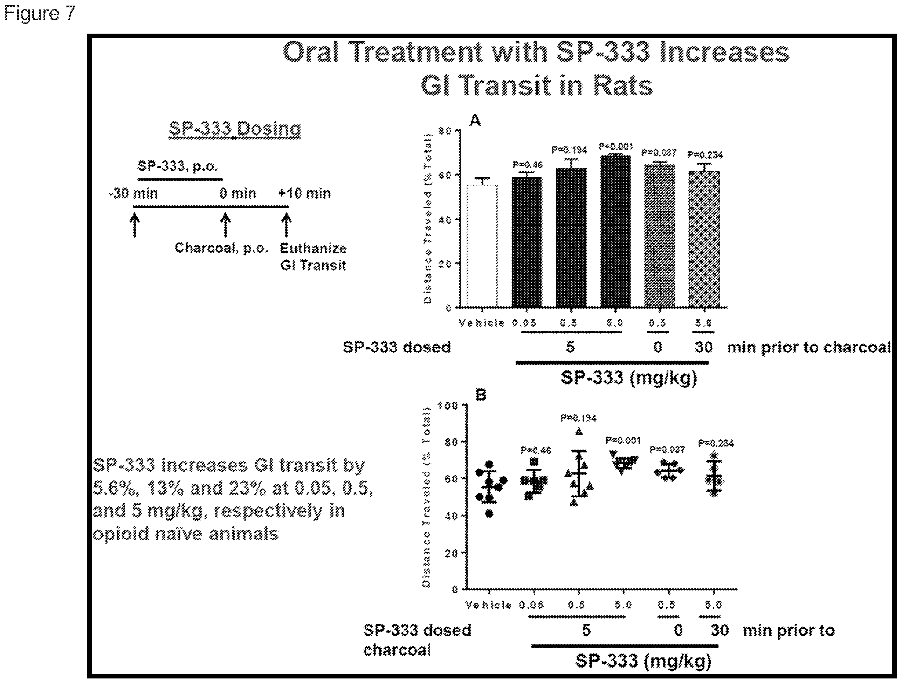

**Figure 7A-B** is a series of graphs showing that oral treatment with SP-333 increases GI transit in rats. The left panel shows SP-333 dosing scheme. Panel A is a bar graph showing that SP-333 increases GI transit by 5.6%, 13%, and 23% at 0.05, 0.5, and 5 mg/kg, respectively in opioid naive animals. Panel B is a distribution graph showing the data of each individual animal.

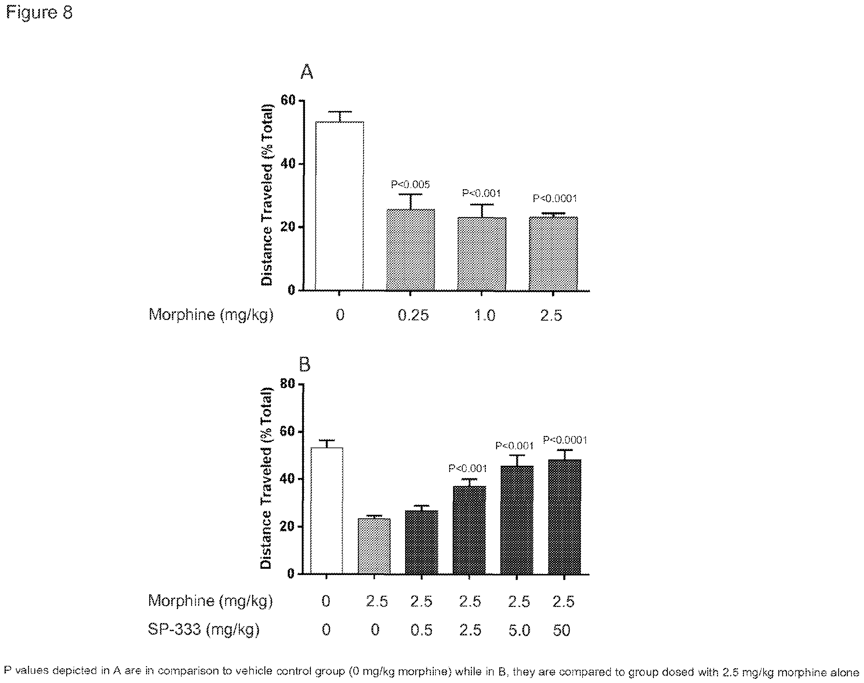

**Figure 8A-B** is a series of graphs showing that oral treatment with SP-333 improved GI transit following morphine treatment. The indicated dose of morphine was administered to female Sprague-Dawley rats via IP injection, and GI transit was evaluated as described in Example 1. Morphine significantly reduced GI transit in rats (A). Compared to vehicle controls, GI transit after a single morphine dose was slowed by 52%, 56% and 56% at 0.25, 1.0 and 2.5 mg/kg of the opioid, respectively. The ability of SP-333 to relieve morphine-induced delay in GI transit was examined by administering 2.5 mg/kg of morphine by IP injection followed by the indicated amounts of SP-333 by oral gavage, and evaluating GI transit as described in Example 1. Morphine significantly reduced GI transit (23% vs 53% in vehicle treated; p<0.0001) (B). Dose-dependent increase in GI transit was observed following SP-333 treatment: 27%, 37%, 46%, and 48% at 0.5, 2.5, 5, and 50 mg/kg, respectively (P ≤0.002) at all doses except 0.5 mg/kg as compared with morphine alone dosed group). Mean GI transit ± SEM is depicted in A and B.

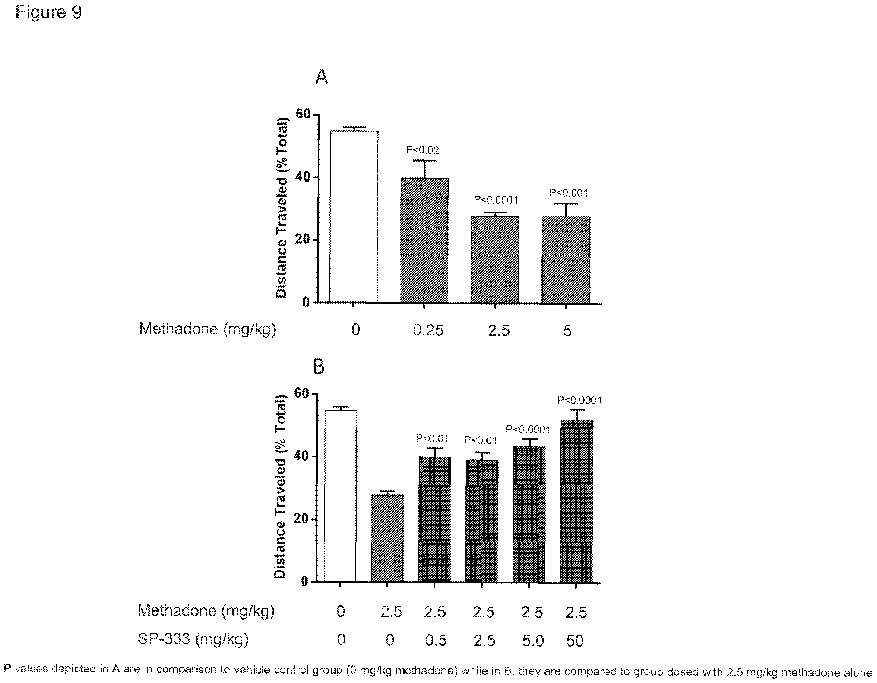

**Figure 9A-B** is a series of graphs showing that oral treatment with SP-333 improved GI transit following methadone treatment. A statistically significant reduction in GI transit was observed in rats administered with 0.25, 2.5 and 5 mg/kg of methadone (A). The GI transit delay observed at 2.5 mg/kg of methadone was comparable to that observed with morphine at the same dose (49% and 56% vs corresponding vehicle controls for methadone and morphine, respectively). SP-333 administration produced a dose-dependent improvement in gut transit in methadone-treated rats: 40%, 39%, 44%, and 52% at 0.5, 2.5, 5, and 50 mg/kg, respectively (P≤0.01 at all doses). Mean GI transit ± SEM is depicted in A and B.

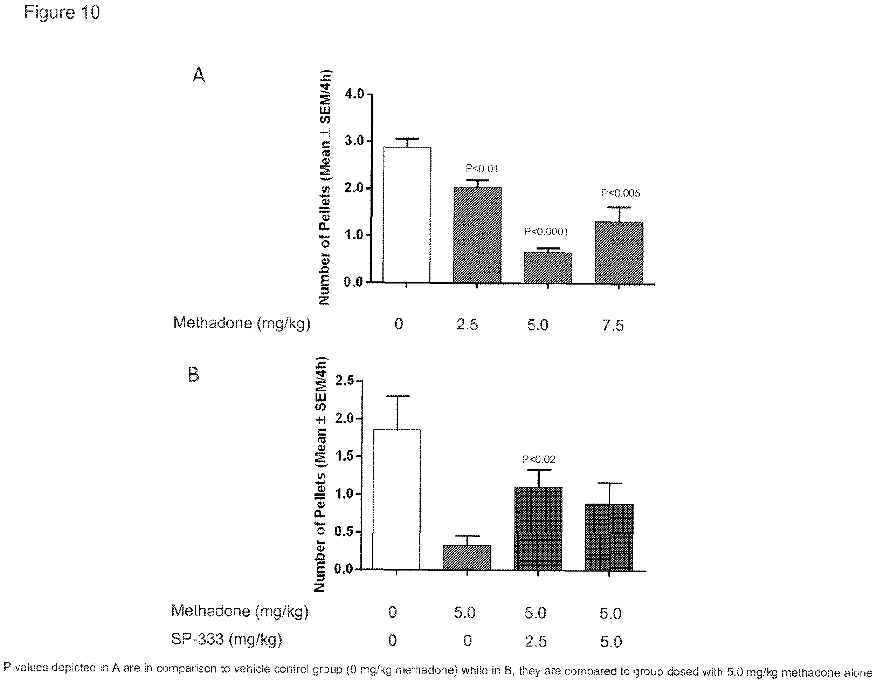

**Figure 10A-B** is a series of graphs showing that once daily treatment with SP-333 normalized GI transit and restored fecal output following methadone treatment. Constipation was induced in rats by administering a daily dose of 2.5, 5, and 7.5 mg/kg of methadone for 7 days. The average fecal output over a 4h post dosing during the light period was recorded. A statistically significant reduction in fecal output was observed at all doses of methadone. To evaluate the ability of SP-333 to relieve methadone-induced constipation, rats were dosed with 5 mg/kg of methadone once daily for 7 days. SP-333 was dosed 10 min following methadone dosing and fecal output monitored as described above. SP-333 administered to methadone-treated animals at 2.5 mg/kg significantly relieved methadone-induced constipation (B). The mean number of fecal pellet ± SEM per treatment group depicted in A and B.

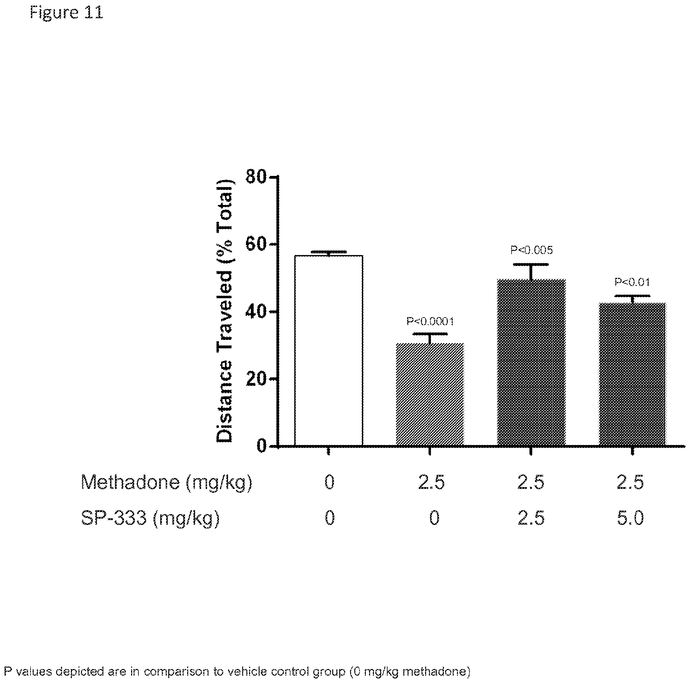

**Figure 11** is a graph showing that oral daily dosing with SP-333 relieves GI-transit delay caused by repeated methadone dosing. The effect of repeated methadone administration on GI transit was monitored after rats were dosed once daily with 5 mg/kg of methadone for 7 days followed by 2.5 mg/kg for an additional week. On the day of testing, rats were dosed with 2.5 mg/kg of methadone followed by an oral gavage of 2.5, and 5 mg/kg of SP-333. GI transit was calculated as described in Example 1 and plotted as Mean GI transit ± SEM. Methadone treatment resulted in a significant reduction in GI transit. However, a statistically significant improvement in GI transit delay was observed in SP-333 dosed animals.

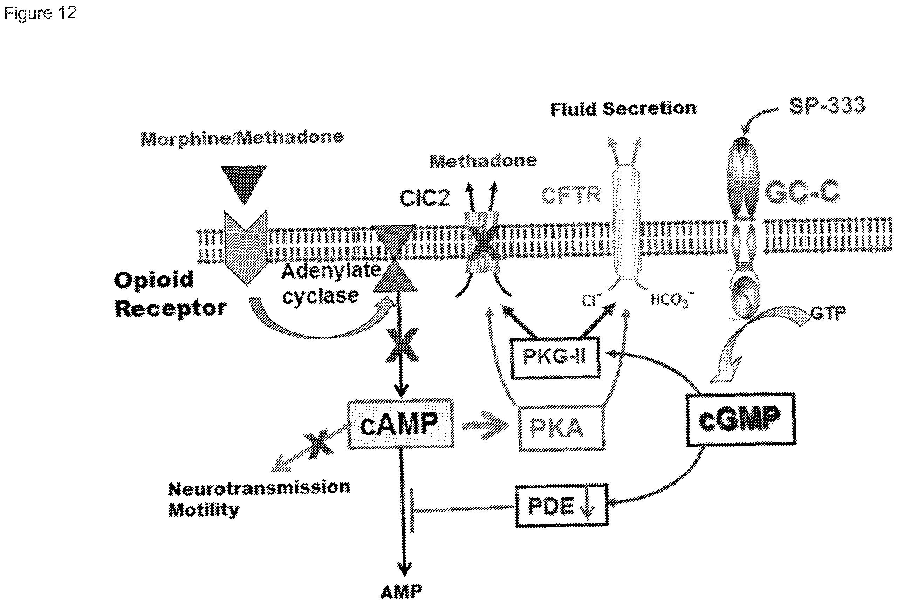

**Figure 12** is a schematic demonstrating the mechanism for SP-333 mediated reduction of opioid-induced delay of GI transit. Morphine and methadone activate peripherally expressed opioid receptors to triggers downstream signaling resulting in decrease in cAMP levels due to inhibition of adenylyl cyclase that affects release of neurotransmitters. Consequently, GI propulsion, motility and fluid secretion are impaired combined with an increase in absorption of fluids and electrolytes from intestinal lumen resulting in manifestation of OIBD. By biding to GC-C receptor on the intestinal cells, SP-333 promotes synthesis and accumulation of intracellular cGMP which in turn binds and activates PKG-II directly or indirectly via inhibition of phosphodiesterase 3. PKG-II can phosphorylate and activate CFTR channel to promote efflux of CI- ions followed by passive efflux of Na+ and water into the intestinal lumen thus facilitating bowel movement. Although methadone may inhibit CIC-2, SP-333-mediated secretion of CI-from T84 cells and rat jejunum tissues is via PKG-II and CFTR.

### DETAILED DESCRIPTION

It should be understood that singular forms such as "a," "an," and "the" are used throughout this application for convenience, however, except where context or an explicit statement indicates otherwise, the singular forms are intended to include the plural. All numerical ranges should be understood to include each and every numerical point within the numerical range, and should be interpreted as reciting each and every numerical point individually. The endpoints of all ranges directed to the same component or property are inclusive, and intended to be independently combinable.

"About" includes all values having substantially the same effect, or providing substantially the same result, as the reference value. Thus, the range encompassed by the term "about" will vary depending on context in which the term is used, for instance the parameter that the reference value is associated with. Thus, depending on context, "about" can mean, for example, ±15%, ±10%, ±5%, ±4%, ±3%, ±2%, ±1%, or ± less than 1%. Importantly, all recitations of a reference value preceded by the term "about" are intended to also be a recitation of the reference value alone.

The present disclosure is based upon the development of agonists of guanylate cyclase-C (GC-C). The agonists are analogs of uroguanylin, guanylin, lymphoguanylin and E.coli ST peptide.

Opioids are widely used for treatment of chronic pain, but their consumption is often associated varied side effects. Opioid-induced dysfunction includes respiratory depression, endocrine disruption, esophageal dysfunction, and opioid-induced bowel dysfunction. Opioid-induced dysfunction affects the gastrointestinal tract with severe constipation often observed in humans for which standard laxatives are not very effective. This side effect is due to reduction in fluid secretion, affecting gastrointestinal (GI) motility and bowel movement (BM). Table 1 lists several, non-limiting examples of deleterious actions of opioids and their clinical effect. The disclosure is based, in part, upon the discovery of utilizing GC-C agonists for treatment of opioid induced dysfunctions. An analog of uroguanylin activates guanylate cyclase-C/cGMP (GC-C/cGMP) signaling to enhance fluid secretion in the gut lumen for normalizing GI motility and BM and is useful for treatment of opioid-induced constipation (OIC). This treatment completely restores the delay in GI transit caused by morphine or methadone.

**Table 1: Actions of opioids on the gastrointestinal tract**

| Site of Action | Pharmacological Action | Clinical effect |
|---|---|---|
| Stomach | Decreased gastric motility | Anorexia |
| | Decreased pyloric tone | Nausea and vomiting |
| Small intestine | Decreased pancreatic and biliary secretion | Delayed digestion |
| | Reduced propulsion | Delayed absorption of medications |
| | Increased fluid absorption | Hard, dry stool |
| Large intestine | Decreased propulsion | Straining, incomplete evacuation, bloating, abdominal distension, constipation |
| | Increased non-propulsive contractions | Spasm, abdominal cramps, pain |
| | Increased fluid absorption | Hard, dry stool |
| | Increased anal sphincter tone | Incomplete evacuation |

Accordingly, also disclosed herein is a method for preventing, treating or alleviating a symptom of opioid induced dysfunction by administering to a subject in need of a therapeutically effective amount a GC-C agonists.

The GC-C agonists according to the disclosure include amino acid sequences represented by Formulas I-XX as well as those amino acid sequence summarized below in Tables 2-8. The GC-C agonists according to the disclosure are collectively referred to herein as "GCRA peptides". In some embodiments, the GC-C agonist has the sequence of SEQ ID NO: 1 (SP-304), SEQ ID NO.:9 (SP-333), SP373 (SEQ ID NO.:104), SP364 (SEQ ID NO.:100), SP366 (SEQ ID NO.: 102). Also disclosed herein is SED ID NO: 250.

By "opioids" it is meant to include, but are not limited to, alfentanil, anileridine, asimadoline, bremazocine, burprenorphine, butorphanol, codeine, dezocine, diacetylmorphine (heroin), dihydrocodeine, diphenoxylate, ethylmorphine, fedotozine, fentanyl, funaltrexamine, hydrocodone, hydromorphone, levallorphan, levomethadyl acetate, levorphanol, loperamide, meperidine (pethidine), methadone, morphine, morphine-6-glucoronide, nalbuphine, nalorphine, nicomorphine, opium, oxycodone, oxymorphone, papavereturn, pentazocine, propiram, propoxyphene, remifentanyl, sufentanil, tilidine, trimebutine, and tramadol.

As used herein "opioid induced dysfunction" and "side effects of opioid use" are used interchangeably and include, such as, for example, gastrointestinal dysfunction (e.g., inhibition of intestinal motility, constipation, GI sphincter constriction, nausea, emesis (vomiting)), biliary spasm, opioid bowel dysfunction, colic, dysphoria, pruritis, urinary retention, depression of respiration, papillary constriction, cardiovascular effects, chest wall rigidity and cough suppression, depression of stress response, and immune suppression associated with use of narcotic analgesia, or combinations thereof. The methods disclosed herein may thus be beneficial from a quality of life standpoint for subjects undergoing use of opioids, as well as to reduce complications arising from chronic constipation, such as hemorrhoids, appetite suppression, mucosal breakdown, sepsis, colon cancer risk, and myocardial infarction.

In accordance with the first aspect of the invention, provided GC-C agonists are useful for administration to a subject undergoing acute opioid use. Disclosed herein are formulations which are useful for administration to patients suffering from post-operative gastrointestinal dysfunction, bowel dysfunction, or respiratory depression.

In accordance with the first aspect of the invention, provided formulations are also useful for administration to subjects undergoing chronic opioid use (e.g., terminally ill patients receiving opioid therapy such as an AIDS patient, a cancer patient, a cardiovascular patient; subjects receiving chronic opioid therapy for pain management; subjects undergoing opioid therapy for maintenance of opioid withdrawal). In some embodiments, the subject is a subject using opioid therapy for chronic pain management. In certain embodiments, the pain is non-malignant pain (e.g., back pain, neuropathic pain, pain associated with fibromyalgia, osteoarthritis). In some embodiments, the subject is a terminally ill patient. In other embodiments the subject is a person undergoing opioid withdrawal maintenance therapy.

In certain embodiments, the formulations provided herein are administered to subjects that have been selected for treatment with methadone or methylnaltrexone. In specific embodiments, the subject is selected based on the subject having an increased risk for developing one or more of the conditions set forth above. In another embodiment, the subject is selected based on the use of opioid therapy for pain management, or based on having one or more of the conditions set forth herein. In certain embodiments, the subject is constipated or has a history of constipation due to opioid therapy. In one embodiment, a constipated subject has not had a bowel movement in the previous three days. In one embodiment, a constipated subject has had less than three bowel movements in the previous week. In certain embodiments, a constipated subject has had less than three rescue-free bowel movements per week on average over the last four consecutive weeks, and one or more of the following: (a) hard or lumpy stools, (b) straining during bowel movements, and/or (c) sensation of incomplete evacuation after bowel movements. The subject may be using opioids intermittently or regularly.

In one embodiment, the subject that is selected has been taking opioids as needed. In one embodiment, the subject that is selected has been taking opioids for less than one week. In one embodiment, the subject that is selected has been taking opioids over the course of at least one week. In another embodiment, the subject that is selected has been taking opioids over the course of at least two weeks. In another embodiment, the subject that is selected has been taking opioids over the course of at least three weeks. In another embodiment, the subject that is selected has been taking opioids over the course of at least four weeks. In another embodiment, the subject that is selected has been taking opioids over the course of at least three months. In another embodiment, the subject that is selected has been taking opioids over the course of at least six months. In another embodiment, the subject that is selected has been taking opioids over the course of at least twelve months. In another embodiment, the subject that is selected has been taking opioids over the course of more than one year. In another embodiment, the subject that is selected has been taking opioids at least every other day over the course of at least two weeks. In one embodiment, the subject that is selected has been receiving at least 7 doses >25 mg of oral morphine equivalents over at least 14 days. In one embodiment, the subject that is selected has been receiving a daily dose of >50 mg of oral morphine equivalents for at least 14 days. In one embodiment, the subject that is selected is constipated due to opioid therapy and has been receiving a daily dose of >50 mg of oral morphine equivalents for at least 14 days. In certain embodiments, the subject has been receiving a daily dose of >50 mg of oral morphine equivalents for at least 14 days; and has had less than three (3) rescue-free bowel movements per week on average over the least four consecutive weeks that were associated with one or more of the following: (a) a Bristol Stool Form Scale type 1 or 2 for at least 25% of the rescue-free bowel movements, (b) straining during at least 25% of the rescue-free bowel movements; and/or (c) a sensation of incomplete evacuation after at least 25% of the rescue-free bowel movements. A rescue-free bowel movement refers to a bowel movement associated with no laxative use within the 24 hours prior to the bowel movement and/or (c) sensation of incomplete evacuation after bowel movements.

In certain aspects, provided formulations may be used in methods for preventing, inhibiting, reducing, delaying, diminishing or treating gastrointestinal dysfunction, including, but not limited to, irritable bowel syndrome, opioid-induced bowel dysfunction, colitis, post-operative or postpartum ileus, nausea and/or vomiting, decreased gastric motility and emptying, inhibition of the stomach, and small and/or large intestinal propulsion, increased amplitude of non-propulsive segmental contractions, constriction of sphincter of Oddi, increased anal sphincter tone, impaired reflex relaxation with rectal distention, diminished gastric, biliary, pancreatic or intestinal secretions, increased absorption of water from bowel contents, gastro-esophageal reflux, gastroparesis, cramping, bloating, abdominal or epigastric pain and discomfort, constipation, idiopathic constipation, post-operative gastrointestinal dysfunction following abdominal surgery (e.g., colectomy (e.g., right hemicolectomy, left hemicolectomy, transverse hemicolectomy, colectomy takedown, low anterior resection), hysterectomy), and delayed absorption of orally administered medications or nutritive substances.

### GCRA PEPTIDES

The GCRA peptides of the present disclosure are analogues of, uroguanylin, guanylin, lymphoguanylin and ST peptides. No particular length is implied by the term "peptide". In some embodiments, the GCRA peptide is less than 25 amino acids in length, e.g., less than or equal to 20, 15, 14, 13, 12, 11, 10, or 5 amino acid in length.

The GCRA peptides can be polymers of L-amino acids, D-amino acids, or a combination of both. For example, the peptides are D retro-inverso peptides. The term "retro-inverso isomer" refers to an isomer of a linear peptide in which the direction of the sequence is reversed and the chirality of each amino acid residue is inverted. *See, e.g.,* Jameson et al., Nature, 368, 744-746 (1994); Brady et al., Nature, 368, 692-693 (1994). The net result of combining D-enantiomers and reverse synthesis is that the positions of carbonyl and amino groups in each amide bond are exchanged, while the position of the side-chain groups at each alpha carbon is preserved. Unless specifically stated otherwise, it is presumed that any given L-amino acid sequence of the disclosure may be made into a D retro-inverso peptide by synthesizing a reverse of the sequence for the corresponding native L-amino acid sequence. For example a GCRA peptide includes the sequence defined by Formulas I-XX and those listed on Tables 2-8.

By inducing cGMP production is meant that the GCRA peptide induces the production of intracellular cGMP. Intracellular cGMP is measured by methods known in the art. For example, the GCRA peptide of the disclosure stimulate 5%, 10%, 20%, 30%, 40%, 50%, 75%, 90% or more intracellular cGMP compared to naturally occurring GC-C agonists. In further embodiments, the GCRA peptide stimulates apoptosis, e.g., programmed cell death or activates the cystic fibrosis transmembrane conductance regulator (CFTR).

As used herein PEG3, 3 PEG, is meant to denote polyethylene glycol such as include aminoethyloxy-ethyloxy-acetic acid (AeeA).

As used herein, the term "AMIDE" is meant to denote that the terminal carboxylic acid is replaced with an amide group, *i.e.,* the terminal COOH is replaced with CONH₂.

As used herein, the term "pyGlu" refers to pyroglutamic acid.

As used herein, *(e.g.,* in Formulas I- XX) Xₐₐ is any natural, unnatural amino acid or amino acid analogue; Mₐₐ is a Cysteine (Cys), Penicillamine (Pen) homocysteine, or 3-mercaptoproline. Xaaₙ₁ is meant to denote an amino acid sequence of any natural, unnatural amino acid or amino acid analogue that is one, two or three residues in length; Xaaₙ₂ is meant to denote an amino acid sequence of any natural, unnatural amino acid or amino acid analogue that is zero or one residue in length; and Xaaₙ₃ is meant to denote an amino acid sequence of any natural, unnatural amino acid or amino acid analogue that is zero, one, two, three, four , five or six residues in length. Additionally, any amino acid represented by Xaa, may be an L-amino acid, a D-amino acid, a methylated amino acid, a florinated amino acid or any combination of thereof. Preferably the amino acids at the N- terminus, C-terminus or both are D-amino acids. Optionally, any GCRA peptide represented by Formulas I-XX may contain on or more polyethylene glycol residues at the N- terminus, C-terminus or both. An exemplary polyethylene glycol includes aminoethyloxy-ethyloxy-acetic acid and polymers thereof.

Specific examples of GCC agonist peptides disclosed herein include a peptide selected from Tables 2-8.

In some embodiments, GCC agonist peptides include peptides having the amino acid sequence of Formula I, wherein at least one amino acid of Formula I is a D-amino acid or a methylated amino acid and/or the amino acid at position 16 is a serine. Preferably, the amino acid at position 16 of Formula I is a D-amino acid or a methylated amino acid. For example, the amino acid at position 16 of Formula I is a d-leucine or a d-serine. Optionally, one or more of the amino acids at positions 1-3 of Formula I are D-amino acids or methylated amino acids or a combination of D-amino acids or methylated amino acids. For example, Asn¹, Asp² or Glu³ (or a combination thereof) of Formula I is a D-amino acid or a methylated amino acid. Preferably, the amino acid at position Xaa⁶ of Formula I is a leucine, serine or tyrosine.

In alternative embodiments, GCC agonist peptides include peptides having the amino acid sequence of Formula II, wherein at least one amino acid of Formula II is a D-amino acid or a methylated amino acid. Preferably, the amino acid denoted by Xaaₙ₂ of Formula II is a D-amino acid or a methylated amino acid. In some embodiments, the amino acid denoted by Xaaₙ₂ of Formula II is a leucine, a d-leucine, a serine, or a d-serine. Preferably, the one or more amino acids denoted by Xaaₙ₁ of Formula II are D-amino acids or methylated amino acids. Preferably, the amino acid at position Xaa⁶ of Formula II is a leucine, a serine, or a tyrosine.

In some embodiments, GCC agonist peptides include peptides having the amino acid sequence of Formula III, wherein at least one amino acid of Formula III is a D-amino acid or a methylated amino acid and/or Maa is not a cysteine. Preferably, the amino acid denoted by Xaaₙ₂ of Formula III is a D-amino acid or a methylated amino acid. In some embodiments the amino acid denoted by Xaaₙ₂ of Formula III is a leucine, a d-leucine, a serine, or a d-serine. Preferably, the one or more amino acids denoted by Xaaₙ₁ of Formula III are D-amino acids or methylated amino acids. Preferably, the amino acid at position Xaa⁶ of Formula III is a leucine, a serine, or a tyrosine.

In other embodiments, GCC agonist peptides include peptides having the amino acid sequence of Formula IV, wherein at least one amino acid of Formula IV is a D-amino acid or a methylated amino acid, and/or Maa is not a cysteine. Preferably, the Xaaₙ₂ of Formula IV is a D-amino acid or a methylated amino acid. In some embodiments, the amino acid denoted by Xaaₙ₂ of Formula IV is a leucine, a d-leucine, a serine, or a d-serine. Preferably, the one or more of the amino acids denoted by Xaaₙ₁ of Formula IV are D-amino acids or methylated amino acids. Preferably, the amino acid denoted Xaa⁶ of Formula IV is a leucine, a serine, or a tyrosine.

In further embodiments, GCC agonist peptides include peptides having the amino acid sequence of Formula V, wherein at least one amino acid of Formula V is a D-amino acid or a methylated amino acid. Preferably, the amino acid at position 16 of Formula V is a D-amino acid or a methylated amino acid. For example, the amino acid at position 16 (i.e., Xaa¹⁶) of Formula V is a d-leucine or a d-serine. Optionally, one or more of the amino acids at position 1-3 of Formula V are D-amino acids or methylated amino acids or a combination of D-amino acids or methylated amino acids. For example, Asn¹, Asp² or Glu³ (or a combination thereof) of Formula V is a D-amino acids or a methylated amino acid. Preferably, the amino acid denoted at Xaa⁶ of Formula V is a leucine, a serine, or a tyrosine.

In additional embodiments, GCRA peptides include peptides having the amino acid sequence of Formula VI, VII-a, VII-b, VIII, or IX. Preferably, the amino acid at position 6 of Formula VI, VII-a, VII-b, VIII, or IX is a leucine, a serine or a tyrosine. In some aspects the amino acid at position 16 of Formula VI, VII-a, VII-b, VIII or IX is a leucine or a serine. Preferably, the amino acid at position 16 of Formula VI, VII-a, VII-b, VIII or IX is a D-amino acid or a methylated amino acid.

In additional embodiments, GCRA peptides include peptides having the amino acid sequence of Formula X, XI, XII, XIII, XIV, XV, XVI or XVII. Optionally, one or more amino acids of Formulas X, XI, XII, XIII, XIV, XV, XVI or XVII are D-amino acids or methylated amino acids. Preferably, the amino acid at the carboxyl terminus of the peptides according to Formulas X, XI, XII, XIII, XIV, XV, XVI or XVII is a D-amino acid or a methylated amino acid. For example the amino acid at the carboxyl terminus of the peptides according to Formulas X, XI, XII, XIII, XIV, XV, XVI or XVII is a D-tyrosine.

Preferably, the amino acid denoted by Xaa⁶ of Formula XIV is a tyrosine, phenylalanine or a serine. Most preferably the amino acid denoted by Xaa⁶ of Formula XIV is a phenylalanine or a serine. Preferably, the amino acid denoted by Xaa⁴ of Formula XV, XVI or XVII is a tyrosine, a phenylalanine, or a serine. Most preferably, the amino acid position Xaa⁴ of Formula XV, XVI or XVII is a phenylalanine or a serine.

In some embodiments, GCRA peptides include peptides containing the amino acid sequence of Formula XVIII. Preferably, the amino acid at position 1 of Formula XVIII is a glutamic acid, aspartic acid, glutamine or lysine. Preferably, the amino acid at position 2 and 3 of Formula XVIII is a glutamic acid, or an aspartic acid. Preferably, the amino acid at position 5 is a glutamic acid. Preferably, the amino acid at position 6 of Formula XVIII is an isoleucine, valine, serine, threonine or tyrosine. Preferably, the amino acid at position 8 of Formula XVIII is a valine or isoleucine. Preferably, the amino acid at position 9 of Formula XVIII is an asparagine. Preferably, the amino acid at position 10 of Formula XVIII is a valine or a methionine. Preferably, the amino acid at position 11 of Formula XVIII is an alanine. Preferably, the amino acid at position 13 of Formula XVIII is a threonine. Preferably, the amino acid at position 14 of Formula XVIII is a glycine. Preferably, the amino acid at position 16 of Formula XVIII is a leucine, serine or threonine

In alternative embodiments, GCRA peptides include peptides containing the amino acid sequence of Formula XIX. Preferably, the amino acid at position 1 of Formula XIX is a serine or asparagine. Preferably, the amino acid at position 2 of Formula XIX is a histidine or an aspartic acid. Preferably, the amino acid at position 3 of Formula XIX is a threonine or a glutamic acid. Preferably, the amino acid at position 5 of Formula XIX is a glutamic acid. Preferably, the amino acid at position 6 of Formula XIX is an isoleucine, leucine, valine or tyrosine. Preferably, the amino acid at position 8, 10, 11, or 13 of Formula XIX is an alanine. Preferably, the amino acid at position 9 of Formula XIX is an asparagine or a phenylalanine. Preferably, the amino acid at position 14 of Formula XIX is a glycine.

In further embodiments, GCRA peptides include peptides containing the amino acid sequence of Formula XX Preferably, the amino acid at position 1 of Formula XX is a glutamine. Preferably, the amino acid at position 2 or 3 of Formula XX is a glutamic acid or an aspartic acid. Preferably, the amino acid at position 5 of Formula XX is a glutamic acid. Preferably, the amino acid at position 6 of Formula XX is threonine, glutamine, tyrosine, isoleucine, or leucine. Preferably, the amino acid at position 8 of Formula XX is isoleucine or valine. Preferably, the amino acid at position 9 of Formula XX is asparagine. Preferably, the amino acid at position 10 of Formula XX is methionine or valine. Preferably, the amino acid at position 11 of Formula XX is alanine. Preferably, the amino acid at position 13 of Formula XX is a threonine. Preferably, the amino acid at position 1 of Formula XX is a glycine. Preferably, the amino acid at position 15 of Formula XX is a tyrosine. Optionally, the amino acid at position 15 of Formula XX is two-amino acid in length and is Cysteine (Cys), Penicillamine (Pen) homocysteine, or 3-mercaptoproline and serine, leucine or threonine.

In certain embodiments, one or more amino acids of the GCRA peptides can be replaced by a non-naturally occurring amino acid or a naturally or non-naturally occurring amino acid analog. There are many amino acids beyond the standard 20 (Ala, Arg, Asn, Asp, Cys, Gln, Glu, Gly, His, Ile, Leu, Lys, Met, Phe, Pro, Ser, Thr, Trp, Tyr, and Val). Some are naturally-occurring others are not. (*See,* for example, Hunt, The Non-Protein Amino Acids: In Chemistry and Biochemistry of the Amino Acids, Barrett, Chapman and Hall, 1985). For example, an aromatic amino acid can be replaced by 3,4-dihydroxy-L-phenylalanine, 3-iodo-L-tyrosine, triiodothyronine, L-thyroxine, phenylglycine (Phg) or nor-tyrosine (norTyr). Phg and norTyr and other amino acids including Phe and Tyr can be substituted by, *e.g.,* a halogen, -CH3, -OH, - CH2NH3, -C(O)H, -CH2CH3, - CN, -CH2CH2CH3, -SH, or another group. Any amino acid can be substituted by the D-form of the amino acid.

With regard to non-naturally occurring amino acids or naturally and non-naturally occurring amino acid analogs, a number of substitutions in the polypeptide and agonists described herein are possible alone or in combination.

For example, glutamine residues can be substituted with gamma-Hydroxy-Glu or gamma-Carboxy-Glu. Tyrosine residues can be substituted with an alpha substituted amino acid such as L-alpha-methylphenylalanine or by analogues such as: 3-Amino-Tyr; Tyr(CH3); Tyr(PO3(CH3)2); Tyr(SO3H); beta-Cyclohexyl-Ala; beta-(1-Cyclopentenyl)-Ala; beta-Cyclopentyl-Ala; beta-Cyclopropyl-Ala; beta-Quinolyl-Ala; beta-(2-Thiazolyl)-Ala; beta-(Triazole-1-yl)-Ala; beta-(2-Pyridyl)-Ala; beta-(3-Pyridyl)-Ala; Amino-Phe; Fluoro-Phe; Cyclohexyl-Gly; tBu-Gly; beta-(3-benzothienyl)-Ala; beta-(2-thienyl)-Ala; 5-Methyl-Trp; and A- Methyl-Trp. Proline residues can be substituted with homopro (L-pipecolic acid); hydroxy-Pro; 3,4-Dehydro-Pro; 4-fluoro-Pro; or alpha-methyl-Pro or an N(alpha)-C(alpha) cyclized amino acid analogues with the structure: n = 0, 1, 2, 3 Alanine residues can be substituted with alpha-substituted or N-methylated amino acid such as alpha-amino isobutyric acid (aib), L/D-alpha-ethylalanine (L/D-isovaline), L/D-methylvaline, or L/D-alpha-methylleucine or a non-natural amino acid such as beta-fluoro-Ala. Alanine can also be substituted with: n = 0, 1, 2, 3 Glycine residues can be substituted with alpha-amino isobutyric acid (aib) or L/D-alpha-ethylalanine (L/D-isovaline).

Further examples of unnatural amino acids include: an unnatural analog of alanine (e.g., L-1-Nal or L-2-Nal); an unnatural analog of tyrosine; an unnatural analogue of glutamine; an unnatural analogue of phenylalanine; an unnatural analogue of serine; an unnatural analogue of threonine; an alkyl, aryl, acyl, azido, cyano, halo, hydrazine, hydrazide, hydroxyl, alkenyl, alkynl, ether, thiol, sulfonyl, seleno, ester, thioacid, borate, boronate, phospho, phosphono, phosphine, heterocyclic, enone, imine, aldehyde, hydroxylamine, keto, or amino substituted amino acid, or any combination thereof; an amino acid with a photoactivatable cross-linker; a spin-labeled amino acid; a fluorescent amino acid; an amino acid with a novel functional group; an amino acid that covalently or noncovalently interacts with another molecule; a metal binding amino acid; an amino acid that is amidated at a site that is not naturally amidated, a metal-containing amino acid; a radioactive amino acid; a photocaged and/or photoisomerizable amino acid; a biotin or biotin-analogue containing amino acid; a glycosylated or carbohydrate modified amino acid; a keto containing amino acid; amino acids comprising polyethylene glycol or polyether; a heavy atom substituted amino acid *(e.g.,* an amino acid containing deuterium, tritium, ¹³C, ¹⁵N, or ¹⁸O); a chemically cleavable or photocleavable amino acid; an amino acid with an elongated side chain; an amino acid containing a toxic group; a sugar substituted amino acid, *e.g.,* a sugar substituted serine or the like; a carbon-linked sugar-containing amino acid; a redox-active amino acid; an α-hydroxy containing acid; an amino thio acid containing amino acid; an α, α disubstituted amino acid; a β- amino acid; a cyclic amino acid other than proline; an O-methyl-L-tyrosine; an L-3-(2- naphthyl)alanine; a 3-methyl-phenylalanine; a p-acetyl-L-phenylalanine; an O-4-allyl-L-tyrosine; a 4-propyl-L-tyrosine; a tri-O-acetyl-GlcNAc β -serine; an L-Dopa; a fluorinated phenylalanine; an isopropyl-L-phenylalanine; a p-azido-L-phenylalanine; a p-acyl-L-phenylalanine; a p- benzoyl-L-phenylalanine; an L-phosphoserine; a phosphonoserine; a phosphonotyrosine; a p- iodo-phenylalanine; a 4-fluorophenylglycine; a p-bromophenylalanine; a p-amino-L- phenylalanine; an isopropyl-L-phenylalanine; L-3-(2-naphthyl)alanine; D- 3-(2-naphthyl)alanine (dNal); an amino-, isopropyl-, or O-allyl-containing phenylalanine analogue; a dopa, O-methyl-L-tyrosine; a glycosylated amino acid; a p-(propargyloxy)phenylalanine; dimethyl-Lysine; hydroxy-proline; mercaptopropionic acid; methyl-lysine; 3-nitro-tyrosine; norleucine; pyro-glutamic acid; Z (Carbobenzoxyl); ε- Acetyl-Lysine; β-alanine; β-aspartic acid; β-cyclohexylalanine; aminobenzoyl derivative; aminobutyric acid (Abu); citrulline; aminohexanoic acid (Ahx); aminoisobutyric acid (AIB); cyclohexylalanine; d-cyclohexylalanine; cyclohexylglycine; hydroxyproline; nitro-arginine; nitro-phenylalanine; nitro-tyrosine; norvaline; octahydroindole carboxylate; ornithine (Orn); penicillamine (PEN); tetrahydroisoquinoline; diaminobutyric acid; diaminopimelic acid; pyroglutamic acid; homocysteine; homoserine; N-ε-dinitrophenyl-lysine; N-ε-methyl-lysine; N-ε-dimethyl-lysine; N,N,N- ε-trimethyl-lysine; acetamidomethyl protected amino acids and pegylated amino acids. Further examples of unnatural amino acids and amino acid analogs can be found in U.S. 20030108885, U.S. 20030082575, US20060019347 (paragraphs 410-418) and the references cited therein. The polypeptides of the disclosure can include further modifications including those described in US20060019347, paragraph 589.

"Nal" used herein refers to both L-1-naphthylalanine (L-1-Nal) and L-2-naphthylalanine (L-2-Nal).

In some embodiments, an amino acid can be replaced by a naturally-occurring, non-essential amino acid, *e*.*g*., taurine.

Alternatively, the GCRA peptides are cyclic peptides. GCRA cyclic peptides are prepared by methods known in the art. For example, macrocyclization is often accomplished by forming an amide bond between the peptide N- and C-termini, between a side chain and the Nor C-terminus [*e.g*., with K₃Fe(CN)₆ at pH 8.5] (Samson et al., Endocrinology, 137: 5182-5185 (1996)), or between two amino acid side chains, such as cysteine. See, *e.g.,* DeGrado, Adv Protein Chem, 39: 51-124 (1988). In some embodiments, the GCRA peptides of the present disclosure are bicyclic peptides. In various aspects the GCRA peptides are [4,12; 7,15] bicycles.

In some GCRA peptides one or both members of one or both pairs of Cys residues which normally form a disulfide bond can be replaced by homocysteine, penicillamine, 3-mercaptoproline (Kolodziej et al. 1996 Int J Pept Protein Res 48:274); β, β dimethylcysteine (Hunt et al. 1993 Int JPept Protein Res 42:249) or diaminopropionic acid (Smith et al. 1978 J Med Chem 2 1:117) to form alternative internal cross-links at the positions of the normal disulfide bonds.

In addition, one or more disulfide bonds can be replaced by alternative covalent cross-links, *e.g.,* an amide linkage (-CH2CH(O)NHCH 2- or -CH2NHCH(O)CH 2-), an ester linkage, a thioester linkage, a lactam bridge , a carbamoyl linkage, a urea linkage, a thiourea linkage, a phosphonate ester linkage, an alkyl linkage (-CH2CH2CH2CH2-), an alkenyl linkage(-CH 2CH=CHCH 2-), an ether linkage (-CH2CH2OCH2- or -CH2OCH2CH2-), a thioether linkage (-CH2CH2SCH2- or - CH2SCH2CH2-), an amine linkage (-CH2CH2NHCH2- or -CH2NHCH 2CH2-) or a thioamide linkage (-CH2CH(S)HNHCH 2- or -CH2NHCH(S)CH 2-). For example, Ledu et al. (Proc Nat'l Acad. Sci. 100:11263-78, 2003) describe methods for preparing lactam and amide cross-links. Exemplary GCRA peptides which include a lactam bridge include for example SP-370.

The GCRA peptides can have one or more conventional polypeptide bonds replaced by an alternative bond. Such replacements can increase the stability of the polypeptide. For example, replacement of the polypeptide bond between a residue amino terminal to an aromatic residue (e.g. Tyr, Phe, Trp) with an alternative bond can reduce cleavage by carboxy peptidases and may increase half-life in the digestive tract. Bonds that can replace polypeptide bonds include: a retro-inverso bond (C(O)-NH instead of NH-C(O); a reduced amide bond (NH-CH2); a thiomethylene bond (S-CH2 or CH2-S); an oxomethylene bond (0-CH 2 or CH2-O); an ethylene bond (CH2-CH2); a thioamide bond (C(S)-NH); a trans-olefine bond (CH=CH); a fiuoro substituted trans-olefme bond (CF=CH); a ketomethylene bond (C(O)-CHR or CHR-C(O) wherein R is H or CH3; and a fluoro-ketomethylene bond (C(O)-CFR or CFR-C(O) wherein R is H or F or CH3.

The GCRA peptides can be modified using standard modifications. Modifications may occur at the amino (N-), carboxy (C-) terminus, internally or a combination of any of the preceding. In one aspect described herein, there may be more than one type of modification on the polypeptide. Modifications include but are not limited to: acetylation, amidation, biotinylation, cinnamoylation, farnesylation, formylation, myristoylation, palmitoylation, phosphorylation (Ser, Tyr or Thr), stearoylation, succinylation, sulfurylation and cyclisation (via disulfide bridges or amide cyclisation), and modification by Cys3 or Cys5. The GCRA peptides described herein may also be modified by 2, 4-dinitrophenyl (DNP), DNP-lysine, modification by 7-Amino-4-methyl- coumarin (AMC), flourescein, NBD (7-Nitrobenz-2-Oxa-1,3-Diazole), p-nitro-anilide, rhodamine B, EDANS (5-((2-aminoethyl)amino)naphthalene-1- sulfonic acid), dabcyl, dabsyl, dansyl, texas red, FMOC, and Tamra (Tetramethylrhodamine). The GCRA peptides described herein may also be conjugated to, for example, polyethylene glycol (PEG); alkyl groups (*e*.*g*., C1-C20 straight or branched alkyl groups); fatty acid radicals; combinations of PEG, alkyl groups and fatty acid radicals (*See,* U.S. Patent 6,309,633; Soltero et al., 2001 Innovations in Pharmaceutical Technology 106-110); BSA and KLH (Keyhole Limpet Hemocyanin). The addition of PEG and other polymers which can be used to modify polypeptides of the disclosure is described in US2006019347 section IX.

In one aspect, the disclosure provides an Aad-GCRA peptide as taught in, for example, WO20140151206.
Table 1 of WO20140151206 discloses examples of various alpha-aminoadipic acid derivatives of GCRA Peptides. The Aad-GCRA peptides are analogues uroguanylin, guanylin, lymphoguanylin and ST peptides. Particularly, these analogs contain an α-aminoadipic acid (Ad), preferably at the 3rd position from the N-terminus of each peptide or at the position to the N-terminal side next to the first cysteine ("Cys) residue. For example an Aad-GCRA peptide includes the sequences defined by Formulae I, II, III, IV, V, VI, VII, VII, VIII, IX, XVIII or XXI which can comprise an α-aminoadipic acid.

In some instances, the Aad-GCRA peptide is Asn¹-Asp²-Aad³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ (SEQ ID NO: 251; SP-304-Aad), dAsn¹-Asp²-Aad³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ (SEQ ID NO: 253; SP-333-Aad); Pyglu¹-Asp²-Aad³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu-AMIDE¹⁶ (SEQ ID NO: 254; SP-373-Aad), dAsn¹-Asp²-Aad³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer¹⁶ (SEQ ID NO: 255; SP-364-Aad); dAsn¹-Asp²-Aad³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dTyr¹⁶ (SEQ ID NO: 256; SP-366-Aad); or Xaaₙ₁-Cys⁴-Xaa⁵-Xaa⁶-Xaa⁷-Xaa⁸-Xaa⁹-Xaa¹⁰-Xaa¹¹-Cys¹²-Xaa¹³-Xaa¹⁴-Xaa¹⁵-Xaaₙ₂¹⁶ (SEQ ID NO: 257).

Also disclosed are peptides that biologically or functional equivalent to the peptides described herein. The term "biologically equivalent" or functional equivalent" is intended to mean that the compositions of the present disclosure are capable of demonstrating some or all of the cGMP production modulatory effects.

GCRA peptides can also include derivatives of GCRA peptides which are intended to include hybrid and modified forms of GCRA peptides in which certain amino acids have been deleted or replaced and modifications such as where one or more amino acids have been changed to a modified amino acid or unusual amino acid and modifications such as glycosylation so long the modified form retains the biological activity of GCRA peptides. By retaining the biological activity, it is meant that cGMP and or apoptosis is induced by the GCRA peptide, although not necessarily at the same level of potency as that of a naturally-occurring GCRA peptide identified.

Preferred variants are those that have conservative amino acid substitutions made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains *(e.g.,* aspartic acid, glutamic acid), uncharged polar side chains *(e.g.,* glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains *(e.g.,* threonine, valine, isoleucine) and aromatic side chains *(e.g.,* tyrosine, phenylalanine, tryptophan, histidine). Thus, a predicted nonessential amino acid residue in a GCRA polypeptide is replaced with another amino acid residue from the same side chain family. Alternatively, in another embodiment, mutations can be introduced randomly along all or part of a GCRA coding sequence, such as by saturation mutagenesis, and the resultant mutants can be screened to identify mutants that retain activity.

**Table 2. GCRA Peptides (SP-304 and Derivatives)**

| Name | Position of Disulfide bonds | Structure | SEQ ID NO |
|---|---|---|---|
| SP-304 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 1 |
| SP-326 | C3:C11,C6:C14 | Asp¹-Glu²-Cys³-Glu⁴-Leu⁵-Cys⁶-Val⁷-Asn⁸-Val⁹-Ala¹⁰-Cys¹¹-Thr¹²-Gly¹³-Cys¹⁴-Leu¹⁵ | 2 |
| SP-327 | C3:C11,C6:C14 | Asp¹-Glu²-Cys³-Glu⁴-Leu⁵-Cys⁶-Val⁷-Asn⁸-Val⁹-Ala¹⁰-Cys¹¹-Thr¹²-Gly¹³-Cys¹⁴ | 3 |
| SP-328 | C2:C10,C5:C13 | Glu¹-Cys²-Glu³-Leu⁴-Cys⁵-Val⁶-Asn⁷-Val⁸-Ala⁹-Cys¹⁰-Thr¹¹-Gly¹²-Cys¹³-Leu¹⁴ | 4 |
| SP-329 | C2:C10,C5:C13 | Glu¹-Cys²-Glu³-Leu⁴-Cys⁵-Val⁶-Asn⁷-Val⁸-Ala⁹-Cys¹⁰-Thr¹¹-Gly¹²-Cys¹³ | 5 |
| SP-330 | C1:C9,C4:C12 | Cys¹-Glu²-Leu³-Cys⁴-Val⁵-Asn⁶-Val⁷-Ala⁸-Cys⁹-Thr¹⁰-Gly¹¹-Cys¹²-Leu¹³ | 6 |
| SP-331 | C1:C9,C4:C12 | Cys¹-Glu²-Leu³-Cys⁴-Val⁵-Asn⁶-Val⁷-Ala⁸-Cys⁹-Thr¹⁰-Gly¹¹-Cys¹² | 7 |
| SP332 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 8 |
| SP-333 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 9 |
| SP-334 | C4:C12,C7:C15 | dAsn¹-dAsp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 10 |
| SP-335 | C4:C12,C7:C15 | dAsn¹-dAsp²-dGlu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 11 |
| SP-336 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 12 |
| SP-337 | C4:C12,C7:C15 | dAsn¹-Asp²-Glu³-Cys⁴-Glu⁵-dLeu⁶-Cys⁷-Val⁸-Asn⁹-Val¹⁰-Ala¹¹-Cys¹²-Thr¹³-Gly¹⁴-Cys¹⁵-dLeu¹⁶ | 13 |
| SP-338 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵ | 14 |
| SP-342 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 15 |
| SP-343 | C4:C12,C7:C15 | PEG3-dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 16 |
| SP-344 | C4:C12,C7:C15 | PEG3-dAsn¹-dAsp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 17 |
| SP-347 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 18 |
| SP-348 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 19 |
| SP-350 | C4:C12,C7:C15 | PEG3-dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ 22 | 20 |
| SP-352 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 21 |
| SP-358 | C4:C12,C7:C15 | PEG3-dAsn1-dAsp2-dGlu3-Cys4-Glu5-Leu6-Cys7-Val8-Asn9-Val10-Ala11-Cys12-Thr13-Gly14-Cys15-dLeu16- PEG3 | 22 |
| SP-359 | C4:C12,C7:C15 | PEG3-dAsn¹-dAsp²-dGlu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 23 |
| SP-360 | C4:C12,C7:C15 | dAsn¹-dAsp²-dGlu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dleu¹⁶-PEG3 | 24 |
| SP-361 | C4:C12,C7:C15 | dAsn¹-dAsp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶-PEG3 | 25 |
| SP-362 | C4:C12,C7:C15 | PEG3-dAsn¹-dAsp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dLeu¹⁶ | 26 |
| SP-368 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dNal¹⁶ | 27 |
| SP-369 | C4:C12,C7:C15 | dAsn¹-Asp²-Glu³-Cys⁴-Glu⁵-Leu⁶-Cys⁷-AIB⁸-Asn⁹-AIB¹⁰-Ala¹¹-Cys¹²-Thr¹³-Gly¹⁴-Cys¹⁵-dLeu¹⁶ | 28 |
| SP-370 | C4:C12,7:15 | dAsn¹-Asp²-Glu³-Cys⁴-Glu⁵-Leu⁶-Asp[Lactam]⁷-Val⁸-Asn⁹-Val¹⁰-Ala¹¹-Cys¹²-Thr¹³-Gly¹⁴-Orn¹⁵-dLeu¹ | 29 |
| SP-371 | C4:C12,C7:C15 | dAsn¹-Asp²-Glu³-Cys⁴-Glu⁵-Tyr⁶-Cys⁷-Val⁸-Asn⁹-Val¹⁰-Ala¹¹-Cys¹²-Thr¹³-Gly¹⁴-Cys¹⁵-dLeu¹⁶ | 30 |
| SP-372 | C4:C12,C7:C15 | dAsn¹-Asp²-Glu³-Cys⁴-Glu⁵-Ser⁶⁻Cys⁷-Val⁸-Asn⁹-Val¹⁰-Ala¹¹-Cys¹²-Thr¹³-Gly¹⁴-Cys¹⁵-dLeu¹⁶ | 31 |
| N1 | C4:C12,C7:C15 | PEG3-dAsn¹-Asp²-Glu³-Cys⁴-Glu⁵-Tyr⁶-Cys⁷-Val⁸-Asn⁹-Val¹⁰-Ala¹¹-Cys¹²-Thr¹³-Gly¹⁴-Cys¹⁵-dLeu¹⁶-PEG3 | 32 |
| N2 | C4:C12,C7:C15 | PEG3-dAsn¹-Asp²-Glu³-Cys⁴-Glu⁵-Tyr⁶-Cys⁷-Val⁸-Asn⁹-Val¹⁰-Ala¹¹-Cys¹²-Thr¹³-Gly¹⁴-Cys¹⁵-dLeu¹⁶ | 33 |
| N3 | C4:C12,C7:C15 | dAsn¹-Asp²-Glu³-Cys⁴-Glu⁵-Tyr⁶-Cys⁷-Val⁸-Asn⁹-V¹¹-Cys¹²-Thr¹³-Gly¹⁴-Cys¹⁵-dLeu¹⁶-PEG3 | 34 |
| N4 | C4:C12,C7:C15 | PEG3-dAsn¹-Asp²-Glu³-Cys⁴-Glu⁵-Ser⁶-Cys⁷-Val⁸-Asn⁹-Val¹⁰-Ala¹¹-Cys¹²-Thr¹³-Gly¹⁴-Cys¹⁵-dLeu¹⁶-PEG3 | 35 |
| N5 | C4:C12,C7:C15 | PEG3-dAsn¹-Asp²-Glu³-Cys⁴-Glu⁵-Ser⁶-Cys⁷-Val⁸-Asn⁹-Val¹⁰-Ala¹¹-Cys¹²-Thr¹³-Gly¹⁴-Cys¹⁵-dLeu¹⁶ | 36 |
| N6 | C4:C12,C7:C15 | dAsn¹-Asp²-Glu³-Cys⁴-Glu⁵-Ser⁶-Cys⁷-Val⁸-Asn⁹-Val¹⁰-Ala¹¹-Cys¹²-Thr¹³-Gly¹⁴-Cys¹⁵-dLeu¹⁶-PEG3 | 37 |
| N7 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 38 |
| N8 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶-PEG3 | 39 |
| N9 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 40 |
| N10 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶-PEG3 | 41 |
| N11 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer¹⁶-PEG3 | 42 |
| N12 | C4:C12,C7:C15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer¹⁶ | 43 |
| N13 | C4:C12,C7:C15 | Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer¹⁶-PEG3 | 44 |
| FormulaI | C4:C12,C7:C15 | Asn¹-Asp²-Glu³-Cys⁴-Xaa⁵-Xaa⁶-Cys⁷-Xaa⁸-Xaa⁹-Xaa¹⁰-Xaa¹¹-Cys¹²-Xaa¹³-Xaa¹⁴-Cys¹⁵-Xaa¹⁶ | 45 |
| FormulaII | C4:C12,C7:C15 | Xaaₙ₁-Cys⁴-Xaa⁵-Xaa⁶-Cys⁷-Xaa⁸-Xaa⁹-Xaa¹⁰-Xaa¹¹-Cys¹²-Xaa¹³-Xaa¹⁴-Cys¹⁵-Xaaₙ₂¹⁶ | 46 |
| Formula III | 4:12,7:15 | Xaaₙ₁-Maa⁴-Glu⁵-Xaa⁶-Maa⁷-Val⁸-Asn⁹-Val¹⁰-Ala¹¹-Maa¹²-Thr¹³-Gly¹⁴-Maa¹⁵-Xaaₙ₂ | 47 |
| Formula IV | 4:12,7:15 | Xaaₙ₁-Maa⁴-Xaa⁵-Xaa⁶-Maa⁷-Xaa⁸-Xaa⁹-Xaa¹⁰-Xaa¹¹-Maa¹²-Xaa¹³-Xaa¹⁴-Maa¹⁵-Xaaₙ₂ | 48 |
| FormulaV | C4:C12,C7:C15 | Asn¹-Asp¹-Asp³-Cys⁴-Xaa⁵-Xaa⁶-Cys⁷-Xaa⁸-Asn⁹-Xaa¹⁰-Xaa¹¹-Cys¹²-Xaa¹³-Xaa¹⁴-Cys¹⁵-Xaa¹⁶ | 49 |
| Formula VI | C4:C12,C7:C15 | dAsn¹-Glu²-Glu³-Cys⁴-Xaa⁵-Xaa⁶-Cys⁷-X3⁸-Asn⁹-Xaa¹⁰-Xaa¹¹-Cys¹²-Xaa¹³-Xaa¹⁴-Cys¹⁵-d-Xaa¹⁶ | 50 |
| Formula VII-a | C4:C12,C7:C15 | dAsn¹-dGlu²-Asp³-Cys⁴-Xaa⁵-Xaa⁶-Cys⁷-Xaa⁸-Asn⁹-Xaa¹⁰-Xaa¹¹-Cys¹²-Xaa¹³-Xaa¹⁴-Cys¹⁵-d-Xaa¹⁶ | 51 |
| Formula VII-b | C4:C12,C7:C15 | dAsn¹-dAsp²-Glu³-Cys⁴-Xaa⁵-Xaa⁶-Cys⁷-Xaa⁸-Asn⁹-Xaa¹⁰-Xaa¹¹-Cys¹²-Xaa¹³-Xaa¹⁴-Cys¹⁵-d-Xaa¹⁶ | 52 |
| Formula VIII | C4:C12,C7:C15 | dAsn¹-dAsp²-dGlu³-Cys⁴-Xaa⁵-Xaa⁶-Cys⁷-Xaa⁸-Tyr⁹-Xaa¹⁰-Xaa¹¹-Cys¹²-Xaa¹³-Xaa¹⁴-Cys¹⁵-d-Xaa¹⁶ | 53 |
| Formula IX | C4:C12,C7:C15 | dAsn¹-dGlu²-dGlu³-Cys⁴-Xaa⁵-Xaa⁶-Cys⁷-Xaa⁸-Tyr⁹-Xaa¹⁰-Xaa¹¹-Cys¹²-Xaa¹³-Xaa¹⁴-Cys¹⁵-d-Xaa¹⁶ | 54 |
| Formula XXI | C4:C12,C7:C15 | Xaaₙ₁-Cys⁴-Xaa⁵-Xaa⁶-Xaa⁷-Xaa⁸-Xaa⁹-Xaa¹⁰-Xaa¹¹-Cys¹²-Xaa¹³-Xaa¹⁴-Xaa¹⁵-Xaaₙ₂¹⁶ | 250 |

**Table 3. Linaclotide and Derivatives**

| Name | Position of Disulfide Bonds | Structure | SEQIDNO: |
|---|---|---|---|
| SP-33 9(linaclotide) | C1:C6, C2:C10, C5:C13 | Cys¹-Cys²-Glu3-Tyr⁴-Cys⁵-Cys⁶-Asn⁷-Pro⁸-Ala⁹-Cys¹⁰-Thr¹¹-Gly¹²-Cys¹³-Tyr¹⁴ | 55 |
| SP-340 | C1:C6, C2:C10, C5:C13 | Cys¹-Cys²-Glu³-Tyr⁴-Cys⁵-Cys⁶-Asn⁷-Pro⁸-Ala⁹-Cys¹⁰-Thr¹¹-Gly¹²-Cys¹³ | 56 |
| SP-349 | C1:C6, C2:C10, C5:C13 | PEG3-Cys1-Cys2-Glu3-Tyr4-Cys5-Cys6-Asn7-Pro8-Ala9-Cys10-Thr11-Gly12-Cys13-Tyr14- PEG3 | 57 |
| SP-353 | C3:C8, C4:C12, C7:C15 | Asn1-Phe2-Cys3-Cys4-Glu5-Ser6-Cys7-Cys8-Asn9-Pro10-Ala11-Cys12-Thr13-Gly14-Cys15- Tyr16 | 58 |
| SP-354 | C3:C8, C4:C12, C7:C15 | Asn1-Phe2-Cys3-Cys4-Glu5-Phe6-Cys7-Cys8-Asn9-Pro10-Ala11-Cys12-Thr13-Gly14-Cys15- Tyr16 | 59 |
| SP-355 | C1:C6, C2:C10, C5:C13 | Cys1-Cys2-Glu3-Tyr4-Cys5-Cys6-Asn7-Pro8-Ala9-Cys10-Thr11-Gly12-Cys13-dTyr14 | 60 |
| SP-357 | C1:C6, C2:C10, C5:C13 | PEG3-Cys1-Cys2-Glu3-Tyr4-Cys5-Cys6-Asn7-Pro8-Ala9-Cys10-Thr11-Gly12-Cys13-Tyr14 | 61 |
| SP-374 | C3:C8, C4:C12, C7:C15 | Asn1-Phe2-Cys3-Cys4-Glu5-Thr6-Cys7-Cys8-Asn9-Pro10-Ala11-Cys12-Thr13-Gly14-Cys15- Tyr16 | 62 |
| SP-375 | C3:C8, C4:C12, C7:C15 | Asn¹-Phe²-Cys³-Cys⁴-Glu⁵-Ser⁶-Cys⁷-Cys⁸-Asn⁹-Pro¹⁰-Ala¹¹-Cys¹²-Thr¹³-Gly¹⁴-Cys¹⁵- dTyr¹⁶ | 63 |
| SP-376 | C3:C8, C4:C12, C7:C15 | dAsn¹-Phe²-Cys³-Cys⁴-Glu⁵-Ser⁶-Cys⁷-Cys⁸-Asn⁹-Pro¹⁰-Ala¹¹-Cys¹²-Thr¹³-Gly¹⁴-Cys¹⁵- Tyr¹⁶ | 64 |
| SP-377 | C3:C8, C4:C12, C7:C15 | dAsn1-Phe2-Cys3-Cys4-Glu5-Ser6-Cys7-Cys8-Asn9-Pro10-Ala11-Cys12-Thr13-Gly14-Cys15- dTyr16 | 65 |
| SP-378 | C3:C8, C4:C12, C7:C15 | Asn1-Phe2-Cys3-Cys4-Glu5-Thr6-Cys7-Cys8-Asn9-Pro10-Ala11-Cys12-Thr13-Gly14-Cys15- dTyr16 | 66 |
| SP-379 | C3:C8, C4:C12, C7:C15 | dAsn¹-Phe²-Cys³-Cys⁴-Glu⁵-Thr⁶-Cys⁷-Cys⁸-Asn⁹-Pro¹⁰-Ala¹¹-Cys¹²-Thr¹³-Gly¹⁴-Cys¹⁵- Tyr¹⁶ | 67 |
| SP-380 | C3:C8, C4:C12, C7:C15 | dAsn1-Phc2-Cys3-Cys4-Glu5-Thr6-Cys7-Cys8-Asn9-Pro10-Ala11-Cys12-Thr13-Gly14-Cys15- dTyr16 | 68 |
| SP-381 | C3:C8, C4:C12, C7:15 | Asn1-Phe2-Cys3-Cys4-Glu5-Phe6-Cys7-Cys8-Asn9-Pro10-Ala11-Cys12-Thr13-Gly14-Cys15- dTyr16 | 69 |
| SP-382 | C3:C8, C4:C12, C7:15 | dAsn¹-Phe²-Cys³-Cys⁴-Glu⁵-Phe⁶-Cys⁷-Cys⁸-Asn⁹-Pro¹⁰-Ala¹¹-Cys¹²-Thr¹³-Gly¹⁴-Cys¹⁵- Tyr¹⁶ | 70 |
| SP-383 | C3:C8, C4:C12, C7:15 | dAsn1-Phe2-Cys3-Cys4-Glu5-Phe6-Cys7-Cys8-Asn9-Pro10-Ala11-Cys12-Thr13-Gly14-Cys15- dTyr16 | 71 |
| SP384 | C1:C6, C2:C10, C5:C13 | Cys¹-Cys²-Glu³-Tyr⁴-Cys⁵-Cys⁶-Asn⁷-Pro⁸-Ala⁹-Cys¹⁰-Thr¹¹-Gly¹²-Cys¹³-Tyr¹⁴-PEG3 | 72 |
| N14 | C1:C6, C2:C10, C5:C13 | PEG3-Cys¹-Cys²-Glu³-Tyr⁴-Cys⁵-Cys⁶-Asn⁷-Pro⁸-Ala⁹-Cys¹⁰-Thr¹¹-Gly¹²-Cys¹³-PEG3 | 73 |
| N15 | C1:C6, C2:C10, C5:C13 | PEG3-Cys¹-Cys²-Glu³-Tyr⁴-Cys⁵-Cys⁶-Asn⁷-Pro⁸-Ala⁹-Cys¹⁰-Thr¹¹-Gly¹²-Cys¹³ | 74 |
| N16 | C1:C6, C2:C10, C5:C13 | Cys¹-Cys²-Glu³-Tyr⁴-Cys⁵-Cys⁶-Asn⁷-Pro⁸-Ala⁹-Cys¹⁰-Thr¹¹-Gly¹²-Cys¹³-PEG3 | 75 |
| N17 | C3:C8, C4:C12, C7:C15 | PEG3-Asn1-Phe2-Cys3-Cys4-Glu5-Ser6-Cys7-Cys8-Asn9-Pro10-Ala11-Cys12-Thr13-Gly14- Cys15-Tyr16-PEG3 | 76 |
| N18 | C3:C8, C4:C12, C7:C15 | PEG3-Asn1-Phe2-Cys3-Cys4-Glu5-Ser6-Cys7-Cys8-Asn9-Pro10-Ala11-Cys12-Thr13-Gly14- Cys15-Tyr16 | 77 |
| N19 | C3:C8, C4:C12, C7:C15 | Asn1-Phe2-Cys3-Cys4-Glu5-Ser6-Cys7-Cys8-Asn9-Pro10-Ala11-Cys12-Thr13-Gly14-Cys15- Tyr16-PEG3 | 78 |
| N20 | C3:C8, C4:C12, C7:C15 | PEG3-Asn^1-Phe^2-Cys^3-Cys^4-Glu^5-Phe^6-Cys^7-Cys^8-Asn^9-Pro^10-Ala^11-Cys^12-Thr^13-Gly^14- Cys^15-Tyr^16-PEG3 | 79 |
| N21 | C3:C8, C4:C12, C7:C15 | PEG3-Asn1-Phe2-Cys3-Cys4-Glu5-Phe6-Cys7-Cys8-Asn9-Pro10-Ala11-Cys12-Thr13-Gly14- Cys15-Tyr16 | 80 |
| N22 | C3:C8, C4:C12, C7:C15 | Asn1-Phe2-Cys3-Cys4-Glu5-Phe6-Cys7-Cys8-Asn9-Pro10-Ala11-Cys12-Thr13-Gly14-Cys15- Tyr16-PEG3 | 81 |
| N23 | C3:C8, C4:C12, C7:C15 | PEG3-Asn¹-Phe²-Cys³-Cys⁴-Glu⁵-Tyr⁶-Cys⁷-Cys⁸-Asn⁹-Pro¹⁰-Ala¹¹-Cys¹²-Thr¹³-Gly¹⁴- Cys¹⁵-Tyr¹⁶-PEG3 | 82 |
| N24 | C3:C8, C4:C12, C7:C15 | PEG3-Asn¹-Phe²-Cys³-Cys⁴-Glu⁵-Tyr⁶-Cys⁷-Cys⁸-Asn⁹-Pro¹⁰-Ala¹¹-Cys¹²-Thr¹³-Gly¹⁴- Cys¹⁵-Tyr¹⁶ | 83 |
| N25 | C3:C8, C4:C12, C7:C15 | Asn1-Phe2-Cys3-Cys4-Glu5-Tyr6-Cys7-Cys8-Asn9-Pro10-Ala11-Cys12-Thr13-Gly14-Cys15- Tyr16-PEG3 | 84 |
| N26 | C1:C6, C2:C10, C5:C13 | Cys¹-Cys²-Glu3-Ser⁴-Cys⁵-Cys⁶-Asn⁷-Pro⁸-Ala⁹-Cys¹⁰-Thr¹¹-Gly¹²-Cys¹³-Tyr¹⁴ | 85 |
| N27 | C1:C6, C2:C10, C5:C13 | Cys¹-Cys²-Glu3-Phe⁴-Cys⁵-Cys⁶-Asn⁷-Pro⁸⁻Ala⁹⁻Cys¹⁰-Thr¹¹-Gly¹²-Cys¹³-Tyr¹⁴ | 86 |
| N28 | C1:C6, C2:C10, C5:C13 | Cys¹-Cys²-Glu3-Ser⁴-Cys⁵-Cys⁶-Asn⁷-Pro⁸-Ala⁹-Cys¹⁰-Thr¹¹-Gly¹²-Cys¹³- | 87 |
| N29 | C1:C6, C2:C10, C5:C13 | Cys¹-Cys²-Glu3-Phe⁴-Cys⁵-Cys⁶-Asn⁷-Pro⁸-Ala⁹-Cys¹⁰-Thr¹¹-Gly¹²-Cys¹³ | 88 |
| N30 | 1:6, 2:10, 5:13 | Pen¹-Pen²-Glu3-Tyr⁴-Pen⁵-Pen⁶-Asn⁷-Pro⁸-Ala⁹-Pen¹⁰-Thr¹¹-Gly¹²-Pen¹³-Tyr¹⁴ | 89 |
| N31 | 1:6,2:10,5:13 | Pen¹-Pen²-Glu3-Tyr⁴-Pen⁵-Pen⁶-Asn⁷-Pro⁸-Ala⁹-Pen¹⁰-Thr¹¹-Gly¹²-Pen¹³ | 90 |
| Formula X | C9:C14, C10:C18, C13:C21 | Xaa1-Xaa2-Xaa3-Xaa4-Xaa5-Xaa6-Asn7-Tyr8-Cys9-Cys10-Xaa11-Tyr12-Cys13-Cys14-Xaa15- Xaa16-Xaa17-Cys18-Xaa19-Xaa20-Cys21-Xaa22 | 91 |
| Formula XI | C9:C14, C10:C18, C13:C21 | Xaa^1-Xaa^2-Xaa^3-Xaa^4-Xaa^5-Xaa^6-Asn^7-Phe^8-Cys^9-Cys^10-Xaa^11-Phe^12-Cys^13-Cys^14-Xaa^15- Xaa^16-Xaa^17-Cys^18-Xaa^19-Xaa^20-Cys^21-Xaa^22 | 92 |
| Formula XII | C3:C8, C4:C12, C7:C15 | Asn¹-Phe²-Cys³-Cys⁴-Xaa⁵-Phe⁶-Cys⁷-Cys⁸-Xaa⁹-Xaa¹⁰-Xaa¹¹-Cys¹²-Xaa³-Xaa¹⁴-Cys¹⁵- Xaa¹⁶ | 93 |
| Formula XIII | 3:8,4:12,7:15 | Asn1-Phe2-Pen3-Cys4-Xaa5-Phe6-Cys7-Pen8-Xaa9-Xaa10-Xaa11-Cys12-Xaa13-Xaa14-Cys15- Xaa16 | 94 |
| Formula XIV | 3:8,4:12,7:15 | Asn¹-Phe²-Maa³-Maa⁴-Xaa⁵-Xaa⁶-Maa⁷⁻Maa⁸-Xaa⁹-Xaa¹⁰-Xaa¹¹⁻Maa¹²-Xaa¹³-Xaa¹⁴⁻ Maa¹⁵-Xaa¹⁶ | 95 |
| Formula XV | 1:6, 2:10, 5:13 | Maa¹-Maa²-Glu3-Xaa⁴-Maa⁵-Maa⁶-Asn⁷-Pro⁸-Ala⁹-Maa¹⁰-Thr¹¹-Gly¹²-Maa¹³-Tyr¹⁴ | 96 |
| Formula XVI | 1:6, 2:10, 5:13 | Maa¹-Maa²-Glu3-Xaa⁴-Maa⁵-Maa⁶-Asn⁷-Pro⁸-Ala⁹-Maa¹⁰-Thr¹¹-Gly¹²-Maa¹³ | 97 |
| Formula XVII | 1:6, 2:10, 5:13 | Xaaₙ₃-Maa¹-Maa²-Xaa³-Xaa⁴-Maa⁵-Maa⁶-Xaa⁷-Xaa⁸-Xaa⁹-Maa¹⁰-Xaa¹¹-Xaa¹²-Maa¹³- Xaaₙ₂ | 98 |

**Table 4.GCRA Peptides**

| Name | Position of Disulfide bonds | Structure | SEQIDNO: |
|---|---|---|---|
| SP-363 | C4:C12,C7:C15 | dAsn1-Asp2-Glu3-Cys4-Glu5-Leu6-Cys7-Val8-Asn9-Val10-Ala11-Cys12-Thr13-Gly14-Cys15-dLeu- AMIDE16 | 99 |
| SP-364 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer¹⁶ | 100 |
| SP-365 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dSer- AMIDE¹⁶ | 101 |
| SP-366 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dTyr¹⁶ | 102 |
| SP-367 | C4:C12,C7:C15 | dAsn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-dTyr- AMIDE¹⁶ | 103 |
| SP-373 | C4:C12,C7:C15 | Pyglu1-Asp2-Glu3-Cys4-Glu5-Leu6-Cys7-Val8-Asn9-Val10-Ala11-Cys12-Thr13-Gly14-Cys15-dLeu- AMIDE16 | 104 |
| / | C4:C12,C7:C15 | Pyglu1-Asp2-Glu3-Cys4-Glu5-Leu6-Cys7-Val8-Asn9-Val10-Ala11-Cys12-Thr13-Gly14-Cys15-Leu16 | 251 |
| SP-304diPEG | C4:C12,C7:C15 | PEG3-Asn1-Asp2-Glu3-Cys4-Glu5-Leu6-Cys7-Val8-Asn9-Val10-Ala11-Cys12-Thr13-Gly14-Cys15-Leu16- PEG3 | 105 |
| SP-304N-PEG | C4:C 12,C7:C 15 | PEG3-Asn¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 106 |
| SP-304C-PEG | C4:C 12,C7:C 15 | Asn¹-Asp²-Glu³-Cys⁴-Glu⁵ -Leu⁶-Cys⁷-Val⁸-Asn⁹-Val¹⁰-Ala¹¹-Cys¹²-Thr¹³ -Gly¹⁴-Cys¹⁵-Leu¹⁶-PEG3 | 107 |

**Table 5. SP-304 Analogs, Uroguanylin, and Uroguanylin Analogs**

| Name | Position of Disulfide bonds | Structure | SEQIDNO |
|---|---|---|---|
| Formula XVIII | C4:C12, C7:C15 | Xaa¹-Xaa²-Xaa³-Maa⁴-Xaa⁵-Xaa⁶-Maa⁷-Xaa⁸-Xaa⁹-Xaa¹⁰-Xaa¹¹-Maa¹²-Xaa¹³-Xaa¹⁴-Maa¹⁵-Xaa¹⁶ | 108 |
| Uroguanylin | C4:C12, C7:C15 | Asn¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 109 |
| N32 | C4:C12,C7:C15 | Glu¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 110 |
| N33 | C4:C12,C7:C15 | Glu¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 111 |
| N34 | C4:C12,C7:C15 | Glu¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 112 |
| N35 | C4:C12,C7:C15 | Glu¹-Glu²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 113 |
| N36 | C4:C12,C7:C15 | Asp¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 114 |
| N37 | C4:C12,C7:C15 | Asp¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 115 |
| N38 | C4:C12,C7:C15 | Asp¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 116 |
| N39 | C4:C12,C7:C15 | Asp¹-Glu²⁻Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 117 |
| N40 | C4:C12,C7:C15 | Gln¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 118 |
| N41 | C4:C12,C7:C15 | Gln¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 119 |
| N42 | C4:C12,C7:C15 | Gln¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 120 |
| N43 | C4:C12,C7:C15 | Gln¹-Glu²⁻Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 121 |
| N44 | C4:C12,C7:C15 | Lys¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 122 |
| N45 | C4:C12,C7:C15 | Lys¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 123 |
| N46 | C4:C12,C7:C15 | Lys¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 124 |
| N47 | C4:C12,C7:C15 | Lys¹-Glu²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Leu¹⁶ | 125 |
| N48 | C4:C12,C7:C15 | Glu¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 126 |
| N49 | C4:C12,C7:C15 | Glu¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 127 |
| N50 | C4:C12,C7:C15 | Glu¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 128 |
| N51 | C4:C12,C7:C15 | Glu¹-Glu²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 129 |
| N52 | C4:C12,C7:C15 | Asp¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 130 |
| N53 | C4:C12,C7:C15 | Asp¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 131 |
| N54 | C4:C12,C7:C15 | Asp¹-Glu²⁻Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 132 |
| N55 | C4:C12,C7:C15 | Asp¹-Glu²⁻Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 133 |
| N56 | C4:C12,C7:C15 | Gln¹-Asp²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 134 |
| N57 | C4:C12,C7:C15 | Gln¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 135 |
| N58 | C4:C12,C7:C15 | Gln¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 136 |
| N59 | C4:C12,C7:C15 | Gln¹-Glu²⁻Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 137 |
| N60 | C4:C12,C7:C15 | Lys¹-Asp²-Asp³-Cys⁴-Glu⁵⁻Leu⁶-Cys⁷-Val⁸-Asn⁹-Val¹⁰-Ala¹¹-Cys¹²-Thr¹³-Gly¹⁴-Cys¹⁵-Ser¹⁶ | 138 |
| N61 | C4:C12,C7:C15 | Lys¹-Asp²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 139 |
| N62 | C4:C12,C7:C15 | Lys¹-Glu²-Asp³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 140 |
| N63 | C4:C12,C7:C15 | Lys¹-Glu²-Glu³-Cys⁴-Glu⁵-Leu⁶-Cys⁷-Val⁸-Asn⁹-Val¹⁰-Ala¹¹-Cys¹²-Thr¹³-Gly¹⁴-Cys¹⁵-Ser¹⁶ | 141 |
| N65 | C4:C12,C7:C15 | Glu¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 142 |
| N66 | C4:C12,C7:C15 | Glu¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 143 |
| N67 | C4:C12,C7:C15 | Glu¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 144 |
| N68 | C4:C12,C7:C15 | Glu¹-Glu²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 145 |
| N69 | C4:C12,C7:C15 | Asp¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 146 |
| N70 | C4:C12,C7:C15 | Asp¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 147 |
| N71 | C4:C12,C7:C15 | Asp¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 148 |
| N72 | C4:C12,C7:C15 | Asp¹-Glu²⁻Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 149 |
| N73 | C4:C12,C7:C15 | Gln¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 150 |
| N74 | C4:C12,C7:C15 | Gln¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 151 |
| N75 | C4:C12,C7:C15 | Gln¹-Glu²⁻Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 152 |
| N76 | C4:C12,C7:C15 | Gln¹-Glu²⁻Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 153 |
| N77 | C4:C12,C7:C15 | Lys¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 154 |
| N78 | C4:C12,C7:C15 | Lys¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 155 |
| N79 | C4:C12,C7:C15 | Lys¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 156 |
| N80 | C4:C12,C7:C15 | Lys¹-Glu²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Leu¹⁶ | 157 |
| N81 | C4:C12,C7:C15 | Glu¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 158 |
| N82 | C4:C12,C7:C15 | Glu¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 159 |
| N83 | C4:C12,C7:C15 | Glu¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 160 |
| N84 | C4:C12,C7:C15 | Glu¹-Glu²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 161 |
| N85 | C4:C12,C7:C15 | Asp¹-Asp²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 162 |
| N86 | C4:C12,C7:C15 | Asp¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 163 |
| N87 | C4:C12,C7:C15 | Asp¹-Glu²-Asp³-Cys⁴-Glu⁵-Leu⁶-Cys⁷-Ile⁸-Asn⁹ -Met¹⁰-Ala¹¹-Cys¹²-Thr¹³-Gly¹⁴-Cys¹⁵-Ser¹⁶ | 164 |
| N88 | C4:C12,C7:C15 | Asp¹-Glu²-Glu³-Cys⁴-Glu⁵⁻Leu⁶-Cys⁷-Ile⁸-Asn⁹ -Met¹⁰-Ala¹¹-Cys¹²-Thr¹³-Gly¹⁴-Cys¹⁵-Ser¹⁶ | 165 |
| N89 | C4:C12,C7:C15 | Gln¹-Asp²-Asp³-Cys⁴-Glu⁵-Leu⁶-Cys⁷-Ile⁸-Asn⁹ -Met¹⁰-Ala¹¹-Cys¹²-Thr¹³-Gly¹⁴-Cys¹⁵-Ser¹⁶ | 166 |
| N90 | C4:C12,C7:C15 | Gln¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 167 |
| N91 | C4:C12,C7:C15 | Gln¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 168 |
| N92 | C4:C12,C7:C15 | Gln¹-Glu²⁻Glu³-Cys⁴-Glu⁵-Leu⁶-Cys⁷-Ile⁸-Asn⁹ -Met¹⁰-Ala¹¹-Cys¹²-Thr¹³-Gly¹⁴-Cys¹⁵-Ser¹⁶ | 169 |
| N93 | C4:C12,C7:C15 | Lys¹-Asp²-Asp³-Cys⁴-Glu⁵-Leu⁶-Cys⁷-Ile⁸-Asn⁹ -Met¹⁰-Ala¹¹-Cys¹²-Thr¹³-Gly¹⁴-Cys¹⁵-Ser¹⁶ | 170 |
| N94 | C4:C12,C7:C15 | Lys¹-Asp²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 171 |
| N95 | C4:C12,C7:C15 | Lys¹-Glu²-Asp³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 172 |
| N96 | C4:C12,C7:C15 | Lys¹-Glu²-Glu³-Cys⁴-Glu⁵-Leu⁶-Cys⁷-Ile⁸-Asn⁹-Met¹⁰-Ala¹¹-Cys¹²-Thr¹³-Gly¹⁴-Cys¹⁵-Ser¹⁶ | 173 |

**Table 6. Guanylin and Analogs**

| Name | Position of Disulfide bonds | Structure | SEQIDNO |
|---|---|---|---|
| Formula XIX | 4:12,7:15 | Xaa¹-Xaa²-Xaa³-Maa⁴-Xaa⁵-Xaa⁶-Maa⁷-Xaa⁸-Xaa⁹-Xaa¹⁰-Xaa¹¹-Maa¹²-Xaa¹³-Xaa¹⁴-Maa¹⁵ | 174 |
| Guanylin | C4:C 12,C7:C 15 | Ser¹-His²-Thr³-Cys⁴-Glu⁵-Ile⁶-Cys⁷-Ala⁸-Phe⁹-Ala¹⁰-Ala¹¹-Cys¹²-Ala¹³-Gly¹⁴-Cys¹⁵ | 175 |
| Human Guanylin | C4:C12, C7:C15 | Pro¹-Gly²-Thr³-Cys⁴-Glu⁵-Ile⁶-Cys⁷-Ala⁸-Tyr⁹-Ala¹⁰-Ala¹¹-Cys¹²-Thr¹³-Gly¹⁴-Cys¹⁵ | 252 |
| N97 | C4:C12,C7:C15 | Glu⁵-Ile⁶-Cys⁷-Ala⁸-Asn⁹-Ala¹⁰-Ala¹¹-Cys¹²-Ala¹³-Gly¹⁴-Cys¹⁵ | 176 |
| N98 | C4:C12,C7:C15 | Ser¹-His²-Thr³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 177 |
| N99 | C4:C12,C7:C15 | Ser¹-His²-Thr³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 178 |
| N100 | C4:C12,C7:C15 | Ser¹-His²-Thr³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 179 |
| N101 | C4:C12,C7:C15 | Ser¹-His²-Thr³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 180 |
| N102 | C4:C12,C7:C15 | Ser¹-His²-Thr³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 181 |
| N103 | C4:C12,C7:C15 | Ser¹-His²-Thr³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 182 |
| N104 | C4:C12,C7:C15 | Ser¹-His²-Thr³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 183 |
| N105 | C4:C12,C7:C15 | Ser¹-His²-Thr³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 184 |
| N106 | C4:C12,C7:C15 | Ser¹-His²-Thr³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 185 |
| N107 | C4:C12,C7:C15 | Ser¹-His²-Thr³-Cys⁴⁻Glu⁵-Val⁶-Cys⁷-Ala⁸-Asn⁹-Ala¹⁰-Ala¹¹-Cys¹²-Ala¹³-Gly¹⁴-Cys¹⁵ | 186 |
| N108 | C4:C12,C7:C15 | Ser¹-His²-Thr³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 187 |
| N109 | C4:C12,C7:C15 | Ser¹-His²-Thr³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 188 |
| N110 | C4:C12,C7:C15 | Ser¹-His²-Thr³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 189 |
| N111 | C4:C12,C7:C15 | Ser¹-His²-Thr³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 190 |
| N112 | C4:C12,C7:C15 | Ser¹-His²-Thr³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 191 |
| N113 | C4:C12,C7:C15 | Asn¹-Asp²-Glu³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 192 |
| N114 | C4:C12,C7:C15 | Asn¹-Asp²-Glu³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 193 |
| N115 | C4:C12,C7:C15 | Asn¹-Asp²-Glu³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 194 |
| N116 | C4:C12,C7:C15 | Asn¹-Asp²-Glu³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 195 |
| N117 | C4:C12,C7:C15 | Asn¹-Asp²-Glu³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 196 |
| N118 | C4:C12,C7:C15 | Asn¹-Asp²-Glu³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 197 |
| N119 | C4:C12,C7:C15 | Asn¹-Asp²-Glu³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 198 |
| N120 | C4:C12,C7:C15 | Asn¹-Asp²-Glu³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 199 |
| N121 | C4:C12,C7:C15 | Asn¹-Asp²-Glu³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 200 |
| N122 | C4:C12,C7:C15 | Asn¹-Asp²-Glu³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 201 |
| N123 | C4:C12,C7:C15 | Asn¹-Asp²-Glu³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 202 |
| N124 | C4:C12,C7:C15 | Asn¹-Asp²-Glu³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 203 |
| N125 | C4:C12,C7:C15 | Asn¹-Asp²-Glu³-Cys⁴-Glu⁵-Ile⁶-Cys⁷-Ala⁸-Asn⁹-Ala¹⁰-Ala¹¹-Cys¹²-Ala¹³-Gly¹⁴-Cys¹⁵ | 204 |
| N126 | C4:C12,C7:C15 | Asn¹-Asp²-Glu³-Cys⁴-Glu⁵-Leu⁶-Cys⁷-Ala⁸-Asn⁹-Ala¹⁰-Ala¹¹-Cys¹²-Ala¹³-Gly¹⁴-Cys¹⁵ | 205 |
| N127 | C4:C12,C7:C15 | Asn¹-Asp²-Glu³-Cys⁴-Glu⁵-Val⁶-Cys⁷-Ala⁸-Asn⁹-Ala¹⁰-Ala¹¹-Cys¹²-Ala¹³-Gly¹⁴-Cys¹⁵ | 206 |
| N128 | C4:C12,C7:C15 | Asn¹-Asp²-Glu³-Cys⁴-Glu⁵-Tyr⁶-Cys⁷-Ala⁸-Asn⁹-Ala¹⁰-Ala¹¹-Cys¹²-Ala¹³-Gly¹⁴-Cys¹⁵ | 207 |

**Table 7. Lymphoguanylin and Analogs**

| Name | Position of Disulfide bonds | Structure | SEQIDNO |
|---|---|---|---|
| F ormulaXX | 4:12 | Xaa¹-Xaa²-Xaa³-Maa⁴-Xaa⁵-Xaa⁶-Maa⁷-Xaa⁸-Xaa⁹-Xaa¹⁰-Xaa¹¹-Maa¹²-Xaa¹³-Xaa¹⁴-Xaaₙ₁¹⁵ | 208 |
| Lymphoguanylin | C4:C12 | Gln¹-Glu²-Glu-³Cys⁴-Glu⁵-Leu⁶-Cys⁷-Ile⁸-Asn⁹-Met¹⁰-Ala¹¹-Cys¹²-Thr¹³-Gly¹⁴-Tyr¹⁵ | 209 |
| N129 | C4:C12 | Gln¹-Glu²-Glu³-Cys⁴-Glu⁵-Thr⁶-Cys⁷-Ile⁸-Asn⁹-Met¹⁰-Ala¹¹-Cys¹²-Thr¹³-Gly¹⁴-Tyr¹⁵ | 210 |
| N130 | C4:C12 | Gln¹-Asp²-Glu³-Cys⁴-Glu⁵-Thr⁶-Cys⁷-Ile⁸-Asn⁹-Met¹⁰-Ala¹¹-Cys¹²-Thr¹³-Gly¹⁴-Tyr¹⁵ | 211 |
| N131 | C4:C12 | Gln¹-Asp²-Asp³-Cys⁴-Glu⁵-Thr⁶-Cys⁷-Ile⁸-Asn⁹-Met¹⁰-Ala¹¹-Cys¹²-Thr¹³-Gly¹⁴-Tyr¹⁵ | 212 |
| N132 | C4:C12 | Gln¹-Glu²-Asp³-Cys⁴-Glu⁵-Thr⁶-Cys⁷-Ile⁸-Asn⁹-Met¹⁰-Ala¹¹-Cys¹²-Thr¹³-Gly¹⁴-Tyr¹⁵ | 213 |
| N133 | C4:C12 | Gln¹-Glu²-Glu³-Cys⁴-Glu⁵-Glu⁶-Cys⁷-Ile⁸-Asn⁹-Met¹⁰-Ala¹¹-Cys¹²-Thr¹³-Gly¹⁴-Tyr¹⁵ | 214 |
| N134 | C4:C12 | Gln¹-Asp²-Glu³-Cys⁴-Glu⁵-Glu⁶-Cys⁷-Ile⁸-Asn⁹-Met¹⁰-Ala¹¹-Cys¹²-Thr¹³-Gly¹⁴-Tyr¹⁵ | 215 |
| N135 | C4:C12 | Gln¹-Asp²-Asp³-Cys⁴-Glu⁵-Glu⁶-Cys⁷-Ile⁸-Asn⁹-Met¹⁰-Ala¹¹-Cys¹²-Thr¹³-Gly¹⁴-Tyr¹⁵ | 216 |
| N136 | C4:C12 | Gln¹-Glu²-Asp³-Cys⁴-Glu⁵-Glu⁶-Cys⁷-Ile⁸-Asn⁹-Met¹⁰-Ala¹¹-Cys¹²-Thr¹³-Gly¹⁴-Tyr¹⁵ | 217 |
| N137 | C4:C12 | Gln¹-Glu²-Glu³-Cys⁴-Glu⁵-Tyr⁶-Cys⁷-Ile⁸-Asn⁹-Met¹⁰-Ala¹¹-Cys¹²-Thr¹³-Gly¹⁴-Tyr¹⁵ | 218 |
| N138 | C4:C12 | Gln¹-Asp²-Glu³-Cys⁴-Glu⁵-Tyr⁶-Cys⁷-Ile⁸-Asn⁹-Met¹⁰-Ala¹¹-Cys¹²-Thr¹³-Gly¹⁴-Tyr¹⁵ | 219 |
| N139 | C4:C12 | Gln¹-Asp²-Asp³-Cys⁴-Glu⁵-Tyr⁶-Cys⁷-Ile⁸-Asn⁹-Met¹⁰-Ala¹¹-Cys¹²-Thr¹³-Gly¹⁴-Tyr¹⁵ | 220 |
| N140 | C4:C12 | Gln¹-Glu²-Asp³-Cys⁴-Glu⁵-Tyr⁶-Cys⁷-Ile⁸-Asn⁹-Met¹⁰-Ala¹¹-Cys¹²-Thr¹³-Gly¹⁴-Tyr¹⁵ | 221 |
| N141 | C4:C12 | Gln¹-Glu²-Glu³-Cys⁴-Glu⁵-Ile⁶-Cys⁷-Ile⁸-Asn⁹-Met¹⁰-Ala¹¹-Cys¹²-Thr¹³-Gly¹⁴-Tyr¹⁵ | 222 |
| N142 | C4:C12 | Gln¹-Asp²-Glu³-Cys⁴-Glu⁵-Ile⁶-Cys⁷-Ile⁸-Asn⁹-Met¹⁰-Ala¹¹-Cys¹²-Thr¹³-Gly¹⁴-Tyr¹⁵ | 223 |
| N143 | C4:C12 | Gln¹-Asp²-Asp³-Cys⁴-Glu⁵-Ile⁶-Cys⁷-Ile⁸-Asn⁹-Met¹⁰-Ala¹¹-Cys¹²-Thr¹³-Gly¹⁴-Tyr¹⁵ | 224 |
| N144 | C4:C12 | Gln¹-Glu²-Asp³-Cys⁴-Glu⁵-Ile⁶-Cys⁷-Ile⁸-Asn⁹-Met¹⁰-Ala¹¹-Cys¹²-Thr¹³-Gly¹⁴-Tyr¹⁵ | 225 |
| N145 | C4:C12,C7 :C15 | Gln¹-Glu²-Glu³-Cys⁴-Glu⁵-Thr⁶-Cys⁷-Ile⁸-Asn⁹-Met¹⁰-Ala¹¹-Cys¹²-Thr¹³-Gly¹⁴-Cys¹⁵-Ser¹⁶ | 226 |
| N146 | C4:C12,C7 :C15 | Gln¹-Asp²-Glu³-Cys⁴-Glu⁵-Thr⁶-Cys⁷-Ile⁸-Asn⁹-Met¹⁰-Ala¹¹-Cys¹²-Thr¹³-Gly¹⁴-Cys¹⁵-Ser¹⁶ | 227 |
| N147 | C4:C12,C7 :C15 | Gln¹-Asp²-Asp³-Cys⁴-Glu⁵-Thr⁶-Cys⁷-Ile⁸-Asn⁹-Met¹⁰-Ala¹¹-Cys¹²-Thr¹³-Gly¹⁴-Cys¹⁵-Ser¹⁶ | 228 |
| N148 | C4:C12,C7 :C15 | Gln¹-Glu²-Asp³-Cys⁴-Glu⁵-Thr⁶-Cys⁷-Ile⁸-Asn⁹-Met¹⁰-Ala¹¹-Cys¹²-Thr¹³-Gly¹⁴-Cys¹⁵-Ser¹⁶ | 229 |
| N149 | C4:C12,C7 :C15 | Gln¹-Glu²-Glu³-Cys⁴-Glu⁵-Glu⁶-Cys⁷-Ile⁸-Asn⁹-Met¹⁰-Ala¹¹-Cys¹²-Thr¹³-Gly¹⁴-Cys¹⁵-Ser¹⁶ | 230 |
| N150 | C4:C12,C7 :C15 | Gln¹-Asp²-Glu³-Cys⁴-Glu⁵-Glu⁶-Cys⁷-Ile⁸-Asn⁹-Met¹⁰-Ala¹¹-Cys¹²-Thr¹³-Gly¹⁴-Cys¹⁵-Ser | 231 |
| N151 | C4:C12,C7 :C15 | Gln¹-Asp²-Asp³-Cys⁴-Glu⁵-Glu⁶-Cys⁷-Ile⁸-Asn⁹-Met¹⁰-Ala¹¹-Cys¹²-Thr¹³-Gly¹⁴-Cys¹⁵-Ser¹⁶ | 232 |
| N152 | C4:C12,C7 :C15 | Gln¹-Glu²-Asp³-Cys⁴-Glu⁵-Glu⁶-Cys⁷-Ile⁸-Asn⁹-Met¹⁰-Ala¹¹-Cys¹²-Thr¹³-Gly¹⁴-Cys¹⁵-Ser¹⁶ | 233 |
| N153 | C4:C12,C7 :C15 | Gln¹-Glu²-Glu³-Cys⁴-Glu⁵-Tyr⁶-Cys⁷-Ile⁸-Asn⁹-Met¹⁰-Ala¹¹-Cys¹²-Thr¹³-Gly¹⁴-Cys¹⁵-Ser¹⁶ | 234 |
| N154 | C4:C12,C7 :C15 | Gln¹-Asp²-Glu³-Cys⁴-Glu⁵-Tyr⁶-Cys⁷-Ile⁸-Asn⁹-Met¹⁰-Ala¹¹-Cys¹²-Thr¹³-Gly¹⁴-Cys¹⁵-Ser¹⁶ | 235 |
| N155 | C4:C12,C7 :C15 | Gln¹-Asp²-Asp³-Cys⁴-Glu⁵-Tyr⁶-Cys⁷-Ile⁸-Asn⁹-Met¹⁰-Ala¹¹-Cys¹²-Thr¹³-Gly¹⁴-Cys¹⁵-Ser¹⁶ | 236 |
| N156 | C4:C12,C7 :C15 | Gln¹-Glu²-Asp³-Cys⁴-Glu⁵-Tyr⁶-Cys⁷-Ile⁸-Asn⁹-Met¹⁰-Ala¹¹-Cys¹²-Thr¹³-Gly¹⁴-Cys¹⁵-Ser¹⁶ | 237 |
| N157 | C4:C12,C7 :C15 | Gln¹-Glu²-Glu³-Cys⁴-Glu⁵-Ile⁶-Cys⁷-Ile⁸-Asn⁹-Met¹⁰-Ala¹¹-Cys¹²-Thr¹³-Gly¹⁴-Cys¹⁵-Ser¹⁶ | 238 |
| N158 | C4:C12,C7 :C15 | Gln¹-Asp²-Glu³-Cys⁴-Glu⁵-Ile⁶-Cys⁷-Ile⁸-Asn⁹-Met¹⁰-Ala¹¹-Cys¹²-Thr¹³-Gly¹⁴-Cys¹⁵-Ser¹⁶ | 239 |
| N159 | C4:C12,C7 :C15 | Gln¹-Asp²-Asp³-Cys⁴-Glu⁵-Ile⁶-Cys⁷-Ile⁸-Asn⁹-Met¹⁰-Ala¹¹-Cys¹²-Thr¹³-Gly¹⁴-Cys¹⁵-Ser¹⁶ | 240 |
| N160 | C4:C12,C7 :C15 | Gln¹-Glu²-Asp³-Cys⁴-Glu⁵-Ile⁶-Cys⁷-Ile⁸-Asn⁹-Met¹⁰-Ala¹¹-Cys¹²-Thr¹³-Gly¹⁴-Cys¹⁵-Ser¹⁶ | 241 |

**Table 8. ST Peptide and Analogues**

| Name | Position of Disulfide bonds | Structure | SEQIDNO |
|---|---|---|---|
| STPeptide | C9:C14,C10:C18,C13:C21 | Asn1-Ser2-Ser3-Asn4-Ser5-Ser6-Asn7-Tyr8-Cys9-Cys10-Glu11-Lys12-Cys13-Cys14-Asn15-Pro16- Ala17-Cys18-Thr19-Gly20-Cys21-Tyr22 | 242 |
| N161 | C3:C8,C4:C12,C7:C15 | PEG3-Asn¹-Phe²-Cys³-Cys⁴-Glu⁵-Thr⁶-Cys⁷-Cys⁸-Asn⁹-Pro¹⁰-Ala¹¹-Cys¹²-Thr¹³-Gly¹⁴- Cys¹⁵-Tyr¹⁶-PEG3 | 243 |
| N162 | C3:C8,C4:C12,C7:C15 | PEG3-Asn¹-Phe²-Cys³-Cys⁴-Glu⁵-Thr⁶-Cys⁷-Cys⁸-Asn⁹-Pro¹⁰-Ala¹¹-Cys¹²-Thr¹³-Gly¹⁴- Cys¹⁵-Tyr¹⁶ | 244 |
| N163 | C3:C8,C4:C12,C7:C15 | Asn1-Phe2-Cys3-Cys4-Glu5-Thr6-Cys7-Cys8-Asn9-Pro10-Ala11-Cys12-Thr13-Gly14-Cys15- Tyr16-PEG3 | 245 |
| N164 | C3:C8,C4:C12,C7:C15 | Asn1-Phe2-Cys3-Cys4-Glu5-Tyr6-Cys7-Cys8-Asn9-Pro10-Ala11-Cys12-Thr13-Gly14-Cys15-Tyr16 | 246 |
| N165 | C3:C8,C4:C12,C7:C15 | dAsn1-Phe2-Cys3-Cys4-Glu5-Tyr6-Cys7-Cys8-Asn9-Pro10-Ala11-Cys12-Thr13-Gly14-Cys15- dTyr16 | 247 |
| N166 | C3:C8,C4:C12,C7:C15 | Asn1-Phe2-Cys3-Cys4-Glu5-Tyr6-Cys7-Cys8-Asn9-Pro10-Ala11-Cys12-Thr13-Gly14-Cys15- dTyr16 | 248 |
| N167 | C3:C8,C4:C12,C7:C15 | dAsn¹-Phe²-Cys³-Cys⁴-Glu⁵-Tyr⁶-Cys⁷-Cys⁸-Asn⁹-Pro¹⁰-Ala¹¹-Cys¹²-Thr¹³-Gly¹⁴-Cys¹⁵- Tyr¹⁶ | 249 |

The term "consisting essentially of' includes peptides that are identical to a recited sequence (any one from Tables 2-8) and other sequences that do not differ substantially in terms of either structure or function. For the purpose of the present application, a peptide differs substantially if its structure varies by more than three amino acids from a peptide of any one from Tables 2-8 or if its activation of cellular cGMP production is reduced or enhanced by more than 50%. Preferably, substantially similar peptides should differ by no more than two amino acids and not differ by more than about 25% with respect to activating cGMP production.

Also included within the meaning of substantially homologous is any GCRA peptide which may be isolated by virtue of cross-reactivity with antibodies to the GCRA peptide.

### PREPARATION OF GCRA PEPTIDES

GCRA peptides are easily prepared using modern cloning techniques, or may be synthesized by solid state methods or by site-directed mutagenesis. A GCRA peptide may include dominant negative forms of a polypeptide.

Chemical synthesis may generally be performed using standard solution phase or solid phase peptide synthesis techniques, in which a peptide linkage occurs through the direct condensation of the amino group of one amino acid with the carboxy group of the other amino acid with the elimination of a water molecule. Peptide bond synthesis by direct condensation, as formulated above, requires suppression of the reactive character of the amino group of the first and of the carboxyl group of the second amino acid. The masking substituents must permit their ready removal, without inducing breakdown of the labile peptide molecule.

In solution phase synthesis, a wide variety of coupling methods and protecting groups may be used (*See,* Gross and Meienhofer, eds., "The Peptides: Analysis, Synthesis, Biology," Vol. 1-4 (Academic Press, 1979); Bodansky and Bodansky, "The Practice of Peptide Synthesis," 2d ed. (Springer Verlag, 1994)). In addition, intermediate purification and linear scale up are possible. Those of ordinary skill in the art will appreciate that solution synthesis requires consideration of main chain and side chain protecting groups and activation method. In addition, careful segment selection is necessary to minimize racemization during segment condensation. Solubility considerations are also a factor. Solid phase peptide synthesis uses an insoluble polymer for support during organic synthesis. The polymer-supported peptide chain permits the use of simple washing and filtration steps instead of laborious purifications at intermediate steps. Solid-phase peptide synthesis may generally be performed according to the method of Merrifield et al., J. Am. Chem. Soc., 1963, 85:2149, which involves assembling a linear peptide chain on a resin support using protected amino acids. Solid phase peptide synthesis typically utilizes either the Boc or Fmoc strategy, which is well known in the art.

Those of ordinary skill in the art will recognize that, in solid phase synthesis, deprotection and coupling reactions must go to completion and the side-chain blocking groups must be stable throughout the synthesis. In addition, solid phase synthesis is generally most suitable when peptides are to be made on a small scale.

Acetylation of the N-terminal can be accomplished by reacting the final peptide with acetic anhydride before cleavage from the resin. C-amidation is accomplished using an appropriate resin such as methylbenzhydrylamine resin using the Boc technology.

Alternatively, the GCRA peptides are produced by modern cloning techniques. For example, the GCRA peptides are produced either in bacteria including, without limitation, E. coli, or in other existing systems for polypeptide or protein production (*e.g*., Bacillus subtilis, baculovirus expression systems using Drosophila Sf9 cells, yeast or filamentous fungal expression systems, mammalian cell expression systems), or they can be chemically synthesized. If the GCRA peptide or variant peptide is to be produced in bacteria, *e.g.,* E. coli, the nucleic acid molecule encoding the polypeptide may also encode a leader sequence that permits the secretion of the mature polypeptide from the cell. Thus, the sequence encoding the polypeptide can include the pre-sequence and the pro sequence of, for example, a naturally-occurring bacterial ST polypeptide. The secreted, mature polypeptide can be purified from the culture medium.

The sequence encoding a GCRA peptide described herein can be inserted into a vector capable of delivering and maintaining the nucleic acid molecule in a bacterial cell. The DNA molecule may be inserted into an autonomously replicating vector (suitable vectors include, for example, pGEM3Z and pcDNA3, and derivatives thereof). The vector nucleic acid may be a bacterial or bacteriophage DNA such as bacteriophage lambda or M13 and derivatives thereof. Construction of a vector containing a nucleic acid described herein can be followed by transformation of a host cell such as a bacterium. Suitable bacterial hosts include but are not limited to, E. coli, B subtilis, Pseudomonas, Salmonella. The genetic construct also includes, in addition to the encoding nucleic acid molecule, elements that allow expression, such as a promoter and regulatory sequences. The expression vectors may contain transcriptional control sequences that control transcriptional initiation, such as promoter, enhancer, operator, and repressor sequences.

A variety of transcriptional control sequences are well known to those in the art. The expression vector can also include a translation regulatory sequence (*e.g*., an untranslated 5' sequence, an untranslated 3' sequence, or an internal ribosome entry site). The vector can be capable of autonomous replication or it can integrate into host DNA to ensure stability during polypeptide production.

The protein coding sequence that includes a GCRA peptide described herein can also be fused to a nucleic acid encoding a polypeptide affinity tag, *e.g*., glutathione S-transferase (GST), maltose E binding protein, protein A, FLAG tag, hexa-histidine, myc tag or the influenza HA tag, in order to facilitate purification. The affinity tag or reporter fusion joins the reading frame of the polypeptide of interest to the reading frame of the gene encoding the affinity tag such that a translational fusion is generated. Expression of the fusion gene results in translation of a single polypeptide that includes both the polypeptide of interest and the affinity tag. In some instances where affinity tags are utilized, DNA sequence encoding a protease recognition site will be fused between the reading frames for the affinity tag and the polypeptide of interest.

Genetic constructs and methods suitable for production of immature and mature forms of the GCRA peptides and variants described herein in protein expression systems other than bacteria, and well known to those skilled in the art, can also be used to produce polypeptides in a biological system.

The peptides disclosed herein may be modified by attachment of a second molecule that confers a desired property upon the peptide, such as increased half-life in the body, for example, pegylation. Such modifications also fall within the scope of the term "variant" as used herein.

By "inhibiting" or "inhibition" or "reduce", it means the GCRA peptide decreases the activity and/or production of a protein by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%, or more relative to the activity and/or production of the protein without the GCRA peptide.

By "induce", it means the GCRA peptide increases the activity and/or production of a protein by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%, or more relative to the activity and/or production of the protein without the GCRA peptide.

The term "treatment" or "treating" refers to reducing or alleviating symptoms in a subject, preventing symptoms from worsening or progressing, and/or preventing disease in a subject who is free therefrom. For a given subject, improvement, worsening, regression, or progression of a symptom may be determined by any objective or subjective measure. Efficacy of the treatment may be measured as an improvement in morbidity or mortality (*e.g*., lengthening of survival curve for a selected population). Thus, effective treatment would include therapy of existing disease, control of disease by slowing or stopping its progression, prevention of disease occurrence, reduction in the number or severity of symptoms, or a combination thereof. The effect may be shown in a controlled study using one or more statistically significant criteria.

The term "prevention" in relation to a given disease or disorder means: preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

Intracellular cGMP produced by exposing, *e.g.,* contacting a tissue (*e.g.,* gastrointestinal tissue; lung tissue, esophageal tissue) or cell with GCRA agonists. By inducing is meant an increase in cGMP production compared to a tissue or cell that has not been in contact with GCRA peptide or variant. Tissues or cells are directly contacted with a GCRA peptide or variant. Alternatively, the GCRA peptide or variant is administered systemically. GCRA peptide or variant are administered in an amount sufficient to increase intracellular cGMP concentration. cGMP production is measured by a cell-based assay known in the art (25).

Disorders are treated, prevented or alleviated by administering to a subject, *e.g*., a mammal such as a human in need thereof, a therapeutically effective dose of a GCRA peptide. The GCRA peptides may be in a pharmaceutical composition in unit dose form, together with one or more pharmaceutically acceptable excipients. The term "unit dose form" refers to a single drug delivery entity, *e.g.,* a tablet, capsule, solution or inhalation formulation. The amount of peptide present should be sufficient to have a positive therapeutic effect when administered to a patient (typically, between 10 µg and 3 g). What constitutes a "positive therapeutic effect" will depend upon the particular condition being treated and will include any significant improvement in a condition readily recognized by one of skill in the art.

The GCRA peptides can be administered alone or in combination with an opioid. Opioids useful for analgesia are known in the art. For example, opioid compounds include, but are not limited to, alfentanil, anileridine, asimadoline, bremazocine, burprenorphine, butorphanol, codeine, dezocine, diacetylmorphine (heroin), dihydrocodeine, diphenoxylate, ethylmorphine, fedotozine, fentanyl, funaltrexamine, hydrocodone, hydromorphone, levallorphan, levomethadyl acetate, levorphanol, loperamide, meperidine (pethidine), methadone, morphine, morphine-6-glucoronide, nalbuphine, nalorphine, nicomorphine, opium, oxycodone, oxymorphone, papavereturn, pentazocine, propiram, propoxyphene, remifentanyl, sufentanil, tilidine, trimebutine, and tramadol. In some embodiments the opioid is at least one opioid selected from alfentanil, buprenorphine, butorphanol, codeine, dezocine, dihydrocodeine, fentanyl, hydrocodone, hydromorphone, levorphanol, meperidine (pethidine), methadone, morphine, nalbuphine, nicomorphine, oxycodone, oxymorphone, papavereturn, pentazocine, propiram, propoxyphene, sufentanil and/or tramadol. In certain embodiments of the present disclosure, the opioid is selected from morphine, codeine, oxycodone, hydrocodone, dihydrocodeine, propoxyphene, fentanyl, tramadol, and mixtures thereof. In a particular embodiment, the opioid is loperamide. In other embodiments, the opioid is a mixed agonist such as butorphanol. In some embodiments, the subjects are administered more than one opioid, for example, morphine and heroin or methadone and heroin.

The GCRA peptides can be administered alone or in combination with another therapeutic agent, for example, those to treat, prevent, or ameliorate symptoms of opioid-induced dysfunction. Exemplary therapeutic agents, include but are not limited to oral laxatives (e.g. softening or peristalsis-inducing agents), suppositories, enemas, opioid antagonists (e.g. Naloxone, nalmefene, methylnaltrexone), polyethylene glycol, drugs that selectively target peripheral mu-opioid receptors (e.g. methylnaltrexone, alvimopan), type 2 chloride channel activators (e.g. lubiprostone), dopamine receptor antagonists (e.g. metoclopramide), nonopioids, 5-Hydroxytryptamine (Serotonin, 5HT) Receptor Ligands, ampakines, N-methyl-D-aspartate receptor inhibitors, AMPA receptor modulators, Tapentadol, NKTR-118, TD-1211, and/or microglial Inhibitors (e.g. Minocycline).

The term "combination therapy" means administering two or more active agents concurrently or sequentially. Concurrent administration may be achieved with a formulation in which two or more active agents are mixed, or with simultaneous administration of two or more active agents formulated independently. Sequential administration of two or more active agents may be achieved with two or more active agents, formulated independently, administered in sequence with one agent administered first followed by the second agent administered seconds, minutes, hours, or days after the first agent.

Combination therapy can be achieved by administering two or more agents, *e.g.,* a GCRA peptide described herein or a composition described herein and another compound, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or 10 weeks of each other. In some cases even longer intervals are possible. While in many cases it is desirable that the two or more agents used in a combination therapy be present in within the patient's body at the same time, this need not be so.

The GCRA peptides described herein may be combined with phosphodiesterase inhibitors, *e.g.,* sulindae sulfone, Zaprinast, sildenafil, vardenafil or tadalafil to further enhance levels of cGMP in the target tissues or organs.

Combination therapy can also include two or more administrations of one or more of the agents used in the combination. For example, if agent X and agent Y are used in a combination, one could administer them sequentially in any combination one or more times, *e.g.,* in the order X-Y-X, X-X-Y, Y-X-Y,Y-Y-X,X-X-Y-Y, etc.

Combination therapy can also include the administration of two or more agents via different routes or locations. For example, (a) one agent is administered orally and another agent is administered intravenously or (b) one agent is administered orally and another is administered locally. In each case, the agents can either simultaneously or sequentially. Approximated dosages for some of the combination therapy agents described herein are found in the "BNF Recommended Dose" column of tables on pages 11-17 of WO01/76632 (the data in the tables being attributed to the March 2000 British National Formulary) and can also be found in other standard formularies and other drug prescribing directories. For some drugs, the customary presecribed dose for an indication will vary somewhat from country to country.

The GCRA peptides, alone or in combination, can be combined with any pharmaceutically acceptable carrier or medium. Thus, they can be combined with materials that do not produce an adverse, allergic or otherwise unwanted reaction when administered to a patient. The carriers or mediums used can include solvents, dispersants, coatings, absorption promoting agents, controlled release agents, and one or more inert excipients (which include starches, polyols, granulating agents, microcrystalline cellulose (*e.g.* celphere, Celphere beads®), diluents, lubricants, binders, disintegrating agents, and the like), etc. If desired, tablet dosages of the disclosed compositions may be coated by standard aqueous or nonaqueous techniques.

A pharmaceutical composition of the disclosure is formulated to be compatible with its intended route of administration. Examples of routes of administration include parenteral, *e.g.,* intravenous, intradermal, subcutaneous, oral (*e.g.,* inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include: sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.,* a GCRA agonist) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. Such as mannitol, fructooligosaccharides, polyethylene glycol and other excepients. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.,* with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

The pharmaceutical compositions can be included in a container, pack, or dispenser together with instructions for administration.

Compositions of the present disclosure may also optionally include other therapeutic ingredients, anti-caking agents, preservatives, sweetening agents, colorants, flavors, desiccants, plasticizers, dyes, glidants, anti-adherents, anti-static agents, surfactants (wetting agents), anti-oxidants, film- coating agents, and the like. Any such optional ingredient must be compatible with the compound described herein to insure the stability of the formulation.

The composition may contain other additives as needed, including for exanple lactose, glucose, fructose, galactose, trehalose, sucrose, maltose, raffnose, maltitol, melezitose, stachyose, lactitol, palatinite, starch, xylitol, mannitol, myoinositol, and the like, and hydrates thereof, and amino acids, for example alanine, glycine and betaine, and polypeptides and proteins, for example albumen.

Examples of excipients for use as the pharmaceutically acceptable carriers and the pharmaceutically acceptable inert carriers and the aforementioned additional ingredients include, but are not limited to binders, fillers, disintegrants, lubricants, anti-microbial agents, and coating agents such as: BINDERS: corn starch, potato starch, other starches, gelatin, natural and synthetic gums such as acacia, xanthan, sodium alginate, alginic acid, other alginates, powdered tragacanth, guar gum, cellulose and its derivatives (*e.g.,* ethyl cellulose, cellulose acetate, carboxymethyl cellulose calcium, sodium carboxymethyl cellulose), polyvinyl pyrrolidone (*e.g.,* povidone, crospovidone, copovidone, etc), methyl cellulose, Methocel, pre-gelatinized starch (*e.g.,* STARCH 1500® and STARCH 1500 LM®, sold by Colorcon, Ltd.), hydroxypropyl methyl cellulose, microcrystalline cellulose (FMC Corporation, Marcus Hook, PA, USA), or mixtures thereof, FILLERS: talc, calcium carbonate (*e.g.,* granules or powder), dibasic calcium phosphate, tribasic calcium phosphate, calcium sulfate (*e.g.,* granules or powder), microcrystalline cellulose, powdered cellulose, dextrates, kaolin, mannitol, silicic acid, sorbitol, starch, pre-gelatinized starch, dextrose, fructose, honey, lactose anhydrate, lactose monohydrate, lactose and aspartame, lactose and cellulose, lactose and microcrystalline cellulose, maltodextrin, maltose, mannitol, microcrystalline cellulose & guar gum, molasses, sucrose,or mixtures thereof, DISINTEGRANTS: agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums (like gellan), low-substituted hydroxypropyl cellulose, or mixtures thereof, LUBRICANTS: calcium stearate, magnesium stearate, mineral oil, light mineral oil, glycerin, sorbitol, mannitol, polyethylene glycol, other glycols, stearic acid, sodium lauryl sulfate, sodium stearyl fumarate, vegetable based fatty acids lubricant, talc, hydrogenated vegetable oil (*e.g.,* peanut oil, cottonseed oil, sunflower oil, sesame oil, olive oil, corn oil and soybean oil), zinc stearate, ethyl oleate, ethyl laurate, agar, syloid silica gel (AEROSIL 200, W.R. Grace Co., Baltimore, MD USA), a coagulated aerosol of synthetic silica (Deaussa Co., Piano, TX USA), a pyrogenic silicon dioxide (CAB-O-SIL, Cabot Co., Boston, MA USA), or mixtures thereof, ANTI-CAKING AGENTS: calcium silicate, magnesium silicate, silicon dioxide, colloidal silicon dioxide, talc, or mixtures thereof, ANTIMICROBIAL AGENTS: benzalkonium chloride, benzethonium chloride, benzoic acid, benzyl alcohol, butyl paraben, cetylpyridinium chloride, cresol, chlorobutanol, dehydroacetic acid, ethylparaben, methylparaben, phenol, phenylethyl alcohol, phenoxyethanol, phenylmercuric acetate, phenylmercuric nitrate, potassium sorbate, propylparaben, sodium benzoate, sodium dehydroacetate, sodium propionate, sorbic acid, thimersol, thymo, or mixtures thereof, and COATING AGENTS: sodium carboxymethyl cellulose, cellulose acetate phthalate, ethylcellulose, gelatin, pharmaceutical glaze, hydroxypropyl cellulose, hydroxypropyl methylcellulose (hypromellose), hydroxypropyl methyl cellulose phthalate, methylcellulose, polyethylene glycol, polyvinyl acetate phthalate, shellac, sucrose, titanium dioxide, carnauba wax, microcrystalline wax, gellan gum, maltodextrin, methacrylates, microcrystalline cellulose and carrageenan or mixtures thereof.

The formulation can also include other excipients and categories thereof including but not limited to L-histidine, Pluronic®, Poloxamers (such as Lutrol® and Poloxamer 188), ascorbic acid, glutathione, permeability enhancers (*e.g.* lipids, sodium cholate, acylcarnitine, salicylates, mixed bile salts, fatty acid micelles, chelators, fatty acid, surfactants, medium chain glycerides), protease inhibitors (*e.g.* soybean trypsin inhibitor, organic acids), pH lowering agents and absorption enhancers effective to promote bioavailability (including but not limited to those described in US6086918 and US5912014), creams and lotions (like maltodextrin and carrageenans); materials for chewable tablets (like dextrose, fructose, lactose monohydrate, lactose and aspartame, lactose and cellulose, maltodextrin, maltose, mannitol, microcrystalline cellulose and guar gum, sorbitol crystalline); parenterals (like mannitol and povidone); plasticizers (like dibutyl sebacate, plasticizers for coatings, polyvinylacetate phthalate); powder lubricants (like glyceryl behenate); soft gelatin capsules (like sorbitol special solution); spheres for coating (like sugar spheres); spheronization agents (like glyceryl behenate and microcrystalline cellulose); suspending/gelling agents (like carrageenan, gellan gum, mannitol, microcrystalline cellulose, povidone, sodium starch glycolate, xanthan gum); sweeteners (like aspartame, aspartame and lactose, dextrose, fructose, honey, maltodextrin, maltose, mannitol, molasses, sorbitol crystalline, sorbitol special solution, sucrose); wet granulation agents (like calcium carbonate, lactose anhydrous, lactose monohydrate, maltodextrin, mannitol, microcrystalline cellulose, povidone, starch), caramel, carboxymethylcellulose sodium, cherry cream flavor and cherry flavor, citric acid anhydrous, citric acid, confectioner's sugar, D&C Red No. 33, D&C Yellow #10 Aluminum Lake, disodium edetate, ethyl alcohol 15%, FD&C Yellow No. 6 aluminum lake, FD&C Blue # 1 Aluminum Lake, FD&C Blue No. 1, FD&C blue no. 2 aluminum lake, FD&C Green No.3, FD&C Red No. 40, FD&C Yellow No. 6 Aluminum Lake, FD&C Yellow No. 6, FD&C Yellow No. 10, glycerol palmitostearate, glyceryl monostearate, indigo carmine, lecithin, manitol, methyl and propyl parabens, mono ammonium glycyrrhizinate, natural and artificial orange flavor, pharmaceutical glaze, poloxamer 188, Polydextrose, polysorbate 20, polysorbate 80, polyvidone, pregelatinized corn starch, pregelatinized starch, red iron oxide, saccharin sodium, sodium carboxymethyl ether, sodium chloride, sodium citrate, sodium phosphate, strawberry flavor, synthetic black iron oxide, synthetic red iron oxide, titanium dioxide, and white wax.

Solid oral dosage forms may optionally be treated with coating systems (*e.g*. Opadry® fx film coating system, for example Opadry® blue (OY-LS-20921), Opadry® white (YS-2-7063), Opadry® white (YS- 1-7040), and black ink (S- 1-8 106).

The agents either in their free form or as a salt can be combined with a polymer such as polylactic-glycoloic acid (PLGA), poly-(I)-lactic-glycolic-tartaric acid (P(I)LGT) (WO 01/12233), polyglycolic acid (U.S. 3,773,919), polylactic acid (U.S. 4,767,628), poly(ε-caprolactone) and poly(alkylene oxide) (U.S. 20030068384) to create a sustained release formulation. Such formulations can be used to implants that release a polypeptide or another agent over a period of a few days, a few weeks or several months depending on the polymer, the particle size of the polymer, and the size of the implant (*See, e.g.,* U.S. 6,620,422). Other sustained release formulations and polymers for use in are described in EP 0 467 389 A2, WO 93/24150, U.S. 5,612,052, WO 97/40085, WO 03/075887, WO 01/01964A2, U.S. 5,922,356, WO 94/155587, WO 02/074247A2, WO 98/25642, U.S. 5,968,895, U.S. 6,180,608, U.S. 20030171296, U.S. 20020176841, U.S. 5,672,659, U.S. 5,893,985, U.S. 5,134,122, U.S. 5,192,741, U.S. 5,192,741, U.S. 4,668,506, U.S. 4,713,244, U.S. 5,445,832 U.S. 4,931,279, U.S. 5,980,945, WO 02/058672, WO 97/26015, WO 97/04744, and US20020019446. In such sustained release formulations microparticles (Delie and Blanco-Prieto 2005 Molecule 10:65-80) of polypeptide are combined with microparticles of polymer. One or more sustained release implants can be placed in the large intestine, the small intestine or both. U.S. 6,011,011 and WO 94/06452 describe a sustained release formulation providing either polyethylene glycols (*i.e.* PEG 300 and PEG 400) or triacetin. WO 03/053401 describes a formulation which may both enhance bioavailability and provide controlled releaseof the agent within the GI tract. Additional controlled release formulations are described in WO 02/38129, EP 326151, U.S. 5,236,704, WO 02/30398, WO 98/13029; U.S. 20030064105, U.S. 20030138488A1, U.S. 20030216307A1, U.S. 6,667,060, WO 01/49249, WO 01/49311, WO 01/49249, WO 01/49311, and U.S. 5,877,224 materials which may include those described in WO04041195 (including the seal and enteric coating described therein) and pH-sensitive coatings that achieve delivery in the colon including those described in US4,910,021 and WO9001329. US4910021 describes using a pH-sensitive material to coat a capsule. WO9001329 describes using pH-sensitive coatings on beads containing acid, where the acid in the bead core prolongs dissolution of the pH-sensitive coating. U. S. Patent No. 5,175,003 discloses a dual mechanism polymer mixture composed of pH-sensitive enteric materials and film-forming plasticizers capable of conferring permeability to the enteric material, for use in drug-delivery systems; a matrix pellet composed of a dual mechanism polymer mixture permeated with a drug and sometimes covering a pharmaceutically neutral nucleus; a membrane- coated pellet comprising a matrix pellet coated with a dual mechanism polymer mixture envelope of the same or different composition; and a pharmaceutical dosage form containing matrix pellets. The matrix pellet releases acid-soluble drugs by diffusion in acid pH and by disintegration at pH levels of nominally about 5.0 or higher.

In some embodiments, the compositions described herein are formulated in a pH dependent release form. Alternatively, such compositions are formulated in a form that releases the peptides at a specific region of the gastrointestinal (GI) tract (*e.g.,* duodenum, jejunum, ileum, terminal ileum, or ascending colon). The formulation may contain an inert carrier coated with a composition and an enteric coating which releases the peptides at a specific pH (such as pH5 or pH7). Preferred pH for duodenum or jejunum release is pH 4.5-5.5 or pH 5.5-6.5. Preferred pH for ileum, terminal ileum, or ascending colon release is pH 5.5-6.5 or pH 6.5-7.5. Preferably, the inert carrier is a selected from mannitol, lactose, a microcrystalline cellulose, or starch.

The GCRA peptideds described herein may be formulated in the pH triggered targeted control release systems described in WO04052339. The agents described herein may be formulated according to the methodology described in any of WO03105812 (extruded hyrdratable polymers); WO0243767 (enzyme cleavable membrane translocators); WO03007913 and WO03086297 (mucoadhesive systems); WO02072075 (bilayer laminated formulation comprising pH lowering agent and absorption enhancer); WO04064769 (amidated polypeptides); WO05063156 (solid lipid suspension with pseudotropic and/or thixotropic properties upon melting); WO03035029 and WO03035041 (erodible, gastric retentive dosage forms); US5007790 and US5972389 (sustained release dosage forms); WO041 1271 1 (oral extended release compositions); WO05027878, WO02072033, and WO02072034 (delayed release compositions with natural or synthetic gum); WO05030182 (controlled release formulations with an ascending rate of release); WO05048998 (microencapsulation system); US Patent 5,952,314 (biopolymer); US5,108,758 (glassy amylose matrix delivery); US 5,840,860 (modified starch based delivery). JP10324642 (delivery system comprising chitosan and gastric resistant material such as wheat gliadin or zein); US 5,866,619 and US 6,368,629 (saccharide containing polymer); US 6,531,152 (describes a drug delivery system containing a water soluble core (Ca pectinate or other water-insoluble polymers) and outer coat which bursts (*e.g.* hydrophobic polymer-Eudragrit)); US 6,234,464; US 6,403,130 (coating with polymer containing casein and high methoxy pectin; WO0174 175 (Maillard reaction product); WO05063206 (solubility increasing formulation); WO040 19872 (transferring fusion proteins).

### CONTROLLED RELEASE FORMULATIONS

In one embodiment, the GCC agonist formulation comprises a targeting material which provides a controlled (time-dependent) release of the GCC agonist. Controlled release in this context includes delayed sustained release, delayed controlled release, delayed slow release, delayed prolonged release, delayed extended release, and a sudden release or "burst."

Preferably, the controlled release formulation comprises a slowly disintegrating core comprising the GCC agonist surrounded by the targeting material. The targeting material preferably comprises at least one swellable polymer. Non-limiting examples of swellable polymers for use in a controlled release formulation of the disclosure include acrylic copolymers, e.g., EUDRAGIT RL, EUDRAGIT RS, or EUDRAGIT NE; polyvinylacetate, e.g., KOLLICOAT SR 30D; and cellulose derivatives such as ethylcellulose or cellulose acetate, e.g., SURELEASE and AQUACOAT ECD. In a preferred embodiment, the targeting material comprises one or more of EUDRAGIT RL, EUDRAGIT RS, or EUDRAGIT NE to provide controlled time release of the GCC agonist by pH-independent swelling. In a particular embodiment, the targeting material comprises EUDRAGIT RL:RS (2:8) and an outing coating comprising EUDRAGIT FS.

Further non-limiting examples of swellable polymers that can be used in the sustained release formulations include poly(hydroxalkyl methacrylate) having a molecular weight of from 30,000 to 5,000.000; kappa-carrageenan; polyvinylpyrrolidone having a molecular weight of from 10,000 to 360,000; anionic and cationic hydrogels; polyelectrolyte complexes; poly(vinyl alcohol) having low amounts of acetate, cross-linked with glyoxal, formaldehyde, or glutaraldehyde and having a degree of polymerization from 200 to 30,000; a mixture comprising methyl cellulose, cross-linked agar and carboxymethyl cellulose; a water-insoluble, water-swellable copolymer produced by forming a dispersion of finely divided maleic anhydride with styrene, ethylene, propylene, butylene or isobutylene; water-swellable polymers of N-vinyl lactams; polysaccharide, water swellable gums, high viscosity hydroxyipropylmethyl cellulose and/or mixtures thereof. In certain embodiments, the swellable polymer is selected from the group consisting of calcium pectinate, cross-linked polysaccharide, water insoluble starch, microcrystalline cellulose, water insoluble cross-linked peptide, water insoluble cross-linked protein, water insoluble cross-linked gelatin, water insoluble cross-linked hydrolyzed gelatin, water insoluble cross-linked collagen, modified cellulose, and cross-linked polyacrylic acid. Non-limiting examples of a cross-linked polysaccharide include insoluble metal salts or cross-linked derivatives of alginate, pectin, xantham gum, guar gum, tragacanth gum, and locust bean gum, carrageenan, metal salts thereof, and covalently cross-linked derivatives thereof. Non-limiting examples of modified cellulose include cross-linked derivatives of hydroxypropylcellulose, hydroxypropylmethylcellulose, hydroxyethylcellulose, methylcellulose, carboxymethylcellulose, and metal salts of carboxymethylcellulose.

In certain embodiments, the swellable core also comprises a wicking agent such as silicon dioxide. The wicking agent may also be selected from a disintegrant such as microcrystalline cellulose to enhance the speed of water uptake. Other suitable wicking agents include, but are not limited to, kaolin, titanium dioxide, fumed silicon dioxide, alumina, niacinamide, sodium lauryl sulfate, low molecular weight polyvinyl pyrrolidone, m-pyrol, bentonite, magnesium aluminum silicate, polyester, polyethylene, and mixtures thereof.

In certain embodiments, the targeting material, which may comprise part of the core and/or form one or more layers coating the core, optionally further comprises at least one of a lubricant, a flow promoting agent, a plasticizer, an anti-sticking agent, surfactant, wetting agent, suspending agent and dispersing agent.

In certain embodiments, the targeting material comprises a water insoluble polymer and a pore-forming agent. Non-limiting examples of pore forming agents include saccharose, sodium chloride, potassium chloride, polyvinylpyrrolidone, and/or polyethyleneglycol, water soluble organic acids, sugars and sugar alcohol. In certain embodiments, the pore forming agent forms part of an outer layer or coating. In other embodiments, the pore forming agent is distributed uniformly throughout the water insoluble polymer.

In one embodiment, the targeting material comprises a compression coating. Non-limiting examples of materials that can be used as a compression coating include a gum selected from the group consisting of xanthan gum, locust bean gum, galactans, mannans, alginates, gum karaya, pectin, agar, tragacanth, acacia, carrageenan, tragacanth, chitosan, agar, alginic acid, hydrocolloids acacia catechu, salai guggal, indian bodellum, copaiba gum, asafetida, cambi gum, Enterolobium cyclocarpum, mastic gum, benzoin gum, sandarac, gambier gum, butea frondosa (Flame of Forest Gum), myrrh, konjak mannan, guar gum, welan gum, gellan gum, tara gum, locust bean gum, carageenan gum, glucomannan, galactan gum, sodium alginate, tragacanth, chitosan, xanthan gum, deacetylated xanthan gum, pectin, sodium polypectate, gluten, karaya gum, tamarind gum, ghatti gum, Accaroid/Yacca/Red gum, dammar gum, juniper gum, ester gum, ipil-ipil seed gum, gum talha (acacia seyal), and cultured plant cell gums including those of the plants of the genera: acacia, actinidia, aptenia, carbobrotus, chickorium, cucumis, glycine, hibiscus, hordeum, letuca, lycopersicon, malus, medicago, mesembryanthemum, oryza, panicum, phalaris, phleum, poliathus, polycarbophil, sida, solanum, trifolium, trigonella, Afzelia africana seed gum, Treculia africana gum, detarium gum, cassia gum, carob gum, Prosopis africana gum, Colocassia esulenta gum, Hakea gibbosa gum, khaya gum, scleroglucan, and zea, as well as mixtures of any of the foregoing.

In some embodiments, the targeting material further comprises a plasticizer, a stiffening agent, a wetting agent, a suspending agent, or a dispersing agent, or a combination thereof. Non-limiting examples of a plasticizer include dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, butyl and/or glycol esters of fatty acids, refined mineral oils, oleic acid, castor oil, corn oil, camphor, glycerol and sorbitol or a combination thereof. In one embodiment, the stiffening agent comprises cetyl alcohol. Non-limiting examples of wetting agents include a poloxamer, polyoxyethylene ethers, polyoxyethylene sorbitan fatty acid esters, polyoxymethylene stearate, sodium lauryl sulfate, sorbitan fatty acid esters, benzalkonium chloride, polyethoxylated castor oil, and docusate sodium. Non-limiting examples of suspending agents include alginic acid, bentonite, carbomer, carboxymethylcellulose, carboxymethylcellulose calcium, hydroxyethylcellulose, hydroxypropylcellulose, microcrystalline cellulose, colloidal silicon dioxide, dextrin, gelatin, guar gum, xanthan gum, kaolin, magnesium aluminum silicate, maltitol, medium chain triglycerides, methylcellulose, polyoxyethylene sorbitan fatty acid esters, polyvinylpyrrolidinone, propylene glycol alginate, sodium alginate, sorbitan fatty acid esters, and tragacanth. Non-limiting examples of dispersing agents include poloxamer, polyoxyethylene sorbitan fatty acid esters and sorbitan fatty acid esters.

In certain embodiments, the targeted release formulation further comprises an outer enteric coating over the targeted release material. Preferably, the enteric coating is selected from the group consisting of cellulose acetate phthalate, hydroxy propyl methyl cellulose acetate succinate, EUDRAGIT LI00 and EUDRAGIT L30D-55.

### BURST FORMULATION

In one embodiment, the GCC agonist formulation is a time-delayed formulation designed to release the GCC agonist in a fast burst in the colon or small intestine ("burst formulation"). The formulation comprises a core and an outer layer. The core comprises at least one GCC agonist and at least one burst controlling agent. In certain embodiments, the core further comprises at least one disintegrant selected from the group consisting of croscarmellose sodium, crospovidone (cross-linked PVP), sodium carboxymethyl starch (sodium starch glycolate), cross-linked sodium carboxymethyl cellulose (Croscarmellose), pregelatinized starch (starch 1500), microcrystalline starch, water insoluble starch, calcium carboxymethyl cellulose, and magnesium aluminum silicate, or a combination thereof. In other embodiments, the core further comprises at least one of an absorption enhancer, a binder, a hardness enhancing agent, a buffering agent, a filler, a flow regulating agent, a lubricant, a synergistic agent, a chelator, an antioxidant, a stabilizer and a preservative. Optionally, the core also comprises one or more other excipients.

The burst controlling agent in the core preferably comprises a water insoluble polymer for controlling the rate of penetration of water into the core and raising the internal pressure (osmotic pressure) inside the core. Such a burst controlling agent is preferably able to swell upon contact with liquid. Non-limiting examples of suitable water insoluble polymers include cross-linked polysaccharide, water insoluble starch, microcrystalline cellulose, water insoluble cross-linked peptide, water insoluble cross-linked protein, water insoluble cross-linked gelatin, water insoluble cross-linked hydrolyzed gelatin, water insoluble cross-linked collagen modified cellulose, and cross-linked polyacrylic acid. In one embodiment, the water insoluble polymer is a cross-linked polysaccharide selected from the group consisting of insoluble metal salts or cross-linked derivatives of alginate, pectin, xanthan gum, guar gum, tragacanth gum, and locust bean gum, carrageenan, metal salts thereof, and covalently cross-linked derivatives thereof. In one embodiment, the water insoluble polymer is a modified cellulose selected from the group consisting of cross-linked derivatives of hydroxypropylcellulose, hydroxypropylmethylcellulose, hydroxyethylcellulose, methylcellulose, carboxymethylcellulose, and metal salts of carboxymethylcellulose. In another embodiment, the water insoluble polymer is selected from calcium pectinate, microcrystalline cellulose, or a combination thereof.

The outer layer comprises a water insoluble hydrophobic carrier and a pore forming agent comprised of a water insoluble hydrophilic particular matter. The pore forming agent is a water permeable agent which allows entry of liquid into the core. Optionally, the outer layer further comprises at least one of a wetting agent, a suspending agent, a dispersing agent, a stiffening agent, and a plasticizer.

In certain embodiments, the water insoluble hydrophobic carrier is selected from the group consisting of a dimethylaminoethylacrylate/ethylmethacrylate copolymer, the copolymer being based on acrylic and methacrylic acid esters with a low content of quaternary ammonium groups, wherein the molar ratio of the ammonium groups to the remaining neutral (meth)acrylic acid esters is approximately 1:20, the polymer corresponding to USP/NF "Ammonio Methacrylate Copolymer Type A", an ethylmethacrylate/chlorotrimethylammoniumethyl methacrylate copolymer, the copolymer based on acrylic and methacrylic acid esters with a low content of quaternary ammonium groups wherein the molar ratio of the ammonium groups to the remaining neutral (meth)acrylic acid esters is 1:40, the polymer corresponding to USP/NF "Ammonio Methacrylate Copolymer Type B", a dimethylaminoethylmethacrylate/methylmethacrylate and butylmethacrylate copolymer, a copolymer based on neutral methacrylic acid esters and dimethylaminoethyl methacrylate esters wherein the polymer is cationic in the presence of acids, an ethylacrylate and methylacrylate/ethylmethacrylate and methyl methylacrylate copolymer, the copolymer being a neutral copolymer based on neutral methacrylic acid and acrylic acid esters, ethylcellulose, shellac, zein, and waxes.

In certain embodiments, the water insoluble particulate matter is a hydrophilic yet water insoluble polymer, preferably selected from the group consisting of a water insoluble cross-linked polysaccharide, a water insoluble cross-linked protein, a water insoluble cross-linked peptide, water insoluble cross-linked gelatin, water insoluble cross-linked hydrolyzed gelatin, water insoluble cross-linked collagen, water insoluble cross linked polyacrylic acid, water insoluble cross-linked cellulose derivatives, water insoluble cross-linked polyvinyl pyrrolidone, micro crystalline cellulose, insoluble starch, micro crystalline starch and a combination thereof. Most preferably, the water insoluble particulate matter is microcrystalline cellulose.

In certain embodiments, the burst formulation further comprises an enteric coating on the outer layer. The enteric coating is preferably selected from the group consisting of cellulose acetate phthalate, hydroxy propyl methyl cellulose acetate succinate, and a EUDRAGIT polymer such as EUDRAGIT L100 or EUDRAGIT L30D-55.

### BIODEGRADABLE FORMULATIONS

In one embodiment, the GCC agonist formulation comprises a natural or synthetic polymer which is susceptible to being degraded by at least one colonic bacterial enzyme. Preferably, the GCC agonist is embedded in the polymer matrix. Non-limiting examples of such polymers include polymers of polysaccharides such as amylase, chitosan, chondroitin sulfate, cyclodextrin, dextran, guar gum, pectin, and xylan. Preferably, the natural or synthetic polymer is gelled or crosslinked with a cation such as a zinc cation, for example from zinc sulfate, zinc chloride, or zinc acetate. The formulation is preferably in the form of ionically crosslinked beads which are subsequently coated with an enteric coating. The enteric coating can comprise any suitable enteric coating material, such as hydroxypropylmethyl cellulose phthalate, polyvinyl acetate phthalate, cellulose acetate phthalate, hydroxypropylmethyl cellulose acetate succinate, alginic acid, and sodium alginate, or a EUDRAGIT polymer.

In another embodiment, the GCC agonist formulation comprises a GCC agonist covalently conjugated to a carrier molecule such that the covalent bond between the GCC agonist and the carrier is stable in the stomach and small intestine but labile in the lower gastrointestinal tract, especially the colon. The GCC agonist covalently linked to a carrier molecule is referred to as the "GCC prodrug." In certain embodiments, the GCC prodrug comprises a GCC agonist covalently conjugated to a carrier molecule via an azo bond or a glycosidic bond. In other embodiments, the GCC prodrug comprises a glucuronide, a cyclodextrin, a dextran ester, or a polar amino acid. In certain embodiments, the GCC prodrug is a polymeric prodrug. In one embodiment, the polymeric prodrug comprises polyamides containing azo groups.

### DOSAGE

The GCRA peptides described herein may be formulated using gastrointestinal retention system technology (GIRES; Merrion Pharmaceuticals). GIRES comprises a controlled-release dosage form inside an inflatable pouch, which is placed in a drug capsule for oral administration. Upon dissolution of the capsule, a gas-generating system inflates the pouch in the stomach where it is retained for 16-24 hours, all the time releasing agents described herein.

The GCRA peptides described herein can be formulated in an osmotic device including the ones disclosed in US4,503,030, US5,609,590 and US5,358,502. US4,503,030 discloses an osmotic device for dispensing a drug to certain pH regions of the gastrointestinal tract. More particularly, the disclosure relates to an osmotic device comprising a wall formed of a semi-permeable pH sensitive composition that surrounds a compartment containing a drug, with a passageway through the wall connecting the exterior of the device with the compartment. The device delivers the drug at a controlled rate in the region of the gastrointestinal tract having a pH of less than 3.5, and the device self- destructs and releases all its drug in the region of the gastrointestinal tract having a pH greater than 3.5, thereby providing total availability for drug absorption. U.S. Patent Nos. 5,609,590 and 5, 358,502 disclose an osmotic bursting device for dispensing a beneficial agent to an aqueous environment. The device comprises a beneficial agent and osmagent surrounded at least in part by a semi-permeable membrane. The beneficial agent may also function as the osmagent. The semi-permeable membrane is permeable to water and substantially impermeable to the beneficial agent and osmagent. A trigger means is attached to the semi-permeable membrane (*e.g.,* joins two capsule halves). The trigger means is activated by a pH of from 3 to 9 and triggers the eventual, but sudden, delivery of the beneficial agent. These devices enable the pH-triggered release of the beneficial agent core as a bolus by osmotic bursting.

Dosage levels of active ingredients in a pharmaceutical composition can also be varied so as to achieve a transient or sustained concentration of the compound in a subject, especially in and around the site of inflammation or disease area, and to result in the desired response. It is well within the skill of the art to start doses of the compound at levels lower than required to achieve the desired effect and to gradually increase the dosage until the desired effect is achieved. It will be understood that the specific dose level for any particular subject will depend on a variety of factors, including body weight, general health, diet, natural history of disease, route and scheduling of administration, combination with one or more other drugs, and severity of disease.

An effective dosage of the composition will typically be between about 1 µg and about 10 mg per kilogram body weight, preferably between about 10 µg to 5 mg of the compound per kilogram body weight. Adjustments in dosage will be made using methods that are routine in the art and will be based upon the particular composition being used and clinical considerations.

The guanylate cyclase receptor agonists used in the methods described above may be administered orally, systemically or locally. Dosage forms include preparations for inhalation or injection, solutions, suspensions, emulsions, tablets, capsules, topical salves and lotions, transdermal compositions, other known peptide formulations and pegylated peptide analogs. Agonists may be administered as either the sole active agent or in combination with other drugs, *e.g.,* an inhibitor of cGMP-dependent phosphodiesterase and anti-inflammatory agent. In all cases, additional drugs should be administered at a dosage that is therapeutically effective using the existing art as a guide. Drugs may be administered in a single composition or sequentially.

Dosage levels of the GCR agonist for use in methods disclosed herein typically are from about 0.001 mg to about 10,000 mg daily, preferably from about 0.005 mg to about 1,000 mg daily. For example, an effective dosage of the GCRA peptide for use in methods disclosed herein is about 0.1, about 0.2. about 0.3,about 0.4.about 0.5, about 0.6, about 0.7, about 0.8, about 0.9, about 1.0, about 1.5, about 2.0, about 2.5, about 3.0, about 3.5, about 4.0, about 4.5, about 5.0, about 5.5, about 6.0, about 6.5, about 7.0, about 7.5, about 8.0, about 8.5, about 9.0, about 9.5, about 10, about 11, about 12, about 13, about 14, about 15, about 16, about 17, about 18, about 19, or about 20 mg per day, or optionally twice a day. Preferably the GCRA peptide is given after a meal (i.e, 30 minutes). In some embodiments a second agent useful for treating a lipid metabolism disorder, a billary disorder, a cardiovascular disease, obesity or an an endocrine disorder is administered. Suitable second agents are described herein. In some aspects the second agent is administered at less than the standard does for treating the particular disorder because the GCRA peptide acts synergistically with the second agent. For example, about 2.5, about 5. about 7.5 or about 10 mg of Liptor is given twice a day after a meal (i.e, 30 minutes). On the basis of mg/kg daily dose, either given in single or divided doses, dosages typically range from about 0.001/75 mg/kg to about 10,000/75 mg/kg, preferably from about 0.005/75 mg/kg to about 1,000/75 mg/kg.

The total daily dose of each inhibitor can be administered to the patient in a single dose, or in multiple subdoses. Typically, subdoses can be administered two to six times per day, preferably two to four times per day, and even more preferably two to three times per day. Doses can be in immediate release form or sustained release form sufficiently effective to obtain the desired control over the medical condition.

The dosage regimen to prevent, treat, give relief from, or ameliorate a medical condition or disorder, or to otherwise protect against or treat a medical condition with the combinations and compositions of the present disclosure is selected in accordance with a variety of factors. These factors include, but are not limited to, the type, age, weight, sex, diet, and medical condition of the subject, the severity of the disease, the route of administration, pharmacological considerations such as the activity, efficacy, pharmacokinetics and toxicology profiles of the particular inhibitors employed, whether a drug delivery system is utilized, and whether the inhibitors are administered with other active ingredients. Thus, the dosage regimen actually employed may vary widely and therefore deviate from the preferred dosage regimen set forth above.

### EXAMPLES

### EXAMPLE 1: ORAL TREATMENT WITH SP-333, AN ANALOG OF UROGUANYLIN, RELIEVES MORPHINE AND METHADONE INDUCED CONSTIPATION THROUGH ACTIVATION OF GUANYLATE CYCLASE-C AND CYSTIC FIBROSIS TRANSMEMBRANE CONDUCTANCE REGULATOR IN RATS

Opioids are widely used for treatment of chronic pain, but their consumption is often associated with severe constipation in humans for which standard laxatives are not very effective. This side effect is believed to be due to reduction in fluid secretion, affecting gastrointestinal (GI) motility and bowel movement (BM). The mechanism through which opioid induced bowel dysfunction occurs is shown in Figure 2. Thus, there is a need for a safe, orally administrable pharmacological agent that is capable of relieving constipation induced by a broad class of opioids.

SP-333, an analog of uroguanylin, activates GC-C to stimulate production of cyclic GMP, which in turn activates cystic fibrosis transmembrane conductance regulator (CFTR), the apical chloride channel responsible for efflux of chloride ions from enterocytes lining the gastrointestinal (GI) tract, resulting in a net efflux of water into the intestinal lumen to facilitate bowel movement and GI motility (Shailubhai, 2002; Forte, 2004; Basu et al., 2010; Steinbrecher, 2014).

This study demonstrates the potential of SP-333 as an oral drug therapeutic for the treatment of opioid-induced constipation (OIC). Treatment with SP-333 evoked a concentration-dependent increase in short-circuit current (*Isc*) across T84 cells monolayer and rat jejunum tissue, even in the presence of morphine or methadone. This increase in *Isc* appears to be mediated via a GC-C/cGMP mechanism activating protein kinase G-II and cystic fibrosis transmembrane conductance regulator. In rat studies, daily intraperitoneal administration of methadone for seven days produced significant delay in GI transit, resulting in drastically reduced daily fecal output. Orally administered SP-333 completely restored the delay in GI transit caused by morphine or methadone. Oral treatment with SP-333 (2.5 and 5 mg/kg/day) not only normalized GI transit but also partially restored fecal output.

### Materials and Methods

**Cell culture:** T84, human carcinoma cell line, was purchased from American Type Culture Collection (ATCC) (Manassas, VA, USA). Cells were cultured in DMEM/F-12 media (Fisher Scientific, Pittsburgh, PA) containing 10% fetal bovine serum (Atlanta Biologicals, Flowery Branch, GA), 1% penstrep (Invitrogen, Carlsbad, CA) and 1 % glutamax (Invitrogen, Carlsbad, CA) as described (Shailubhai et al., 2000). For Ussing chamber experiments, cells were grown on 12 mm permeable polyester membrane (pore size 0.4 µm) Snapwell insert at a seeding density of ∼ 150,000 cells/insert (CLS 3801, Sigma, Saint Louis, MO) and incubated at 37°C in a humidified atmosphere of 5 % CO₂.

**Animals:** All study procedures were conducted under a study outline approved by the Institutional Animal Care and Use Committee of MB Research Labs (Spinnerstown, PA) or Lampire Biological Laboratories (Pipersville, PA). Seven to eight week old female Sprague-Dawley rats [Crl:CD(SD)], weighing between ∼180-210 grams, were obtained from Charles River Laboratories and allowed to acclimate for a week. Animals were maintained on a 12 hour light-dark cycle. For intestinal tissue collection and evaluation of GI transit, rats were fasted for at least 18 hours before the experiment, with free access to water.

**Rat tissue harvest:** Rats were euthanized by CO₂ inhalation. A midline abdominal incision was made. Proximal jejunum a 5-cm area 1-cm distal of the Treitz ligament was identified, incised and transferred to ice-cold RPMI medium containing indomethacin (10 µM). Ten minutes later, the tissue was transferred to fresh ice-cold RPMI medium without indomethacin. The tissue was pinned on the dissection pad and cut along the mesenteric border, apical side facing upwards and mounted on Ussing chamber sliders as described below.

**Short Circuit current measurements:** *I_{SC}* measurements were performed on EasyMount Ussing chamber system (4 chambers) with VCCMC8 multichannel current-voltage (I-V) clamps (Physiologic Instruments, San Diego, CA). Transepithelial resistance (TER) across the cell monolayer cultured on permeable membranes was regularly monitored using an EVOM epithelial volt ohmmeter (EMD Millipore, MA). Confluent T84 cells with TER >1000 ohm.cm² were used. Cells and tissues were mounted on specific sliders (1.12 cm² slit area for cells and 0.5 cm² for tissues) provided by Physiologic Instruments and mounted on to the Ussing chamber. The chambers were bathed continuously with Krebs Ringer buffer solution. Basolateral bath solution contained (in mM) 115 NaCl, 25 NaHCO₃, 3.3 KH₂PO₄, 0.8 K₂HPO₄, 1.12 MgCl₂, 1.2 CaCl₂ and 10 Hepes (Cuppoletti et al., 2004). In order to examine effect of SP-333 or specific inhibitors on chloride channel on the apical membrane, a basolateral to apical chloride gradient was imposed by substituting NaCI in the buffer in contact with the apical membrane with equal concentration of sodium gluconate. Since gluconate chelates Ca²⁺ ions, CaCl₂ concentration was increased to 4mM. (Bijvelds et al., 2009). Buffer solution was continuously gassed with carbogen (mixture of 95% O₂ and 5% CO₂), and the temperature was held constant at 37 °C with a heating block. The clamps were connected to Acquire & Analyze software (Physiologic Instruments) for automatic data collection from all four chambers. Ag/AgCl reference electrodes were used for measuring transepithelial voltage and passing current.

To evaluate SP-333 *Isc* across T84 cells monolayer (n=4 transwells/ concentration) and rat jejunum tissues (n=4 preparations/concentration), indicated amounts of the agonist was added to the apical buffer solution after stabilization of the baseline and peak activity was recorded and plotted as *I_{SC}* (µA/cm2) ± SEM. Effect of morphine or methadone on SP-333 stimulated *Isc* was determined by pretreating cells (n=3 transwells/opioid concentration) and rat jejunum (n=4-15 preparations/opioid concentration) on the apical side with the opioid for 5 minutes (Cuppoletti et al., 2013). Subsequently, 1µM SP-333 was added to the buffer on the apical side peak activity of stimulated currents was measured and expressed as chloride currents (*Isc*)(relative to vehicle control) ± SEM. To evaluate the role played by PKA and PKG-II and identify relative contribution of CFTR and CIC-2 towards agonists mediated release of CI- ions, experiments were performed in the presence and absence of kinase and CI⁻ channel inhibitors. Inhibitors employed were CdCl2 (CIC-2 inhibitor; Sigma-Aldrich; St Louis, MO), CFTRᵢₙₕ172 (CFTR inhibitor; Santa Cruz Biotech; Dallas, Texas), KT5823 (PKG inhibitor; R&D Systems, Minneapolis, MN), mPKI (PKA inhibitor; EMD Millipore; Billerica, MA). T84 cells (n=4 transwells/condition) and jejunum tissue (n=4-6 preparations/condition) were pretreated for 5 min with each inhibitor at the indicated concentration prior to addition of 1 mM SP-333. Peak current attained after addition of SP-333 was recorded and calculated in terms of percent relative activity ± SEM.

**Effect of SP-333 on opioid-induced GI transit delay in rats:** The ability of opioids to delay GI transit in rats was tested by either administering 0 (vehicle) 0.25, 1 and 2.5 mg/kg of morphine or 0 (vehicle), 0.25, 2.5 and 5 mg/kg of methadone (1.5 ml/kg dose volume) by IP injection. Water served as vehicle control. Ten minutes later, a 2 mL charcoal meal consisting of 10% activated charcoal (Sigma-Aldrich, St. Louis, MO) and 10% gum Arabic (Sigma-Aldrich, St. Louis, MO) in water was administered via oral gavage. Rats were euthanized 10 min after the charcoal meal via IP injection of 150 mg/kg Beuthanasia-D solution (sodium pentobarbital, 390 mg/mL). The small intestine was removed and the total length from the pyloric sphincter to the ileocecal junction was recorded, as well as the distance travelled by the leading edge of the charcoal. Intestinal transit was determined by calculating the ratio (expressed as a percentage) of the distance between the pyloric sphincter and the leading edge of the charcoal to the distance between the pyloric sphincter and the ileocecal junction.

To evaluate the ability of SP-333 to reverse morphine induced delayed GI transit, animals were administered 2.5 mg/kg of morphine by IP injection followed immediately by oral gavage of 0 (vehicle), 0.5, 2.5, 5 and 50 mg/kg of SP-333 (1.5 ml/kg dose volume). Water served as vehicle control. Charcoal meal was administered ten minutes later and animals were euthanized ten minutes after the charcoal meal and intestinal transit was calculated as described above.

To evaluate the ability of SP-333 to reverse methadone induced delayed GI transit, animals were administered 2.5 mg/kg of methadone by IP injection. Ten minutes later, an oral gavage of 0 (vehicle), 0.1, 0.5, 2.5, 5 and 50 mg/kg of SP-333 (1.5 ml/kg dose volume) was administered followed immediately by a 2 mL oral gavage of the charcoal meal. Ten minutes after the charcoal meal, animals were euthanized and intestinal transit was calculated as described above.

The ability of SP-333 to alleviate GI transit delay induced by repeated methadone administration was also evaluated in the similar manner. Female Sprague-Dawley CD rats 7 to 8 weeks of age (n=7/group) received via IP injection 16 doses of methadone (once daily; 7 doses @ 5 mg/kg and 9 doses @ 2.5 mg/kg). Ten minutes following each methadone dose, each group received an oral gavage of vehicle, 2.5, and 5 mg/kg of SP-333. At the end of the dosing regimen, animals were fasted overnight and GI transit was evaluated as described above.

**Effect of SP-333 on methadone-induced constipation in rats:** To identify the methadone dose that induces constipation in rats, female Sprague-Dawley CD rats 7 to 8 weeks of age (n=6/group) were administered 0 (vehicle), 2.5, 5, and 7.5 mg/kg of methadone (1.5ml/kg dose volume) by IP injection once a day for 5 days. Fecal pellets were collected at 4h intervals during the light cycle and average number of pellets was recorded for each group during the course of the study following daily dose administrations of methadone.

To evaluate the ability of SP-333 to relieve methadone-induced constipation, female Sprague-Dawley CD rats 7 to 8 weeks of age (n=7 /group) were administered 5 mg/kg of methadone (or vehicle) each day for 7 days via IP injection to induce constipation. Ten minutes following the IP dose each day, an oral gavage of 2.5, and 5 mg/kg of SP-333 or vehicle (buffer) was administered. Fecal pellets were collected at 4h intervals during the light cycle and average number of pellets recorded for each group during the course of the study following daily administration of vehicle, methadone or combination of methadone and SP-333.

**Statistical Analysis:** GraphPad Prism (version 6.01) was used to calculate summary statistics and inferential tests. Data are expressed as the mean ± SEM and differences between a control group and treatment group assessed for significance by two-tailed Student's *t*-test.

### Results

**PKG-II and CFTR channel dependent stimulation of CI⁻ currents by SP-333 in T84 cells and rat jejunum:** SP-333 stimulates CI⁻ currents (expressed as increase in *I_{sc}*) in T84 cells and rat jejunum. The results are depicted in **Fig. 3****.** Inclusion of SP-333 (0.01-10 µM) in the buffer solution in contact with the mucosal surface of T84 cells **(****Fig. 3A****)** or rat jejunum **(****Fig. 3B****)** resulted in a concentration-dependent increase in *Isc* approaching saturation at 10 µM in both instances. Overall *Isc* from jejunum tissues were much higher than that observed for T84 cells. However, SP-333 concentration that induced 50% increase in *Isc* across T84 cells monolayer (EC₅₀ = 1.69 x 10⁻⁷M) or jejunum tissue (EC₅₀ = 2.99 x 10⁻⁷M) was comparable.

In T84 cells, an 80% suppression in SP-333-stimulated *Isc* was observed in the presence of 10 µM CFTRᵢₙₕ172 as compared that observed with 1 µM SP-333 alone **(****Fig. 4A****).** Relative *Isc* in the presence of **PKG-II** inhibitor (4 µM) were also suppressed by approximately 35% **(****Fig. 4A****).** No appreciable decrease in stimulated *Isc* was observed in the presence of CIC-2 inhibitor, CdCl₂ (300 µM) and 3.2 µM protein kinase A (mPKI-) inhibitor **(****Fig. 4A****).** A similar pattern was observed with rat jejunum, although the magnitude of inhibition with equal amount of CFTRᵢₙₕ172 was less compared to that detected in T84 cells **(****Fig. 4B****).** Taken together the results suggest activated PKG-II and CFTR channel are required for SP-333 mediated increase in *Isc* in model cellular system, as well as in rat tissues.

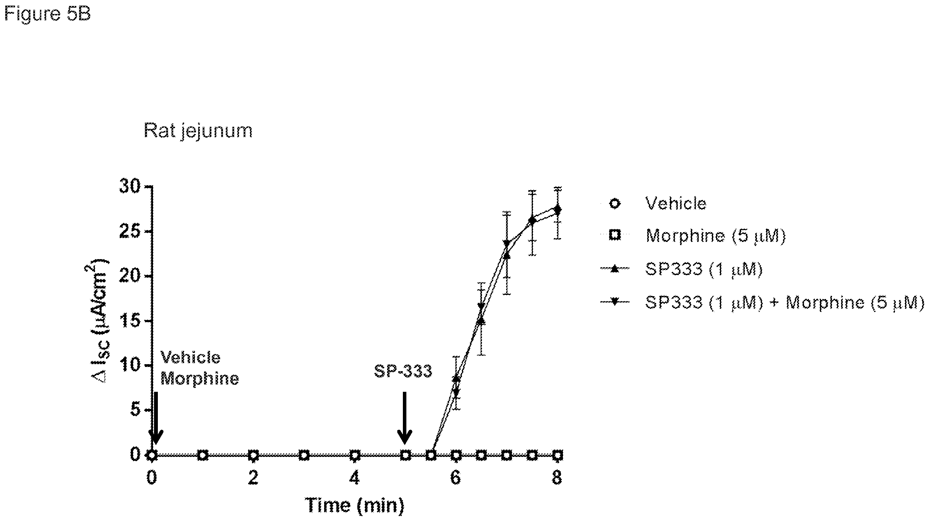

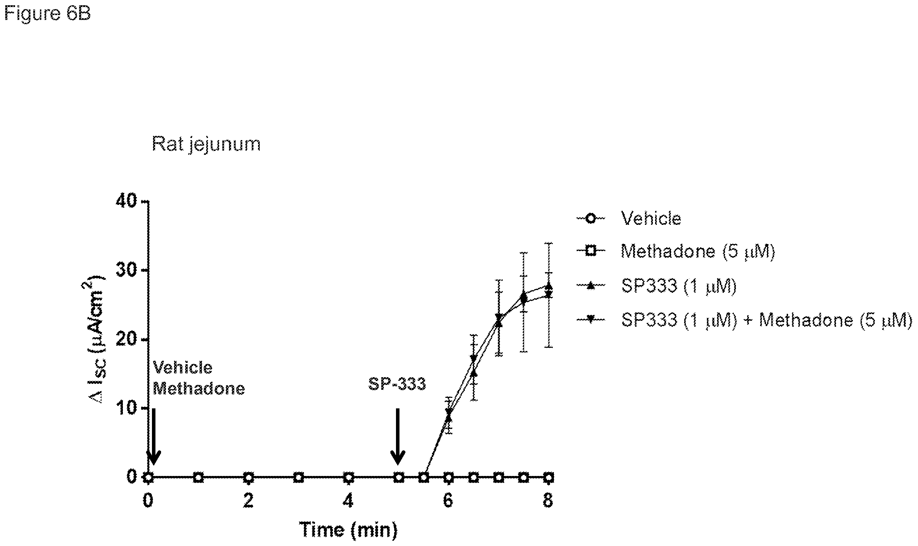

**Effect of morphine and methadone on SP-333-stimulated CI- currents:** The effect of select concentrations of morphine and methadone on SP-333-stimulated *Isc* was evaluated in T84 cells and rat jejunum. The results are shown in **Fig. 5** (morphine) and **Fig. 6** (methadone).

Pre-treatment of the apical membrane with 5 µM of morphine had negligible effect on 1 µM SP-333-stimulated *Isc* from T84 cells and rat jejunum tissue **(****Fig. 5****).** Similarly, T84 cells and rat jejunum pretreated with 5 µM methadone had no effect on SP-333 mediated currents **(****Fig 6A** **and** **B****).**

**Effect of oral administration of SP-333 on morphine-induced delay in rat GI transit:** A dose-dependent increase in small intestinal transit was observed after single oral gavage dose of SP-333 (5.6%, 13%, 23% at 0.05 mg/kg, 0.5 mg/kg and 5 mg/kg, respectively - see **Fig. 7****)** compared to vehicle controls suggesting that SP-333 treatment alone causes an increase in GI transit in opioid naive animals (Joshi et al., 2014). Intraperitoneal (IP) administration of morphine significantly reduced GI transit in rats **(****Fig. 8A****).** Compared to vehicle controls, GI transit after a single morphine dose was slowed by 52%, 56% and 56% at 0.25, 1.0 and 2.5 mg/kg of the opioid, respectively.

To evaluate the ability of SP-333 to relieve morphine-induced delayed GI transit, animals were administered 2.5 mg/kg of morphine by IP injection followed immediately by oral gavage of 0 (vehicle), 0.5, 2.5, 5 and 50 mg/kg of SP-333. Animals were euthanized 10 minutes later and GI transit was calculated as described under Materials and Methods. Morphine significantly reduced GI transit (23% vs 53% in vehicle treated; p<0.0001) **(****Fig. 8B****).** SP-333 produced a dose-dependent improvement in gut transit in morphine-treated rats: 27%, 37%, 46%, and 48% at 0.5, 2.5, 5, and 50 mg/kg, respectively (P ≤0.0001 at all doses except 0.5 mg/kg as compared with morphine alone). Results suggest that an SP-333 dose of 5 mg/kg is sufficient to relieve the effect of morphine on GI transit. Mean GI transit ± SEM is depicted in **Fig. 8B****.**

**Effect of oral administration of SP-333 on methadone-induced delay in rat GI transit:** Intraperitoneal administration of methadone significantly reduced GI transit in rats. A methadone dose of 2.5 mg/kg was sufficient to significantly reduce GI transit. Additionally, results were comparable to that observed with morphine at 2.5 mg/kg (49% and 56% vs corresponding vehicle controls for methadone and morphine, respectively). Mean GI transit ± SEM is depicted in **Fig. 9A****.**

To evaluate the ability of SP-333 to relieve methadone-induced delayed GI transit, animals were administered 2.5 mg/kg of methadone by IP injection. Ten minutes later, an oral gavage of 0 (vehicle), 0.5, 2.5, 5 and 50 mg/kg of SP-333 was administered. Animals were euthanized 10 minutes later and GI transit was calculated as described under Materials and Methods. Methadone significantly reduced GI transit (28% vs 55% in vehicle treated; P<0.0001). As depicted in **Fig. 9B****,** SP-333 administration produced a dose-dependent improvement in gut transit in methadone-treated rats: 40%, 39%, 44%, and 52% at 0.5, 2.5, 5, and 50 mg/kg, respectively (P<0.01 at all doses). These data suggest that SP-333 dose of 5 mg/kg is sufficient to relieve the effect of methadone on GI transit. Mean GI transit ± SEM is depicted in **Fig. 9B****.**

**Effect of oral administration of SP-333 on constipation induced in rats by repeated methadone administration:** Constipation was induced in rats by IP administration of 2.5, 5, and 7.5 mg/kg of methadone once daily for a period of 5 days. The average number of fecal pellets collected over 4h time period during the light cycle was significantly lower in the methadone dosed animal group compared to the vehicle group; 29%, 77%, and 54%, lower at 2.5, 5, and 7.5 mg/kg, respectively (P<0.01 at all doses) **(****Fig. 10A****).** The most pronounced effect of methadone on fecal output was observed at 5 mg/kg dose. To evaluate the ability of SP-333 to relieve methadone-induced constipation, animals were administered 5.0 mg/kg of methadone by IP injection once daily for a period of 7 days. Ten minutes following opioid administration, an oral gavage of 0 (vehicle), 2.5, and 5 mg/kg of SP-333 was administered on each day, and fecal output was monitored over 4h time frame during the light cycle. As shown in **Fig. 10B****,** a significant decrease in the average number of fecal pellets was observed in animals dosed with methadone alone (82% less vs vehicle control). The average number of pellets at all doses of SP-333 was higher compared to the methadone dosed group. A threefold increase in pellet count was observed at 2.5 mg/kg of SP-333 compared to that observed for the methadone dosed group that did not receive any SP-333 demonstrating that SP-333 relieves constipation in rats after repeated methadone administration. Further, administration of SP-333 relieved the GI-transit delay caused by repeated methadone dosing **(****Fig. 11****).**

### Discussion

Ussing chamber experiments performed with T84 cells and rat jejunum suggest that SP-333 mediated stimulation of short circuit current is dependent on functional PKG-II and CFTR channel. *In vivo* animal model studies further demonstrate that orally administered SP-333 is able to alleviate GI transit delay in rats induced by single or repeated dosing with opioids, as well as relieve constipation induced by repeated methadone administration.

The mechanism by which GC-C agonists relieves opioid induced GI transit delay and constipation is presented in **Fig. 12****.** Activation of peripherally expressed opioid receptors by morphine and methadone triggers downstream signaling that results in inhibition of cAMP synthetic enzyme, adenylyl cyclase and release of neurotransmitters such as acetylcholine, substance P and vasoactive intestinal peptide (Sobczak et al., 2013). Consequently, gastric emptying is inhibited, GI transit and peristalsis are impaired, secretion is reduced with a concomitant increase in absorption of fluids and electrolytes from intestinal lumen resulting in manifestation of OIBD (Holzer, 2009; Müller-Lissner, 2010; Sobczak etal., 2013). Engagement of apically expressed intestinal epithelial cell GC-C receptor by SP-333 promotes synthesis and accumulation of intracellular cGMP (Zhang et al., 2012). This second messenger has the potential to impact multiple effectors within the cells; it can bind and activate PKG-II, activate protein kinase A (PKA) either directly or indirectly via inhibition of phosphodiesterase 3, which hydrolyzes cAMP to AMP. PKG-II and PKA can phosphorylate and activate CFTR channel to promote efflux of CI⁻ ions followed by passive efflux of Na⁺ and water into the intestinal lumen thus facilitating bowel movement. Electrophysiological data suggests that SP-333-mediated increase in *Isc* across T84 cells monolayer and rat jejunum tissues is via PKG-II and CFTR. The presence of PKA inhibitor (mPKI) and CIC-2 inhibitor (CdCl₂) had minimal effect on SP-333 stimulated *Isc* from cells and tissues.

Those skilled in the art will recognize, or be able to ascertain using no more than routine experimentation, many equivalents to the specific embodiments described herein.

### REFERENCES

Barrett KE and Keely SJ (2000) Chloride secretion by the intestinal epithelium: molecular basis and regulatory aspects. Annu Rev Physiol 62:535-572.
Basu N, Arshad N and Visweswariah SS (2010) Receptor guanylyl cyclase C (GC-C): regulation and signal transduction. Mol Cell Biochem 334:67-80.
Bell TJ, Panchal SJ, Miaskowski C, Bolge SC, Milanova T and Williamson R (2009) The prevalence, severity, and impact of opioid-induced bowel dysfunction: results of a US and European Patient Survey (PROBE 1). Pain medicine 10:35-42.
Bianchi G, Fiocchi R, Peracchia F, Petrillo P, Tavani A and Manara L (1984) The peripheral narcotic antagonist N-allyl levallorphan-bromide (CM 32191) selectively prevents morphine antipropulsive action and buprenorphine in-vivo binding in the rat intestine. J Pharm Pharmacol 36:326-330.
Bijvelds MJC, Bot AGM, Escher JC and de Jonge HR (2009) Activation of Intestinal Cl- Secretion by Lubiprostone Requires the Cystic Fibrosis Transmembrane Conductance Regulator. Gastroenterology 137:976-985.
Camilleri M (2011) Opioid-induced constipation: challenges and therapeutic opportunities. Am J Gastroenterol 106:835-842; quiz 843.
Clarke LL (2009) A guide to Ussing chamber studies of mouse intestine. Am J Physiol Gastrointest Liver Physiol 296:G1151-1166.
Cook SF, Lanza L, Zhou X, Sweeney CT, Goss D, Hollis K, Mangel AW and Fehnel SE (2008) Gastrointestinal side effects in chronic opioid users: results from a population-based survey. Aliment Pharmacol Ther 27:1224-1232.
Cryer BL, Katz S, Vallejo R, Scott CB, Joswick TR, Dolecek G and Ueno R (2010) 906 A Phase 3, Randomized, Double-Blind, Placebo-Controlled Clinical Trial of Lubiprostone for the Treatment of Opioid-Induced Bowel Dysfunction in Patients With Chronic, Non-Cancer Pain. Gastroenterology 138: S-129.
Cuppoletti J, Chakrabarti J, Tewari K and Malinowska DH (2013) Methadone but not morphine inhibits lubiprostone-stimulated Cl- currents in T84 intestinal cells and recombinant human CIC-2, but not CFTR Cl- currents. Cell Biochem Biophys 66:53-63.
Cuppoletti J, Chakrabarti J, Tewari KP and Malinowska DH (2014) Differentiation between Human C1C-2 and CFTR Cl- Channels with Pharmacological Agents. Am J Physiol Cell Physiol.
Cuppoletti J, Malinowska DH, Tewari KP, Li QJ, Sherry AM, Patchen ML and Ueno R (2004) SPI-0211 activates T84 cell chloride transport and recombinant human C1C-2 chloride currents. Am J Physiol Cell Physiol 287:C1173-1183.
Fiocchi R, Bianchi G, Petrillo P, Tavani A and Manara L (1982) Morphine inhibits gastrointestinal transit in the rat primarily by impairing propulsive activity of the small intestine. Life Sci 31:2221-2223.
Forte LR, Jr. (2004) Uroguanylin and guanylin peptides: pharmacology and experimental therapeutics. Pharmacol Ther 104:137-162.
Furlan AD, Sandoval JA, Mailis-Gagnon A and Tunks E (2006) Opioids for chronic noncancer pain: a meta-analysis of effectiveness and side effects. CMAJ 174:1589-1594.
Golin-Bisello F, Bradbury N and Ameen N (2005) STa and cGMP stimulate CFTR translocation to the surface of villus enterocytes in rat jejunum and is regulated by protein kinase G. Am J Physiol Cell Physiol 289:C708-716.
Holzer P (2009) Opioid receptors in the gastrointestinal tract. Regul Pept 155:11-17.
Joshi A, Patwa V, Thadi A, Vemalapally L, Foss J, Feng R, Comiskey S, Ricci A, Palejwala V, Brancale A and Shailubhai K (2014) Plecanatide and SP-333, Novel Homologs of Uroguanylin, Activate Guanylate Cyclase C to Promote Gastrointestinal Motility and Bowel Movement in Rats and Monkeys. Dig Dis Sci:Communicated.
Ketwaroo GA, Cheng V and Lembo A (2013) Opioid-induced bowel dysfunction. Curr Gastroenterol Rep 15:344.
Kumar L, Barker C and Emmanuel A (2014) Opioid-induced constipation: pathophysiology, clinical consequences, and management. Gastroenterology research and practice 2014:141737.
Kurz A and Sessler DI (2003) Opioid-induced bowel dysfunction: pathophysiology and potential new therapies. Drugs 63:649-671.
Lipecka J, Bali M, Thomas A, Fanen P, Edelman A and Fritsch J (2002) Distribution of C1C-2 chloride channel in rat and human epithelial tissues. Am J Physiol Cell Physiol 282:C805-816.
Love BL, Johnson A and Smith LS (2014) Linaclotide: A novel agent for chronic constipation and irritable bowel syndrome. Am J Health Syst Pharm 71:1081-1091.
Manara L, Bianchi G, Ferretti P and Tavani A (1986) Inhibition of gastrointestinal transit by morphine in rats results primarily from direct drug action on gut opioid sites. J Pharmacol Exp Ther 237:945-949.
Manara L, Bianchi G, Fiocchi R, Notarnicola A, Peracchia F and Tavani A (1982) Inhibition of gastrointestinal transit by morphine and FK 33-824 in the rat and comparative narcotic antagonist properties of naloxone and its N-methyl quaternary analog. Life Sci 31:1271-1274.
Miner PB, Surowitz R, Fogel R, Koltun W, Drossman DA, Camilleri M, Mangel A, Barrow L, Jacob G and Shailubhai K (2013) Plecanatide, a novel guanylate cyclase-C (GC-C) receptor agonist, is efficacious and safe in patients with chronic idiopathic constipation (CIC): results from a 951 patient, 12 week, multi-center trial.. Gastroenterology 144: S-263 (Abstract 925g).
Müller-Lissner S (2010) Opioid-induced Constipation-Mechanisms, Relevance and Management. Eur Gastroenterol Hepatol Rev 6:54-57.
Nighot PK, Moeser AJ, Ryan KA, Ghashghaei T and Blikslager AT (2009) C1C-2 is required for rapid restoration of epithelial tight junctions in ischemic-injured murine jejunum. Exp Cell Res 315: 110-118.
Pappagallo M (2001) Incidence, prevalence, and management of opioid bowel dysfunction. Am J Surg 182: 11S-18S.
Pena-Munzenmayer G, Catalan M, Cornejo I, Figueroa CD, Melvin JE, Niemeyer MI, Cid LP and Sepulveda FV (2005) Basolateral localization of native C1C-2 chloride channels in absorptive intestinal epithelial cells and basolateral sorting encoded by a CBS-2 domain di-leucine motif. J Cell Sci 118:4243-4252.
Penning-van Beest FJ, van den Haak P, Klok RM, Prevoo YF, van der Peet DL and Herings RM (2010) Quality of life in relation to constipation among opioid users. Journal of medical economics 13:129-135.
Quigley C (2005) The role of opioids in cancer pain. BMJ 331:825-829.
Rauck RL (2013) Treatment of Opioid-Induced Constipation: Focus on the Peripheral mu-Opioid Receptor Antagonist Methylnaltrexone. Drugs 73:1297-1306.
Shailubhai K (2002) Therapeutic applications of guanylate cyclase-C receptor agonists. Curr Opin Drug Discov Devel 5:261-268.
Shailubhai K, Comiskey S, Foss JA, Feng R, Barrow L, Comer GM and Jacob GS (2013) Plecanatide, an oral guanylate cyclase C agonist acting locally in the gastrointestinal tract, is safe and well-tolerated in single doses. Dig Dis Sci 58:2580-2586.
Shailubhai K, Yu HH, Karunanandaa K, Wang JY, Eber SL, Wang Y, Joo NS, Kim HD, Miedema BW, Abbas SZ, Boddupalli SS, Currie MG and Forte LR (2000) Uroguanylin treatment suppresses polyp formation in the Apc(Min/+) mouse and induces apoptosis in human colon adenocarcinoma cells via cyclic GMP. Cancer Res 60:5151-5157.
Sobczak M, Salaga M, Storr MA and Fichna J (2013) Physiology, signaling, and pharmacology of opioid receptors and their ligands in the gastrointestinal tract: current concepts and future perspectives. J Gastroenterol.
Steinbrecher KA (2014) The multiple roles of guanylate cyclase C, a heat stable enterotoxin receptor. Current opinion in gastroenterology 30:1-6.
Steinbrecher KA and Cohen MB (2011) Transmembrane guanylate cyclase in intestinal pathophysiology. Current opinion in gastroenterology 27:139-145.
Sun X, Wang X, Wang GD, Xia Y, Liu S, Qu M, Needleman BJ, Mikami DJ, Melvin WS, Bohn LM, Ueno R and Wood JD (2011) Lubiprostone reverses the inhibitory action of morphine on mucosal secretion in human small intestine. Dig Dis Sci 56:330-338.
Sykes NP (1998) The relationship between opioid use and laxative use in terminally ill cancer patients. Palliat Med 12:375-382.
Thiagarajah JR, Broadbent T, Hsieh E and Verkman AS (2004) Prevention of toxin-induced intestinal ion and fluid secretion by a small-molecule CFTR inhibitor. Gastroenterology 126:511-519.
Wang G-D, Wang X, Qu M-H, Xia Y and Wood JD (2014) 414 Reversal of Opioid-Induced Constipation by Lubiprostone (Amitiza®) in Guinea Pig Ileum. Gastroenterology 146:S-89.
Wong BS and Camilleri M (2011) Lubiprostone for the treatment of opioid-induced bowel dysfunction. Expert Opin Pharmacother 12:983-990.
Zhang G, Arjunan KP, Foss J, Comiskey S and Shailubhai K (2012) SP-333, a Proteolysis-resistant Agonist of Guanylate Cyclase-C, Inhibits Activation of NF-kappa B and Suppresses Production of Inflammatory Cytokines to Ameliorate DSS-induced Colitis in Mice. Am J Gastroenterol 107:S626-S626.

## Claims

1. A composition comprising a guanylate cyclase receptor agonist (GCRA) peptide consisting of the amino acid sequence of any one of SEQ ID NOs: 1-54 and 56-249 for use in preventing, treating or alleviating symptoms of an opioid-induced bowel dysfunction, wherein the composition is for administration to a subject in need thereof in a therapeutically effective amount, wherein the subject is undergoing acute opioid use or undergoing chronic opioid use.

2. The composition for the use of claim 1, further comprising the administration of an effective dose of a cGMP-dependent phosphodiesterase inhibitor.

3. The composition for the use of claim 2, wherein said cGMP-dependent phosphodiesterase inhibitor is selected from the group consisting of sulindac sulfone, zaprinast, motapizone, vardenafil, and sildenafil.

4. The composition for the use of claim 2, wherein said cGMP-dependent phosphodiesterase inhibitor is for administration either concurrently or sequentially with said GCRA peptide or pharmaceutical composition thereof.

5. The composition for the use of claim 1, wherein said GCRA peptide is SEQ ID NO: 1 (SP-304), SEQ ID NO.:9 (SP-333), SP373 (SEQ ID NO.:104), SP364 (SEQ ID NO.:100), SP366 (SEQ ID NO.:102) or SEQ ID NO: 250.

6. The composition for the use of claim 1, further comprising an opioid.

7. The composition for the use of claim 6, wherein the opioid and guanylate cyclase receptor agonist (GCRA) peptide are administered in one tablet or capsule.

8. The composition for the use of claim 6 further comprising one or more targeting materials selected from the group consisting of a pH-dependent polymer, a swellable polymer, and a degradable composition.

9. The composition for the use of claim 6, further comprising a pharmaceutical carrier, excipient or diluent.

10. The composition for the use of claim 8, wherein the formulation is optimized for delivery to the duodenum, jejunum, ileum, terminal ileum, or ascending colon, or wherein the formulation is optimized for release throughout the colon.

11. The composition for the use of claim 8, wherein the formulation comprises one or more pH dependent polymers which degrade in a pH range of 4.5 to 5.5, in a pH range of 5.5 to 6.5, or in a pH range of 6.5 to 7.5.

12. The composition for the use of claim 8, wherein the release of the composition is time dependent.

13. The composition for the use of claim 6, wherein the opioid is selected from the group consisting of morphine, codeine, oxycodone, hydrocodone, dihydrocodeine, propoxyphene, fentanyl, tramadol, loperamide, butorphanol, or combinations thereof.

14. The composition for the use of claim 1, wherein said GCRA peptide is SEQ ID NO: 1 (SP-304) or SEQ ID NO.:9 (SP-333).

## Patentansprüche

1. Zusammensetzung, umfassend ein Guanylatcyclase-Rezeptor-Agonist(GCRA)-Peptid, das aus der Aminosäuresequenz unter einer der SEQ ID NO: 1-54 und 56-249 besteht, zur Verwendung bei der Vorbeugung, Behandlung oder Linderung von Symptomen einer opioidinduzierten Darmfunktionsstörung, wobei die Zusammensetzung zur Verabreichung an ein diese benötigendes Individuum in einer therapeutisch wirksamen Menge vorgesehen ist, wobei bei dem Individuum akute Opioidanwendung oder chronische Opioidanwendung vorliegt.

2. Zusammensetzung zur Verwendung nach Anspruch 1, ferner umfassend die Verabreichung einer wirksamen Dosis eines cGMP-abhängigen Phosphodiesterase-Inhibitors.

3. Zusammensetzung zur Verwendung nach Anspruch 2, wobei der cGMP-abhängige Phosphodiesterase-Inhibitor ausgewählt ist aus der Gruppe bestehend aus Sulindacsulfon, Zaprinast, Motapizon, Vardenafil und Sildenafil.

4. Zusammensetzung zur Verwendung nach Anspruch 2, wobei der cGMP-abhängige Phosphodiesterase-Inhibitor zur Verabreichung entweder gleichzeitig mit oder nach dem GCRA-Peptid bzw. der pharmazeutischen Zusammensetzung davon vorgesehen ist.

5. Zusammensetzung zur Verwendung nach Anspruch 1, wobei es sich bei dem GCRA-Peptid um SEQ ID NO.: 1 (SP-304), SEQ ID NO.:9 (SP-333), SP373 (SEQ ID NO.:104), SP364 (SEQ ID NO.:100), SP366 (SEQ ID NO.:102) oder SEQ ID NO.: 250 handelt.

6. Zusammensetzung zur Verwendung nach Anspruch 1, ferner umfassend ein Opioid.

7. Zusammensetzung zur Verwendung nach Anspruch 6, wobei das Opioid und Guanylatcyclase-Rezeptor-Agonist(GCRA)-Peptid in einer Tablette oder Kapsel verabreicht werden.

8. Zusammensetzung zur Verwendung nach Anspruch 6, ferner umfassend ein oder mehrere Targeting-Materialien ausgewählt aus der Gruppe bestehend aus einem pH-abhängigen Polymer, einem quellfähigen Polymer und einer abbaubaren Zusammensetzung.

9. Zusammensetzung zur Verwendung nach Anspruch 6, ferner umfassend einen pharmazeutischen Träger, Exzipienten bzw. ein pharmazeutisches Verdünnungsmittel.

10. Zusammensetzung zur Verwendung nach Anspruch 8, wobei die Formulierung zur Zuführung an das Duodenum, Jejunum, Ileum, terminale Ileum oder Colon ascendens optimiert ist oder wobei die Formulierung zur Freisetzung über den gesamten Dickdarm optimiert ist.

11. Zusammensetzung zur Verwendung nach Anspruch 8, wobei die Formulierung ein oder mehrere pH-abhängige Polymere umfasst, die in einem pH-Bereich von 4,5 bis 5,5, in einem pH-Bereich von 5,5 bis 6,5 oder in einem pH-Bereich von 6,5 bis 7,5 abgebaut werden.

12. Zusammensetzung zur Verwendung nach Anspruch 8, wobei die Freisetzung der Zusammensetzung zeitabhängig ist.

13. Zusammensetzung zur Verwendung nach Anspruch 6, wobei das Opioid ausgewählt ist aus der Gruppe bestehend aus Morphium, Codein, Oxycodon, Hydrocodon, Dihydrocodein, Propoxyphen, Fentanyl, Tramadol, Loperamid, Butorphanol oder Kombinationen davon.

14. Zusammensetzung zur Verwendung nach Anspruch 1, wobei es sich bei dem GCRA-Peptid um SEQ ID NO.: 1 (SP-304) oder SEQ ID NO.:9 (SP-333) handelt.

## Revendications

1. Composition comprenant un peptide agoniste du récepteur de guanylate cyclase (GCRA), constitué par la séquence d'acides aminés de l'une quelconque des SEQ ID n° : 1 à 54 et 56 à 249, destinée à être utilisée dans la prévention, le traitement ou le soulagement de symptômes d'un dysfonctionnement intestinal induit par des opiacés, la composition étant destinée à une administration à un sujet la nécessitant en une quantité efficace d'un point de vue thérapeutique, dans laquelle le sujet a une utilisation aiguë d'opiacés ou a une utilisation chronique d'opiacés.

2. Composition destinée à l'utilisation de la revendication 1, comprenant en outre l'administration d'une dose efficace d'un inhibiteur de phosphodiestérase dépendante de la GMPc.

3. Composition destinée à l'utilisation de la revendication 2, dans laquelle ledit inhibiteur de phosphodiestérase dépendante de la GMPc est choisi dans le groupe constitué par la sulindac sulfone, le zaprinast, la motapizone, le vardénafil et le sildénafil.

4. Composition destinée à l'utilisation de la revendication 2, dans laquelle ledit inhibiteur de phosphodiestérase dépendante de la GMPc est destiné à une administration soit parallèlement soit séquentiellement avec ledit peptide de GCRA ou une composition pharmaceutique de celui-ci.

5. Composition destinée à l'utilisation de la revendication 1, dans laquelle ledit peptide de GCRA est la SEQ ID n° : 1 (SP-304), la SEQ ID n° : 9 (SP-333), la SP373 (SEQ ID n° : 104), la SP364 (SEQ ID n° : 100), la SP366 (SEQ ID n° : 102) ou la SEQ ID n° : 250.

6. Composition destinée à l'utilisation de la revendication 1, comprenant en outre un opiacé.

7. Composition destinée à l'utilisation de la revendication 6, dans laquelle l'opiacé et le peptide agoniste du récepteur de guanylate cyclase (GCRA) sont administrés en un comprimé ou une capsule.

8. Composition destinée à l'utilisation de la revendication 6, comprenant en outre un ou plusieurs matériel(s) de ciblage choisi(s) dans le groupe constitué par un polymère dépendant du pH, un polymère gonflable et une composition dégradable.

9. Composition destinée à l'utilisation de la revendication 6, comprenant en outre un transporteur, un excipient ou un diluant pharmaceutique.

10. Composition destinée à l'utilisation de la revendication 8, dans laquelle la formulation est optimisée pour une délivrance au duodénum, au jéjunum, à l'iléon, à l'iléon terminal ou au côlon ascendant, ou dans laquelle la formulation est optimisée pour une libération le long du côlon.

11. Composition destinée à l'utilisation de la revendication 8, dans laquelle la formulation comprend un ou plusieurs polymère(s) dépendent(s) du pH qui dégrade/dégradent dans une plage de pH de 4,5 à 5,5, dans une plage de pH de 5,5 à 6,5 ou dans une plage de pH de 6,5 à 7,5.

12. Composition destinée à l'utilisation de la revendication 8, dans laquelle la libération de la composition est dépendante du temps.

13. Composition destinée à l'utilisation de la revendication 6, dans laquelle l'opiacé est choisi dans le groupe constitué par la morphine, la codéine, l'oxycodone, l'hydrocodone, la dihydro-codéine, le propoxyphène, le fentanyl, le tramadol, le lopéramide, le butorphanol ou des combinaisons de ceux-ci.

14. Composition destinée à l'utilisation de la revendication 1, dans laquelle ledit peptide de GCRA est la SEQ ID n° : 1 (SP-304) ou la SEQ ID n° : 9 (SP-333) .
